# EUROPEAN PATENT SPECIFICATION

(11) **EP 2 776 063 B1**
(45) Date of publication and mention of the grant of the patent: **03.04.2019**
(21) Application number: 12797747.8
(22) Date of filing: 12.11.2012
(51) Int. Cl.: A61K 39/145, C12N 7/04, A61P 31/16

(54) **INFLUENZA VIRUS-LIKE PARTICLES (VLPS) COMPRISING HEMAGGLUTININ PRODUCED IN NICOTIANA TABACUM**
INFLUENZA-VIRUS-ÄHNLICHE PARTIKEL (VLPS) MIT IN EINER NICOTIANA TABACUM PRODUZIERTEM HÄMAGGLUTININ
PSEUDO-PARTICULES DU VIRUS DE LA GRIPPE (VLP) COMPRENANT DE L'HÉMAGGLUTININE PRODUIT DANS NICOTIANA TABACUM

(30) Priority: 11.11.2011 EP 11188872
(43) Date of publication of application: 17.09.2014
(73) Proprietor: Philip Morris Products S.A., 2000 Neuchâtel (CH)
(72) Inventor: CABRERA, Rosa, CH-2072 Saint Blaise (CH); OISHI, Karen, CH-2000 Neuchâtel (CH); PEREZ, Laurent, CH-6648 Minusio (CH); TALAMO, Fabio, CH-2000 Neuchâtel (CH)
(74) Representative: Vossius & Partner Patentanwälte Rechtsanwälte mbB
(86) International application number: PCT/EP2012/072417
(87) International publication number: WO 2013/068593

(56) References cited:
- WO-A1-2009/009876
- WO-A1-2010/003225
- D'AOUST MARC-ANDRÉ ET AL: "Influenza virus-like particles produced by transient expression in Nicotiana benthamiana induce a protective immune response against a lethal viral challenge in mice", PLANT BIOTECHNOLOGY JOURNAL, BLACKWELL, OXFORD, GB, vol. 6, no. 9, 1 December 2008 (2008-12-01), pages 930-940, XP002598510, ISSN: 1467-7644

## Description

The present invention relates to a composition or preparation of lipid vesicles displaying a proteinaeceous antigen and carbohydrates, and the production thereof in plant cells. The lipid vesicles in said composition have a bilayer structure, a size distribution in the range of from about 40 nm to 250 nm, and an amount of antigen and carbohydrates that effectively stimulates an immune response against the antigen.

In particular, the present invention relates to the production of Virus-Like Particles ('VLPs') in *Nicotiana tabacum* plants and the isolation of VLPs of a size distribution, antigen/carbohydrate ratio and lipid composition that confers to the VLPs advantageous immunogenic properties.

Viral structural proteins can self-assemble into organized macromolecular structures to generate empty shells known as virus-like particles ('VLPs'). Virus-like particles are non-infectious because they lack viral nucleic acid and can be used as carrier for antigen presentation. It can potentially increase immunogenicity of peptide antigens, which by themselves are not efficient at stimulating the immune system, and to enhance production of protective antibodies against said antigens. It has been reported previously that VLPs can be produced and assembled in different prokaryotic and eukaryotic heterologous expression systems such as, for example, bacterial-, yeast- and mammalian-based systems. Recently, production of VLPs has also been reported in several plants - potato, lupine, lettuce, tomatoes, soybean, and *Nicotiana bentaminiana.* VLPs are potentially useful as a safe alternative to vaccines which are based on attenuated live or Inactivated killed viruses.

WO 2009/009876 A1 and WO 2010/003225 A1 disclose the production of VLPS in *Nicotiana benthamiana* plants. Example 16 relates to transient expression of H5 by agroinfiltration in *N. tabacum* plants. Expression of hemagglutinin in infiltrated *N*. *tabacum* leaves is reported to result in the accumulation of the uncleaved translation product (HAO precursor protein).

The present invention now provides VLPs with advantageous immunogenic properties produced in *Nicotiana tabacum* plants.

The present invention is defined by the claims and the following corresponding embodiments:
In a first embodiment, the invention relates to a method of producing a VLP displaying oneor more influenza hemagglutinin antigens or an immunogenic fragment thereof, wherein said VLPs have an average size distribution of between 40 nm and 250 nm in diameter comprising the steps of:
i. providing a combination of a selected variety, breeding line, or cultivar of a *Nicotiana tabacum* plant and a selected strain of an *Agrobacterium* species, which variety, breeding line, or cultivar, exhibits less than 10% necrosis, less than 5% necrosis, less than 2% necrosis or less than 1% necrosis, 5 days after leaves of said variety, breeding line, or cultivar have been injected by syringe with the selected *Agrobacterium* strain at a cell density of OD₆₀₀ of 0.32;
ii. infiltrating a whole plant of the selected variety, breeding line, or cultivar of *Nicotiana tabacum* with a suspension of the selected strain of the *Agrobacterium* species comprising a binary vector, comprising the coding seguence of one or more influenza antigens that is (are) under control of regulatory elements functional in a *Nicotiana tabacum* plant, at an OD₆₀₀ of between 0.1 and 4.0.
iii. incubating the infiltrated plant for a period of between 5 days and 20 days, under conditions that allow expression of the expressible nucleotide sequence in the infiltrated plant and accumulation of the heterologous polypeptide; and optionally the steps of:
iv. purification/cleaning of the VLPs produced; and
v. capturing of VLPs.

In a specific embodiment of the invention, said binary vector is a minimally-sized binary vector comprising seguence elements, which are essential for maintenance and replication of the plasmid in *Escherichia coli* and *Agrobacterium* cells, and for the transfer of the T-DNA to a tobacco plant cell, and further a T-DNA region and wherein the essential sequence elements accounts for at least 60%, 65%, 70%, 75%, 80% of the entire minimally-sized binary vector.

In another specific embodiment, the binary vector comprises a plant selectable marker gene.

In another specific embodiment, the invention relates to the method of any one of the preceding embodiments, wherein the infiltrated plant is incubated for a period of between 7 days and 15 days.

In another specific embodiment,the invention relates to the method of anv one of the preceding embodiments, wherein the infiltrated plant is incubated for a period of between 8 days and 10 days.

In another specific embodiment, the invention relates to the method of any one of the preceding embodiments, wherein said VLPs have an average size distribution of between 50 nm and 200 nm in diameter.

In still another specific embodiment, the invention relates to the method of any one of the preceding embodiments, wherein said VLPs are composed of a lipid bilayer comprising a combination of fatty acids and sterols.

In various other embodiments, the invention relates to the method of any one of the preceding embodiments, wherein the lipid bilayer of the VLPs has a content of
- oleic acid of less than 0.5 µg/mL: and/or
- linoleic acid and/or linolenic acid in a concentration of between 3.0 µg/mL and 5.0 µg/mL: and/or
- arachidic acid of greater than 0.75 µg/mL,

In various further embodiments, the invention relates to the method of any one of the preceding embodiments, wherein the VLPs
-have in the lipid bilayer a cholesterol:sitosterol ratio of >1, when determined by LC-APPCI-MS/MS: and/or
-exhibit a mass ratio of lipid (fatty acid and sterols) to protein that is between 0.30 and 0.45.

The invention further relates to the method of any one of the preceding embodiments, wherein the hemagglutinin is an Influenza type A or an Influenza type B hemagglutinin.

In various specific embodiments, the Influenza sub-type A hemagglutinin is selected from the group consisting of H1, H2, H3, H4, H5, H6, H7, H8, H9, H10 H11, H12, H13, H14, H15, and H16, particularly the Influenza sub-type A hemagglutinin is a hemagglutinin of the H5 sub-type.

In a specific embodiment, the invention relates to the method of the preceding embodiment, wherein the antigen displayed on the VLPs is an influenza A hemagglutinin of the H5 sub-type as shown in SEQ ID NO: 14.

In various other embodiments, the invention relates to the method of any one of the preceding embodiments, wherein the hemagglutinin displayed by the virus-like particles is
- S-acylated;
- N-glvcosylated:
- S-acylated and N-glycosylated.

In a specific embodiment, the invention relates to the method of the preceding embodiment, wherein only two cytoplasmic cysteines are post-translationally modified by acylation with palmitic acid.

Disclosed, herein is a composition of virus-like particles ('VLPs") obtained from a *Nicotiana tabacum* plant and comprising VLPs displaying one or more influenza antigens, wherein said VLPs have an average size distribution of between 40 nm and 250 nm, 40 nm and 200 nm, 50 nm and 250 nm particularly of between 50 nm and 200 nm, in diameter. The VLPs in the above defined composition may be composed of a lipid bilayer comprising fatty acids and sterols. In particular, the fatty acid:sterol ratio is >1.

Further disclosed herein is a composition of Virus-Like Particles ('VLPs") obtained from a *Nicotiana tabacum* plant and comprising VLPs displaying one or more influenza antigens, wherein said VLPs have a peak top representing the most abundant population in a range of between 80 nm and 140 nm, particularly of between 90 and 130 nm, particularly of between 80 nm and 120 nm, particularly of between 90 nm and 120 nm, in diameter. In particlular, the *N. tabacum* produced virus-like particles may contain low concentrations of monounsaturated fatty acids, particularly of C₁₈ monounsaturated fatty acids such as, for example, oleic acid, which is present in the lipid bilayer In a concentration of less than 0.4 µg/mL, less than 0.5 µg/mL, less than 1.0 µg/mL, or less than 1.5 µg/mL when determined according to GC-MS or LC-MS.

Further, the *N. tabacum* produced virus-like particles as disclosed herein may contain increased concentrations of polyunsaturated fatty acids, particularly of C₁₈ polyunsaturated fatty acids such as, for example, linoleic acid and/or linolenic acid, which is present in the lipid bilayer in a concentration of between 3.0 µg/mL and 5.0 µg/mL when determined according to GC-MS or LC-MS.

Further, the *N. tabacum* produced virus-like particles as disclosed herein may contain increased concentrations of saturated fatty acids, particularly of C₂₀ saturated fatty acids such as arachidic acid of greater than 0.5 µg/mL, greater than 0.75 µg/mL, greater than 0.9 µg/mL, or greater than 1.0 µg/mL when determined according to GC-MS or LC-MS.

In the alternative, the *N. tabacum* produced virus-like particles as disclosed herein may contain a combination of monounsaturated fatty acids, particularly of C₁₈ monounsaturated fatty acids such as, for example, oleic acid and polyunsaturated fatty acids, particularly of C₁₈ - C₂₀ polyunsaturated fatty acids such as, for example, linoleic acid and/or linolenic acid and/or C₂₀ saturated fatty acids such as arachidic acid in a concentration as disclosed herein above in the preceding paragraphs.

In particular the lipid bilayer of the VLPs as disclosed in any of the preceding paragraphs has a ratio of cholesterol to sitosterol (cholesterol:sitosterol) of greater than 1, greater than 0.75, greater than 0.5, and greater than 0.4, when determined by LC-APPCI-MS/MS.

Also contemplate herein is a composition of VLPs wherein the VLPs of the preceding paragraphs exhibit a mass ratio of lipid (fatty acid and sterols) to protein that is between 0.30 and 0.45, greater than 0.35, greater than 0.4, and particularly between 0.34 and 0.42.

Further disclosed herein is a composition of virus-like particles ('VLPs') obtained from a *Nicotiana tabacum* plant and comprising VLPs displaying one or more antigens, particularly one or more influenza antigens, wherein said VLPs have an average size distribution of between 40 nm and 250 nm, particularly of between 50 nm and 200 nm, In diameter and are composed of a lipid bilayer comprising a combination of fatty acids and sterols.

In particular, a composition of virus-like particles ('VLPs") obtained from a *Nicotiana tabacum* plant and comprising VLPs is disclosed displaying one or more influenza antigens, wherein said VLPs have a peak top representing the most abundant population in a range of between 80 nm and 140 nm, particularly of between 90 and 130 nm, particularly of between 80 nm and 120 nm, particularly of between 90 nm and 120 nm, in diameter.

Further, a composition of virus-like particles ('VLPs') is disclosed obtained from a *Nicotiana tabacum* plant and comprising VLPs displaying one or more antigens, particularly one or more influenza antigens, wherein said VLPs have an average size distribution of between 40 nm and 250 nm, particularly of between 50 nm and 200 nm in diameter and the lipid bilayer of the VLPs has a content of oleic acid of less than 0.5 µg/mL when determined according to GC-MS or LC-MS and/or a content of arachidic acid of greater than 0.75 µg/mL when determined according to GC-MS or LC-MS, and/or a content of linoleic acid and/or linolenic acid of of between 3.0 µg/mL and 5.0 µg/mL when determined according to GC-MS or LC-MS.

Also disclosed herein is a composition of virus-like particles ('VLPs') obtained from a *Nicotiana tabacum* plant and comprising VLPs displaying one or more antigens, particularly one or more influenza antigens, wherein said VLPs have a peak top representing the most abundant population in a range of between 80 nm and 140 nm, particularly of between 90 and 130 nm, particularly of between 80 nm and 120 nm, particularly of between 90 nm and 120 nm, in diameter.

In particular, the composition of virus-like particles ('VLPs') as described herein obtained from a *Nicotiana tabacum* plant comprises VLPs displaying one or more influenza antigens, wherein said VLPs have an average size distribution of between 40 nm and 250 nm, particularly of between 50 nm and 200 nm, in diameter and the lipid bilayer of the VLPs has a cholesterol:srtosterol ratio of >1, when determined by LC-APPCI-MS/MS.

In the alternative, the composition of virus-like particles ('VLPs') as described herein obtained from a *Nicotiana tabacum* plant comprises VLPs displaying one or more influenza antigens, wherein said VLPs have a peak top representing the most abundant population in a range of between 80 nm and 140 nm, particularly of between 90 and 130 nm, particularly of between 80 nm and 120 nm, particularly of between 90 nm and 120 nm, in diameter.

Also described herein is a composition of virus-like particles ('VLPs') obtained from a *Nicotiana tabacum* plant and comprising VLPs displaying one or more antigens, particularly one or more influenza antigens, wherein said VLPs have (i) an average size distribution of between 40 nm and 250 nm, particularly of between 50 nm and 200 nm, In diameter; (ii) are composed of a lipid bilayer comprising a combination of fatty acids and sterols, wherein, in one embodiment, the fatty acid:sterol ratio Is >1, (iii) the content of oleic add is less than 0.5 µg/mL when determined according to GC-MS or LC-MS and/or the content of arachidic acid is greater than 0.75 µg/mL when determined according to GC-MS or LC-MS, and/or the content of linoleic acid and/or linolenic acid is between 3.0 µg/mL and 5.0 µg/mL when determined according to GC-MS or LC-MS. and (iv) the cholesterol:sitosterol ratio is >1, when determined by LC-APPCI-MS/MS.

Said VLPs may have a peak top representing the most abundant population in a range of between 80 nm and 140 nm, particularly of between 90 and 130 nm, particularly of between 80 nm and 120 nm, particularly of between 90 nm and 120 nm, In diameter.

The antigen displayed on the VLPs of any one of the composition defined in the preceding paragraphs may be a hemagglutinin, particularly a recombinant hemagglutinin. The hemagglutinin may, in particular, be an influenza type A or an influenza type B hemagglutinin, or an Influenza sub-type A hemagglutinin selected from the group consisting of H1, H2, H3, H4, H5, H6, H7, H8, H9, H10 H11, H12, H13, H14, H15, and H16.

In particular, the hemagglutinin is of the H5 sub-type.

In particular, the VLPs display only one Influenza antigen.

In particular, the VLPs display only one influenza antigen, and the antigen is a hemaglutinin.

In particular, the VLPs display only one influenza antigen, and the antigen is a hemaglutinin of the H5 subtype.

Further, the hemagglutinin displayed by the virus-like particles according to any one of the preceding paragraphs may be S-acylated or N-glycosylated, or both, S-acylated and N-glycosylated.

In particular, the hemagglutinin displayed by the virus-like particles according to any one of the preceding paragraphs has only two cytoplasmic cysteines, which are post-translationally modified by acylation with palmitic acid.

The VLPs of the preceding paragraphs may be produced In *Nicotiana tabacum* plants or plant cells *in vivo* or *in vitro.*

In particular, the VLPs of the preceding paragraphs are isolated.

Also disclosed herein is a method for producing VLPs as described inhere before, comprising the steps of: (i) purification/cleaning (ii) capturing of VLPs by using the techniques as described herein.

Further disclosed herein is a composition comprising processed plant biomass enriched with VLPs as disclosed herein.

Also contemplated herein are pharmaceutical compositions comprising VLPs as defined herein, particularly in a therapeutically-effective amount, together with a pharmaceutically acceptable carrier.

In particular, said pharmaceutical composition as described herein comprises VLPs displaying one or more influenza virus hemagglutinin proteins, particularly a hemagglutinin protein of the H5 sub-type.

Further disclosed herein is a pharmaceutical composition comprising the VLPs or the VLP composition as defined herein particularly in an immunologically-effective amount, together with a pharmaceutically acceptable carrier for inducing an Immune response in a subject upon administration of said composition.

Also disclosed herein is a pharmaceutical composition comprising the VLPs or the VLP composition as defined herein, particularly in a therapeutically-effective amount, together with a pharmaceutically acceptable carrier for use in the treatment of or prophylaxis against an influenza virus infection, particularly of an influenza virus infection caused by an influenza virus of the H5 sub-type, in a subject in need of such a treatment.

In particular, any one of the above defined pharmaceutical composition may further comprises an adjuvant.

Further disclosed herein is the use of any one of the VLP compositions or pharmaceutical compositions as defined herein embodiments for inducing an immune response in a subject upon administration of said VLP composition.

Also disclosed herein is the use of any one of the VLP compositions or pharmaceutical compositions as defined herein for the treatment of or prophylaxis against an influenza virus infection, particularly of an influenza virus infection caused by an influenza virus of the H5 sub-type, in a subject in need of such a treatment.

Further contemplated herein are methods for inducing an immune response in a subject against a virus, particularly an influenza virus comprising administering to said subject any one of the VLP compositions or pharmaceutical compositions as disclosed herein in an immunologically effective amount.

Further contemplated herein are methods for the treatment, particularly for the prophylactic treatment or attenuation of a virus infection, particularly an influenza virus infection in a subject In need of such a treatment comprising administering to said subject any one of the VLP compositions or pharmaceutical compositions as disclosed herein in a therapeutically effective amount. Administration of the VLP compositions or pharmaceutical compositions as disclosed herein may be by any suitable route, particularly by oral, parenteral, subcutaneous, intraperitoneal, topical, transmucosal transdermal, intrabronchial, intrapulmonary and intranasal, intraventricular. intraarticular, intrathecal, intravaginal, or intratracheal administration.

### Brief Description of Sequences and Figures

In the description and examples reference is made to the following sequences that are presented in the sequence listing:
SEQ ID NO: 1: depicts the nucleotide sequence of minimal plant selectable binary vector pPMP1.
SEQ ID NO: 2: depicts the nucleotide sequence of the 5'UTR HT-CPMV.
SEQ ID NO: 3: depicts the nucleotide sequence of the 3'UTR HT-CPMV.
SEQ ID NO: 4: depicts the nucleotide sequence of P1-HcPro-P3.
SEQ ID NO: 5: depicts the nucleotide sequence of the double MMV promoter.
SEQ ID NOs: 6 and 7 depict C-terminal hemagglutinin H5 peptide fragments.
SEQ ID NOs: 8-13:depict different peptide fragments obtained from the H5 mature protein backbone.
SEQ ID NO: 14: depicts the nucleotide sequence of influenza haemagglutinin 5 (H5).

In the description and examples reference is made to the following figures:
Figure 1. Lipid vesicle displaying antigen and carbohydrate: AF4-MALS analysis of *Nicotiana*-derived virus-like particles purified by ultracentrifugation on a 60/45% sucrose cushion.
   A. *N. tabacum* PM132 (-x-x-x-), *N. tabacum* PM204 (-o-o-o-), *N. tabacum* Burley 21 (-) and
   B. *N. benthamiana* (-□-□-□-).
Figure 2. GC profile of fatty acids of *N. benthamiana*-derived virus-like particles with zoom from 13.00 to 20.00 minutes. Assignments are indicated on the peak top. The two unidentified fatty acids are tentatively assigned as C18-0 and C20-0 isomers of stearic acid and arachidic acid because their mass spectra are quite similar to those of stearic acid and arachidic acid.
Figure 3. Deconvoluted N-glycosylation patterns of Asn-11 glycopeptide of hemagglutinin H5 displayed on various virus-like particles. H5-PM015, *N. tabacum* Burley 21; H5-PM132, *N. tabacum* PM132; H5-PM204, *N. tabacum* PM204 and H5-PM182, *N. benthamiana.* Also see Example 23.

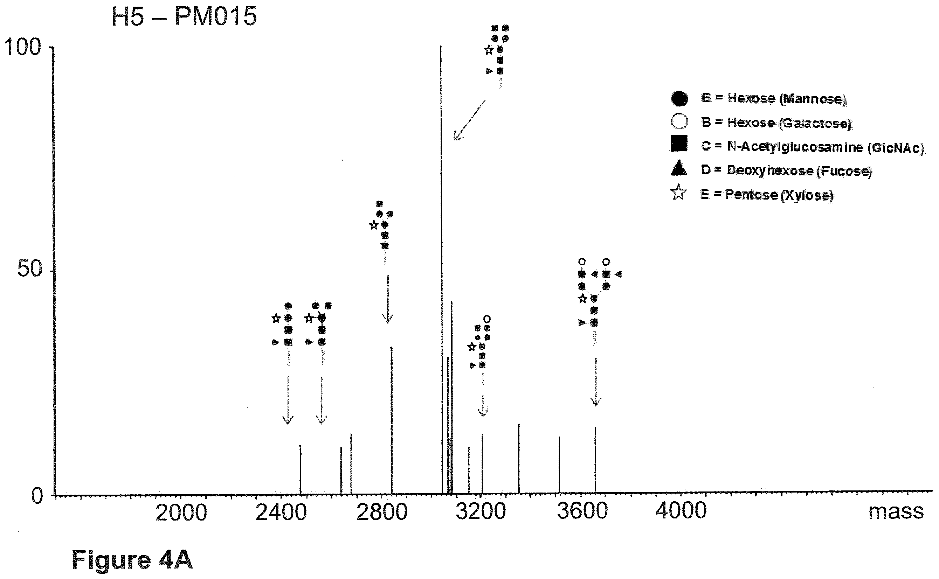

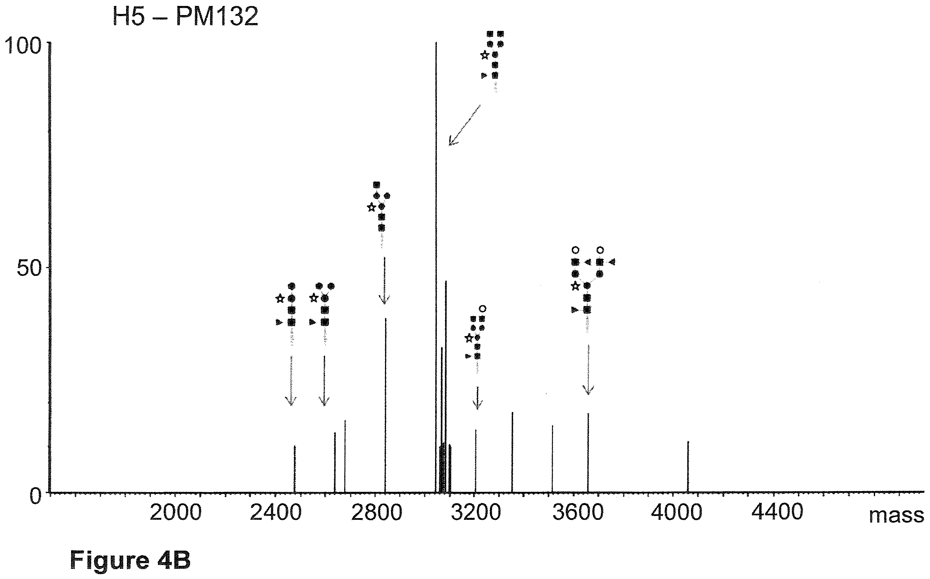

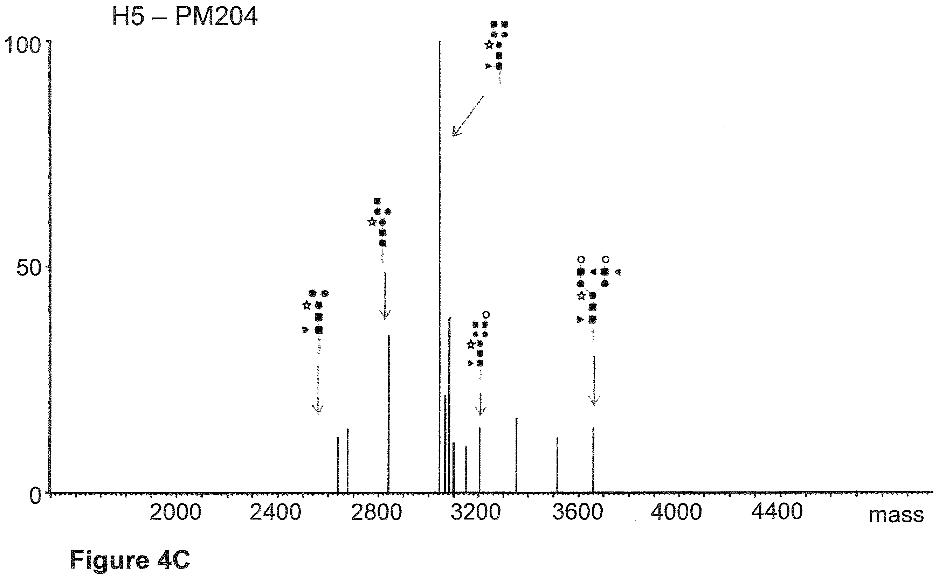

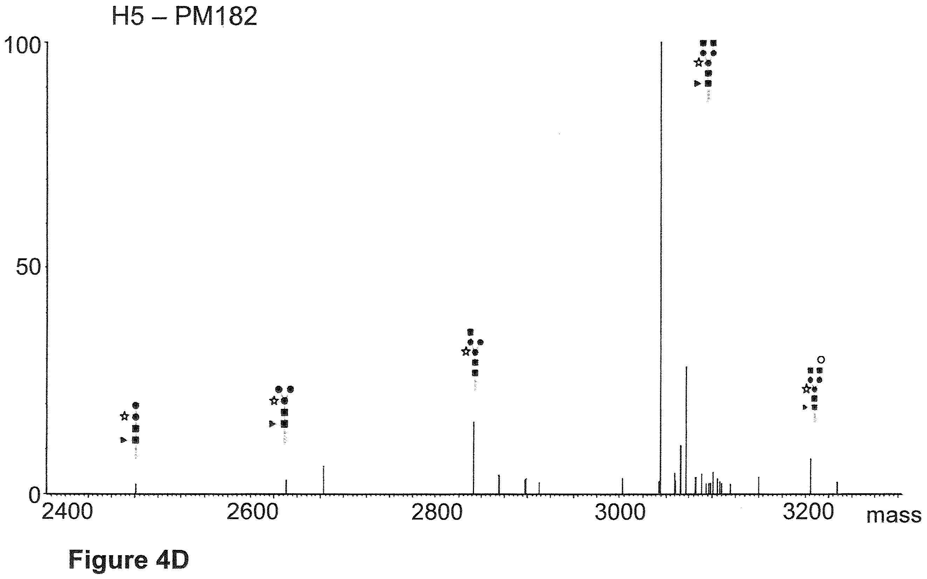

Figure 4. Deconvoluted N-glycosylation patterns of Asn-23 glycopeptide of hemagglutinin H5 displayed on various virus-like particles. H5-PM015, *N. tabacum* Burley 21; H5-PM132, *N. tabacum* PM132; H5-PM204, *N. tabacum* PM204 and H5-PM182, *N. benthamiana.* Also see Example 23.

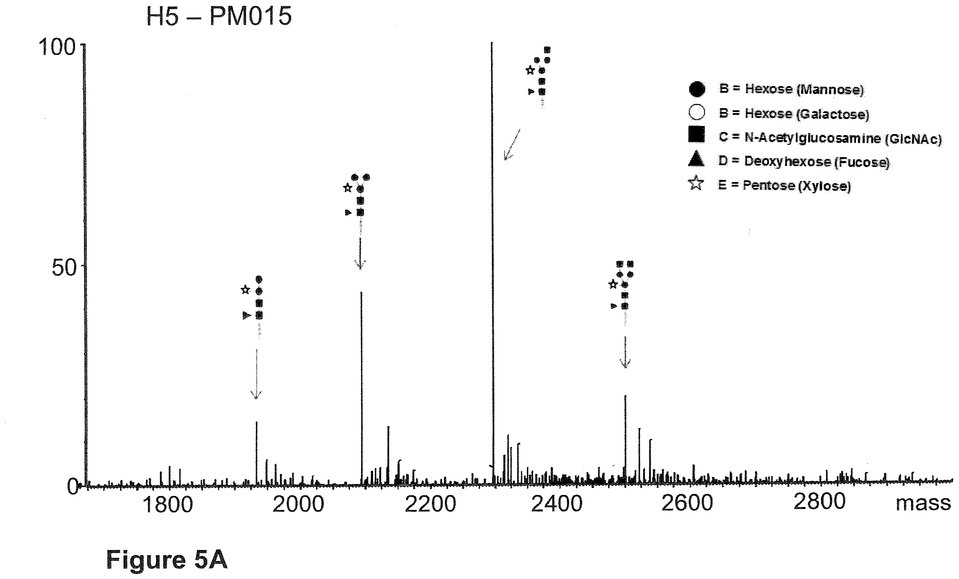

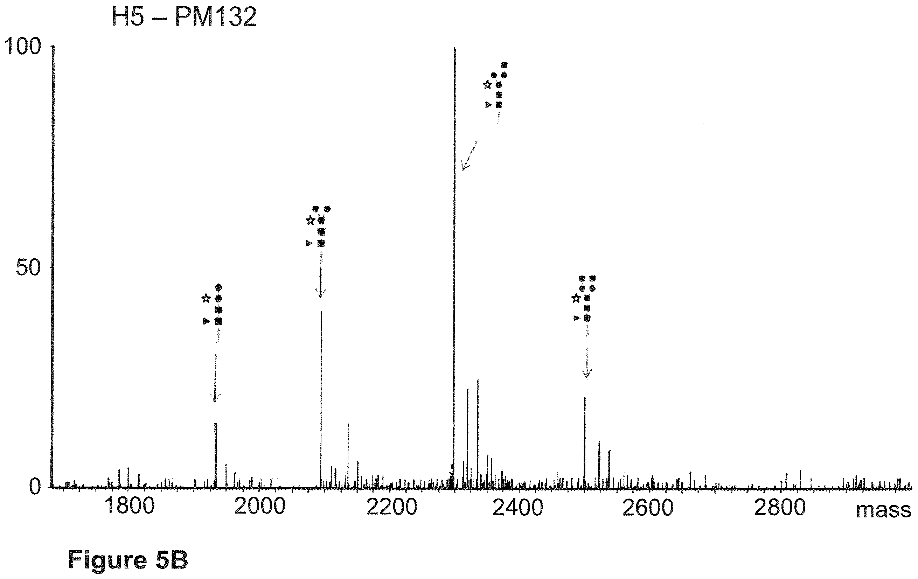

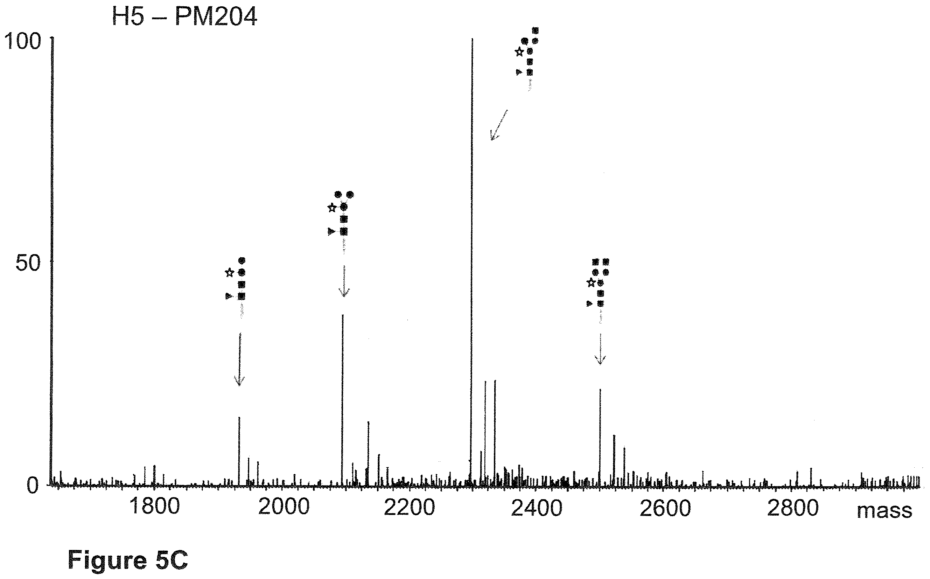

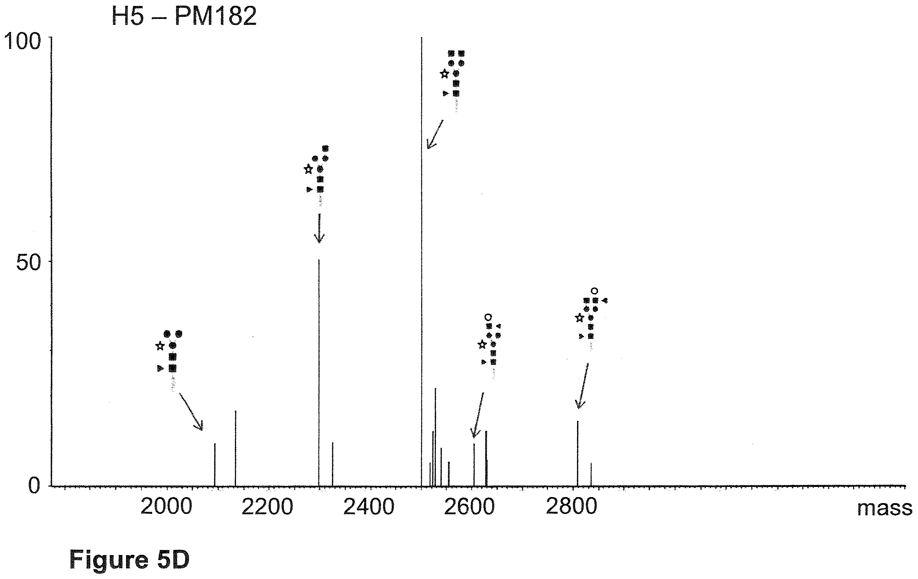

Figure 5. Deconvoluted N-glycosylation patterns of Asn-154 glycopeptide of hemagglutinin H5 displayed on various virus-like particles. H5-PM015, *N. tabacum* Burley 21; H5-PM132, *N. tabacum* PM132; H5-PM204, *N. tabacum* PM204 *and H*5-*PM182*, *N. benthamiana. Also* see *Example 23.*

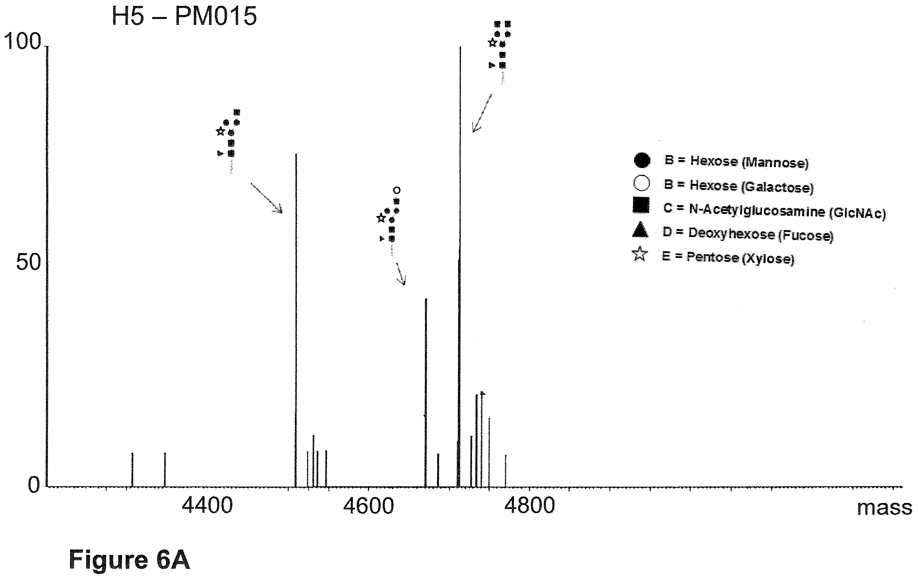

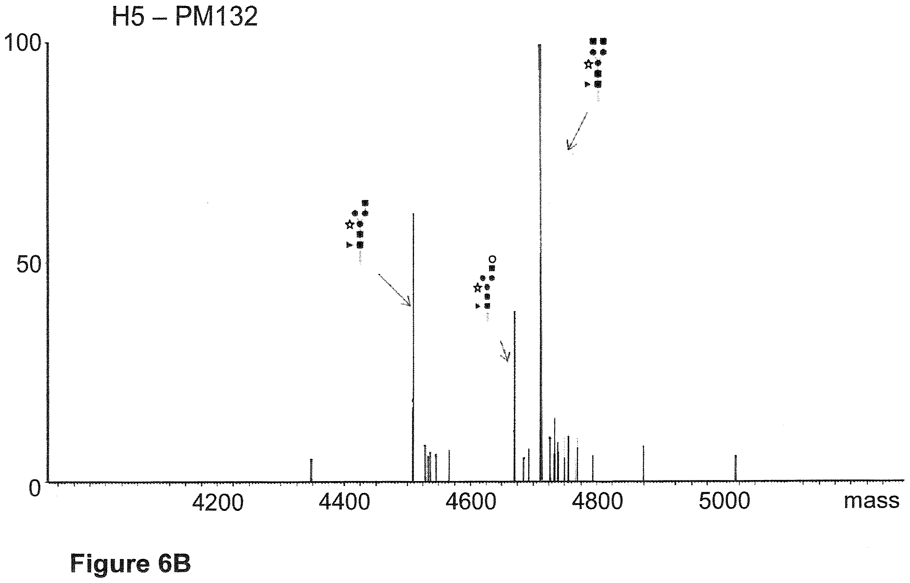

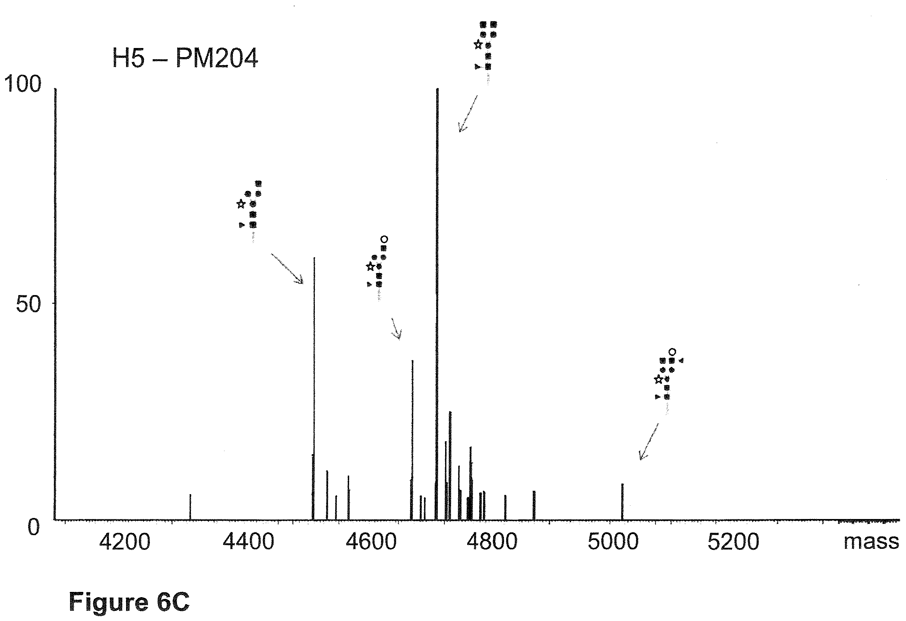

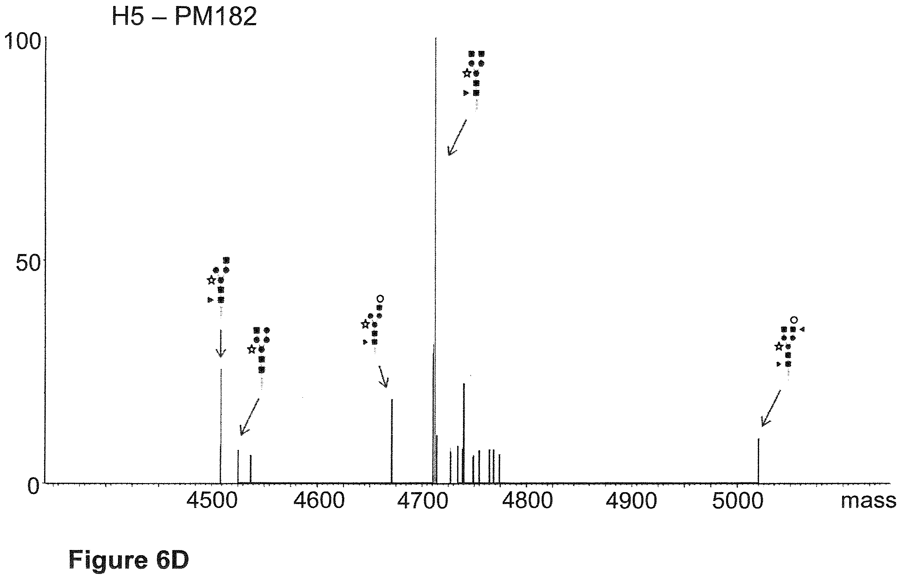

Figure 6. Deconvoluted N-glycosylation patterns of Asn-165 glycopeptide of hemagglutinin H5 displayed on various virus-like particles. H5-PM015, *N. tabacum* Burley 21: H5-PM132, *N. tabacum* PM132; H5-PM204, *N*. *tabacum* PM204 and H5-PM182. *N. benthamiana.* Also see Example 23.

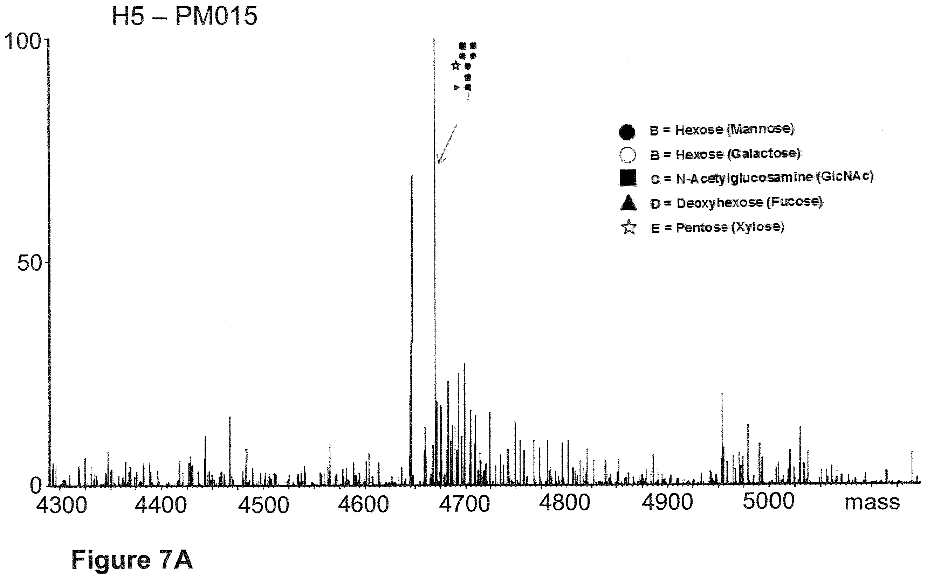

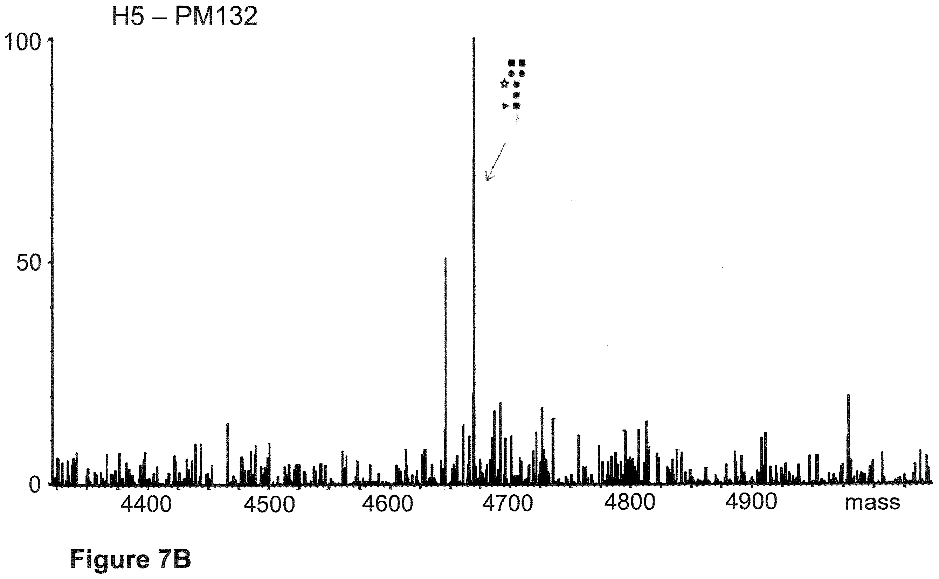

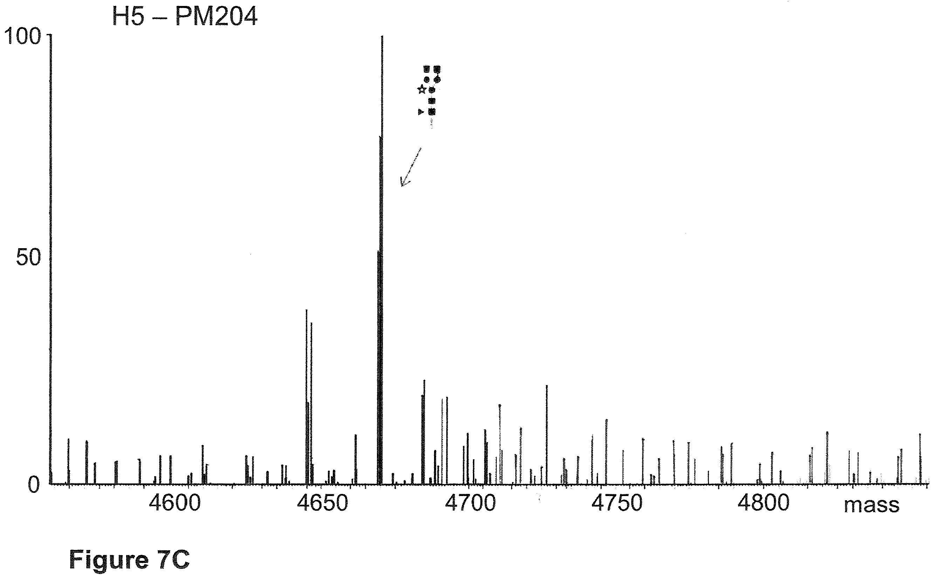

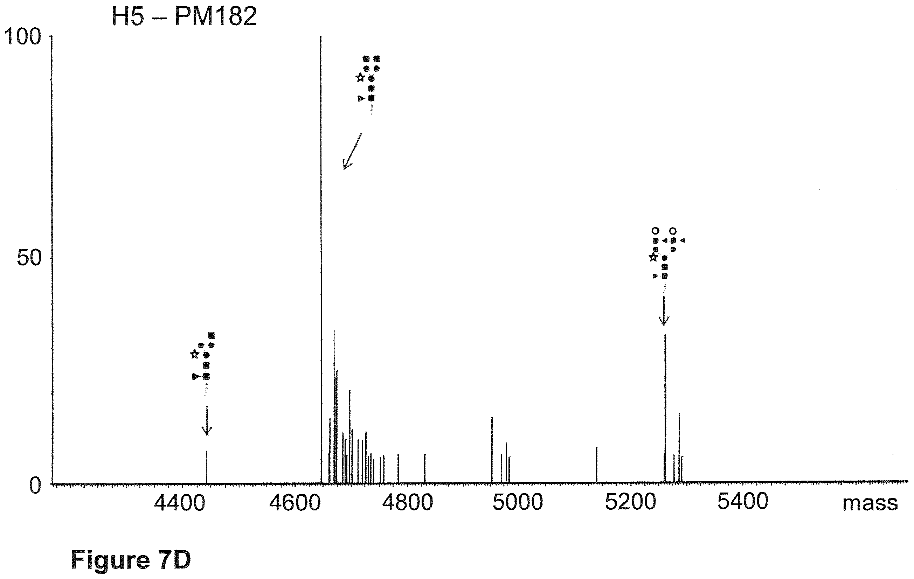

Figure 7. Deconvoluted N-glycosylation patterns of Asn-286 glycopeptide of hemagglutinin H5 displayed on various virus-like particles. H5-PM015, *N. tabacum* Burley 21; H5-PM132, *N. tabacum* PM132; H5-PM204, *N. tabacum* PM204 and H5-PM182, *N. benthamiana.* Also see Example 23.

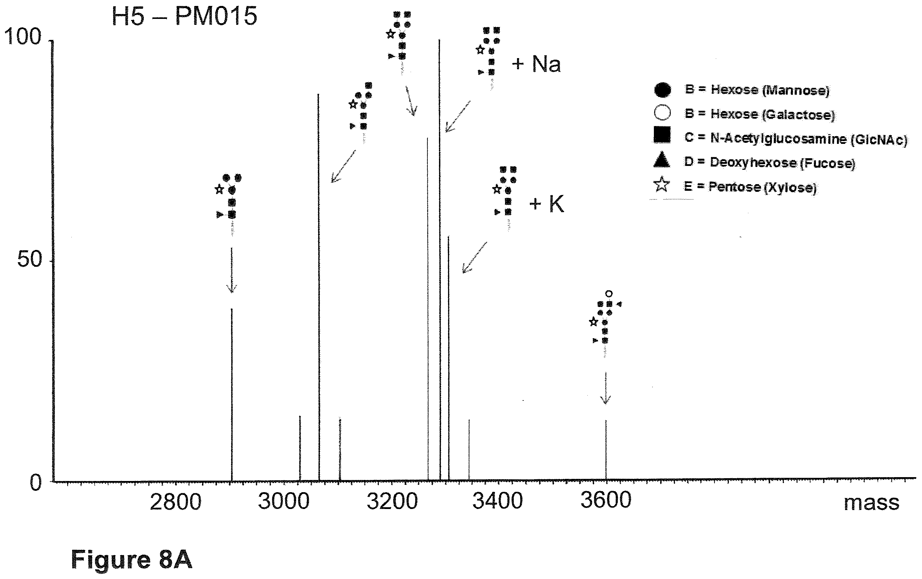

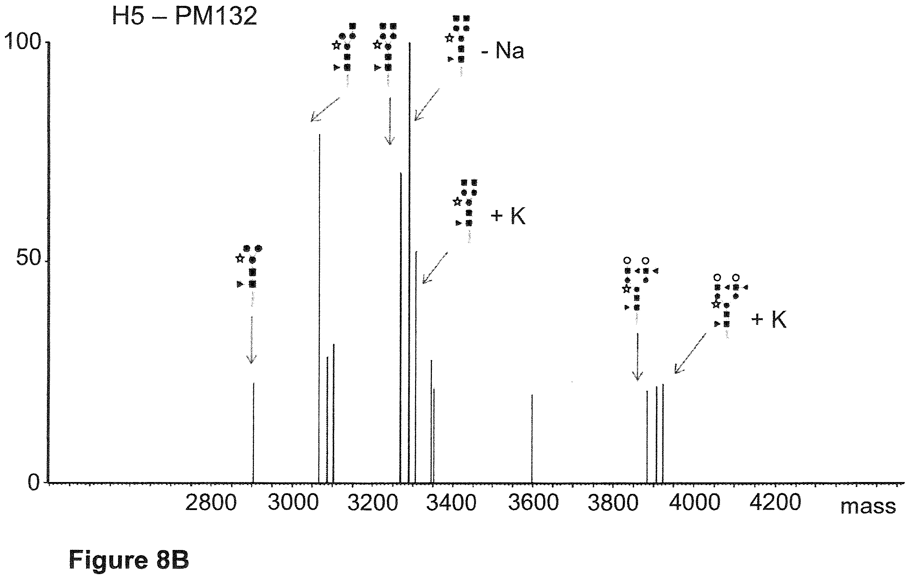

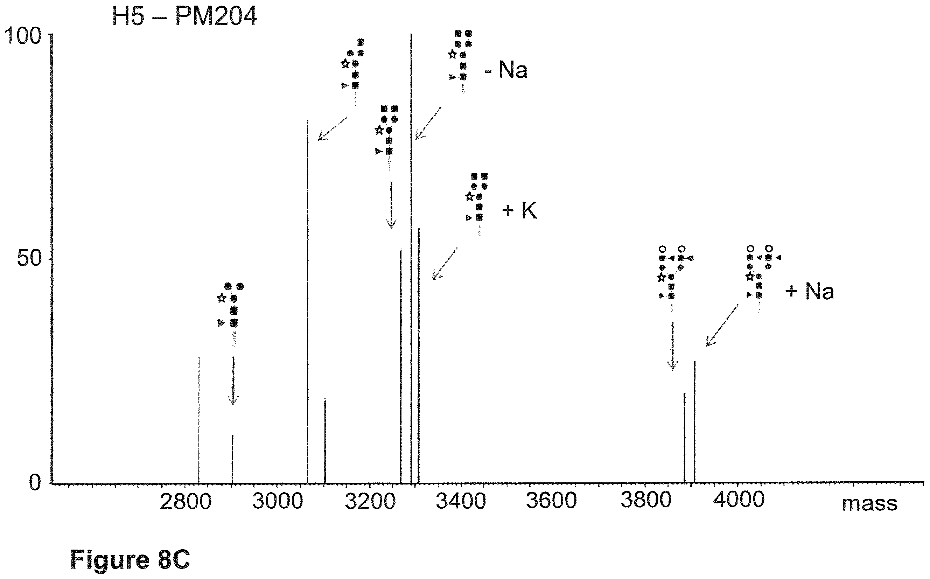

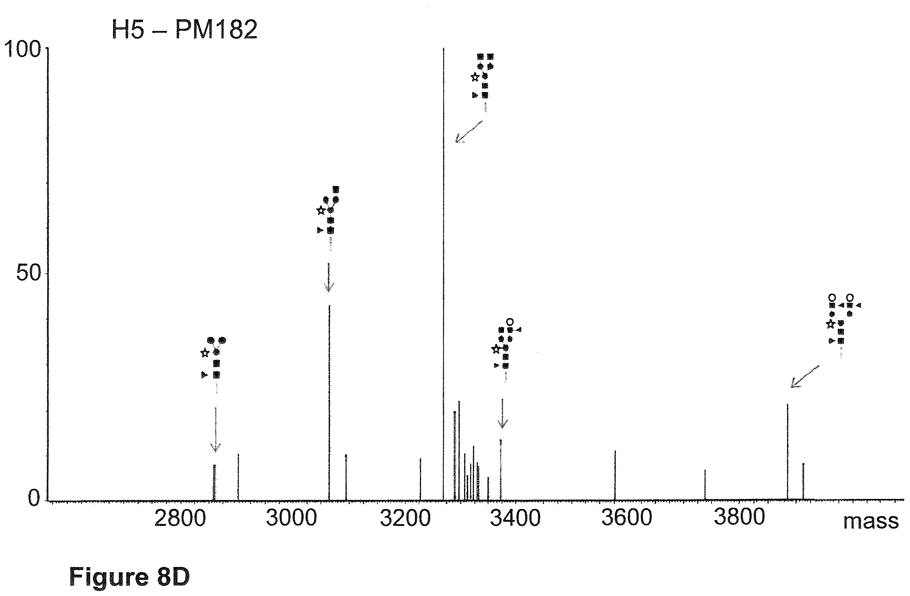

Figure 8. Deconvoluted N-glycosylation patterns of Asn-484 glycopeptide of hemagglutinin H5 displayed on various virus-like particles. H5-PM015, *N. tabacum* Burley 21; H5-PM132, *N. tabacum* PM132; H5-PM204, *N. tabacum* PM204 and H5-PM182, *N. benthamiana.* Also see Example 23.
Figure 9. MALDI-TOF mass spectrum of hemagglutinin H5 with signals corresponding to S-acylated peptide fragments.

### Definitions

Technical and scientific terms and expressions used within the scope of this application are generally to be given the meaning commonly applied to them In the pertinent art of plant biology. Reference is made herein to various methodologies known to those of skill in the art. The practice of the invention will employ, unless otherwise indicated, conventional techniques of chemistry, molecular biology, microbiology, genetic engineering and plant biology, which are within the skill of the art.

Any suitable materials and/or methods known to those of skill can be utilized In carrying out the present invention: however, preferred materials and/or methods are described. Materials, reagents and the like to which reference is made in the following description and examples are obtainable from commercial sources, unless otherwise noted.

All of the following term definitions apply to the complete content of this application. The word "comprising" does not exclude other elements or steps, and the indefinite article "a" or "an" does not exclude a plurality. A single step may fulfil the functions of several features recited in the claims. The terms "essentially", "about", "approximately" and the like in connection with an attribute or a value particularly also define exactly the attribute or exactly the value, respectively. The term "about" in the context of a given numerate value or range refers to a value or range that is within 20 %, within 10 %, or within 5 % of the given value or range.

A "plant" as used within the present invention refers to any plant at any stage of its life cycle or development, and its progenies.

A "plant part" or "part of a plant" as used herein is meant to refer to any part of a plant, i.e. a plant organ, a plant tissue, a plant cell, an embryo, a leaf, etc. in planta or in culture. In certain embodiments of the invention relating to plant inoculation under high or low pressure or a combination thereof, this term refers to plant parts in planta.

A "tobacco plant" as used within the present invention refers to a plant of a species belonging to the genus Nicotiana, including but not limited to *Nicotiana tabacum* (or *N. tabacum*)*.* Certain embodiments of the invention are described herein using the term "tobacco plant" without specifying *Nicotiana tabacum,* such descriptions are to be construed to have included *Nicotiana tabacum* specifically.

A "plant cell" or "tobacco plant cell" as used within the present invention refers to a structural and physiological unit of a plant, particularly a tobacco plant. The plant cell may be in form of a protoplast without a cell wall, an isolated single cell or a cultured cell, or as a part of higher organized unit such as but not limited to, plant tissue, a plant organ, or a whole plant.

"Plant material" as used within the present invention refers to any solid, liquid or gaseous composition, or a combination thereof, obtainable from a plant, including leaves, stems, roots, flowers or flower parts, fruits, pollen, egg cells, zygotes, seeds, cuttings, secretions, extracts, cell or tissue cultures, or any other parts or products of a plant.

"Plant tissue" as used herein means a group of plant cells organized into a structural or functional unit. Any tissue of a plant in planta or in culture is included. This term includes, but is not limited to, whole plants, plant organs, and seeds.

A "plant organ" as used herein relates to a distinct or a differentiated part of a plant such as a root, stem, leaf, flower bud or embryo.

The term "optical density" or "OD" relates to the optical determination of absorbance of an optical element at a given wavelength (e.g. 600nm = OD₆₀₀) measured in a spectrophotometer.

The term "polynucleotide" is used herein to refer to a polymer of nucleotides, which may be unmodified or modified deoxyribonucleic acid (DNA) or ribonucleic acid (RNA). Accordingly, a polynucleotide can be, without limitation, a genomic DNA, complementary DNA (cDNA), mRNA, or antisense RNA. Moreover, a polynucleotide can be single-stranded or double-stranded DNA, DNA that is a mixture of single-stranded and double-stranded regions, a hybrid molecule comprising DNA and RNA, or a hybrid molecule with a mixture of single-stranded and double-stranded regions. In addition, the polynucleotide can be composed of triple-stranded regions comprising DNA, RNA, or both. A polynucleotide can contain one or more modified bases, such as phosphothioates, and can be a peptide nucleic acid (PNA). Generally, polynucleotides provided by this invention can be assembled from isolated or cloned fragments of cDNA, genome DNA, oligonucleotides, or individual nucleotides, or a combination of the foregoing.

The term "gene sequence" as used herein refers to the nucleotide sequence of a nucleic acid molecule or polynucleotide that encodes a protein or polypeptide, particularly a heterologous protein or polypeptide or a biologically active RNA, and encompasses the nucleotide sequence of a partial coding sequence that only encodes a fragment of a heterologous protein. A gene sequence can also include sequences having a regulatory function on expression of a gene that are located upstream or downstream relative to the coding sequence as well as intron sequences of a gene.

The term "transcription regulating nucleotide sequence" or "regulatory sequences", each refer to nucleotide sequences influencing the transcription, RNA processing or stability, or translation of the associated (or functionally linked) nucleotide sequence to be transcribed. The transcription regulating nucleotide sequence may have various localizations with the respect to the nucleotide sequences to be transcribed. The transcription regulating nucleotide sequence may be located upstream (5' non-coding sequences), within, or downstream (3' non-coding sequences) of the sequence to be transcribed (e.g., a coding sequence). The transcription regulating nucleotide sequences may be selected from the group comprising enhancers, promoters, translation leader sequences, introns, 5'-untranslated sequences, 3'-untranslated sequences, and polyadenylation signal sequences. They include natural and synthetic sequences as well as sequences, which may be a combination of synthetic and natural sequences.

The term "promoter" refers to the nucleotide sequence at the 5' end of a gene that directs the initiation of transcription of the gene. Generally, promoter sequences are necessary, but not always sufficient, to drive the expression of a gene to which it is operably linked. In the design of an expressible gene construct, the gene is placed in sufficient proximity to and in a suitable orientation relative to a promoter such that the expression of the gene is controlled by the promoter sequence. The promoter is positioned preferentially upstream to the gene and at a distance from the transcription start site that approximates the distance between the promoter and the gene it controls in its natural setting. As is known in the art, some variation in this distance can be tolerated without loss of promoter function.

As used herein, the term "operatively linked" means that a promoter is connected to a coding region in such a way that the transcription of that coding region is controlled and regulated by that promoter. Means for operatively linking a promoter to a coding region are well known in the art.

The term "suppressor of gene silencing" used in the context of this invention refers to virus-encoded proteins that allow certain viruses to circumvent post-transcriptional gene silencing by binding to silencing RNA's. Also transgenes, when introduced in a plant cell, can trigger post-transcriptional gene silencing as the result of which low or no expression of such genes is established.

The terms "protein", "polypeptide", "peptide" or "peptide fragments" as used herein are interchangeable and are defined to mean a biomolecule composed of two or more amino acids linked by a peptide bond, which may be folded into secondary, tertiary or quaternary structure to achieve a particular morphology.

The term "heterologous" as used herein refers to a biological sequence that does not occur naturally in the context of a specific polynucleotide or polypeptide in a cell or an organism. The term "recombinant protein" or "heterologous protein" or "heterologous polypeptide", as used herein interchangeably, refers to a protein or polypeptide that is produced by a cell but does not occur naturally in the cell. For example, the recombinant or heterologous protein produced in a plant cell or whole plant can be a mammalian or human protein.

The heterologous protein that can be expressed in a modified plant cell can be an antigen (that can be, without limitation, used in a vaccine) including but not limited to a protein of a pathogen, a viral protein, a bacterial protein, a protozoal protein, a nematode protein; an enzyme, including but not limited to an enzyme (that can be, without limitation, used in treatment of a human disease or for industrial uses); a cytokine; a fragment of a cytokine receptor; a blood protein; a hormone; a fragment of a hormone receptor, a lipoprotein; an antibody or a fragment of an antibody.

The terms "antibody" and "antibodies" refer to monoclonal antibodies, multispecific antibodies, human antibodies, humanized antibodies, camelised antibodies, chimeric antibodies, single-chain Fvs (scFv), single chain antibodies, single domain antibodies, domain antibodies (VH, VHH, VLA), Fab fragments, F(ab') fragments, disulfide-linked Fvs (sdFv), and epitope-binding fragments of any of the above. In particular, antibodies include immunoglobulin molecules and immunologically active fragments of immunoglobulin molecules, i.e., molecules that contain an antigen binding site. Immunoglobulin molecules can be of any type (for example, IgG, IgE, IgM, IgD, IgA and IgY), class (for example, IgG1, IgG2, IgG3, IgG4, IgA1 and IgA2) or subclass.

The term "expressible" in the context of this invention refers to an operative linkage of a gene to regulatory elements that direct the expression of the protein or polypeptide encoded by the gene in plant cells comprised within a leaf.

The term "necrosis" and necrotic response" as used herein interchangeably relates to a hypersensitive response in the tissue of a plant, particularly a tobacco plant, triggered by, for example, inoculation of the plant tissue with, for example, an *Agrobacterium* strain. Necrosis is observed when injected leaf tissue has collapsed and cells died (see Klement & Goodman, Annual Review of Phytopathology 5 (1967) 17-44). Necrosis is distinguishable by one of ordinary skill in the art from yellowing a condition where there is no collapse of the leaf tissue and no extensive cell death.

As used herein, a "T-DNA border" refers to a DNA fragment comprising an about 25 nucleotide long sequence capable of being recognized by the virulence gene products of an *Agrobacterium* strain, such as an *A. tumefaciens* or *A. rhizogenes* strain, or a modified or mutated form thereof, and which is sufficient for transfer of a DNA sequence to which it is linked, to eukaryotic cells, preferably plant cells. This definition includes, but is not limited to, all naturally occurring T-DNA borders from wild-type Ti plasmids, as well as any functional derivative thereof, and includes chemically synthesized T-DNA borders. In one aspect, the encoding sequence and expression control sequence of an expression construct according to the invention is located between two T-DNA borders.

The term "vacuum infiltration", as used herein, relates to a method that allows the penetration of pathogenic bacteria, e.g. *Agrobacterium,* into the intercellular or interstitial spaces. Physically, the vacuum generates a negative atmospheric pressure that causes the air spaces between the cells in the plant tissue to decrease. The longer the duration and the lower the pressure, the less air space there is within the plant tissue. A subsequent increase in the pressure allows the bacterial suspension used in the infiltration to relocate into the plant tissue, and causes the *Agrobacterium* cells to contact the plant cells inside the plant tissue..

As used herein, "level of transient expression" refers to the capacity to express at least about 250 microgram, at least about 500 microgram, at least about 750 microgram, at least about 1 mg, at least about 2 mg, at least about 3 mg, at least about 4 mg, at least about 5 mg, at least about 10 mg, at least about 15 mg, at least about 25 mg, at least about 50 mg, at least about 75 mg, at least about 100 mg, at least about 150 mg, at least about 200 mg, at least about 500 mg, at least about 1000 mg, at least about 1.5 g, at least about 2 g, at least about 2.5 g, at least about 5 g, at least about 7.5 g, at least about 10 g, at least about 15 g, or at least about 20 g per kg of plant tissue mass.

As used herein, "transient" refers to a period of time that is long enough to permit isolation of protein from a suitable plant tissue. Preferably, protein expression is at suitably high levels within at least about 1 day, at least about 2 days, at least about 3 days, at least about 4 days, at least about 5 days, at least about 6 days, at least about 7 days, at least about 8 days, at least about 9 days, at least about 10 days, at least about 11 days, at least about 12 days, at least about 13 days, at least about 14 days, or at least about 15 days after introduction of the expression construct into plant tissue. In one aspect, suitably high levels are obtained within 3-7 or 5-10 days and more preferably within 3-5 or 5-7 days, after introduction of an expression construct into the plant tissue.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a subject and includes: (a) preventing a disease related to an undesired immune response from occurring in a subject which may be predisposed to the disease; (b) inhibiting the disease, i.e. arresting its development; or (c) relieving the disease, i.e. causing regression of the disease.

A "patient" or "subject" for the purposes of the present invention is used interchangeably and meant to include both humans and other animals, particularly mammals, and other organisms. Thus, the methods are applicable to both human therapy and veterinary applications. In the preferred embodiment the patient or subject is a mammal, and in the most preferred embodiment the patient or subject is a human.

The term "attenuation" as used herein refers to reduction of a viral infection in a subject or in a tissue of a subject, i.e. reduction or clearance of the amount of virus or viral load. The particular degree or level of the reduction or clearance is at least 15%, 25%, 35%, 50%, 65%, 75%, 80%, 85%, 90%, 95%, 98% or more.

The term "adjuvant" as used herein refers to a substance that increases or promotes the ability of an immunogen (i.e., antigen) to stimulate an immune response against the immunogen in the subject subjected to the immunogen. In particular embodiments, the adjuvant increases the immune response against the immunogen by at least 2, 3, 4, 5, 10, 15, 20, 30, 40, 50, 60, 75, 100, 150, 500, 1000-fold or more. In other embodiments, the adjuvant reduces the amount of immunogen required to achieve a particular level of immune response (cellular and/or humoral and/or mucosal), e.g., a reduction of at least 15%, 25%, 35%, 50%, 65%, 75%, 80%, 85%, 90%, 95%, 98% or more. An adjuvant can further be a substance that prolongs the time over which an immune response, optionally protective immune response, is sustained (e.g., by at least a 2-fold, 3-fold, 5-fold, 10-fold, 20-fold longer time period or more).

The expressions "pharmaceutical composition" and "therapeutical composition" are used herein interchangeably in the widest sense. They are meant to refer, for the purposes of the present invention, to a therapeutically effective amount of the active ingredient, i.e. the VLPs of formula (I) or a pharmaceutically acceptable salt thereof, optionally, together with a pharmaceutically acceptable carrier or diluent.

It embraces compositions that are suitable for the curative treatment, the control, the amelioration, an improvement of the condition or the prevention of a disease or disorder in a human being or a non-human animal. Thus, it embraces pharmaceutical compositions for the use in the area of human or veterinary medicine. Such a "therapeutic composition" is characterized in that it embraces at least one VLP of the invention, and optionally a carrier or excipient whereby the salt and the carrier and excipient are tolerated by the target organism that is treated therewith.

A "therapeutically effective amount" refers to that amount which provides a therapeutic effect for a given condition and administration regimen. In particular, "therapeutically effective amount" means an amount that is effective to prevent, alleviate or ameliorate symptoms of the disease or prolong the survival of the subject being treated, which may be a human or non-human animal. Determination of a therapeutically effective amount is within the skill of the person skilled in the art.

The therapeutically effective amount or dosage of a compound according to this invention can vary within wide limits and may be determined in a manner known in the relevant art. The dosage can vary within wide limits and will, of course, have to be adjusted to the individual requirements in each particular case.

An "immunogenically effective amount" refers to that amount of an immunogen which provides an active immune response (cellular and/or humoral) in a subject. In some embodiments of the invention, said immune response is sufficient to provide a protective effect, which does not need to be complete or permanent. Determination of an immunogenically effective amount is within the skill of the person skilled in the art.

An "adjuvant effective amount" refers to that amount of an adjuvant that enhances or stimulates the active immune response (cellular and/or humoral or optionally an active mucosal immune response) provided by the immunogen in a subject when subjected to the immunogen.

In the context of protective immune responses, the term "adjuvant effective amount" refers to an amount of the adjuvant that is needed to accelerate the induction of the immune response in the host and/or may be sufficient to reduce the need for booster immunizations to achieve protection.

In the context of prolongation of an immune response, the term "adjuvant effective amount" refers to an amount that prolongs the time period over which an immune response, optionally protective immune response, is sustained.

Determination of an adjuvant effective amount in the above addressed contexts is within the skill of the person skilled in the art.

It has previously been reported that a diversity of viral antigens such as HBsAg, NVCP, HPV11-L1 or HPV16-L1 have the capability to assemble nanoparticles in different plant systems including tobacco, potato, lupine, lettuce, tomatoes, soybean, *N. bentaminiana.* These so-called virus-like particles are composed of a lipid bilayer comprising various fatty acids (*i*.*e*. phospholipids) and sterols.

It was further shown that expression of a full length recombinant hemagglutinin protein in *N. bentaminiana* leads to the formation of artificial virus-like particles with a bilayer membrane, wherein said VLPs have a size distribution in the range of between 100 nm and 300 nm. The lipid bilayer of the virus-like particles produced in *N. benthamiana* has equal amounts of fatty acids and sterols.

Within the scope of the present invention ft was now surprisingly found that recombinant influenza virus antigens such as hemagglutinin produced in *Nicotiana tabacum* plants *in vivo* or *in vitro* are capable of assembling VLPs and that these *N. tabacum* produced VLPs have improved properties.

The hemagglutinin is, in one embodiment of the Invention, an influenza type A or an Influenza type B hemagglutinin, or an Influenza sub-type A hemagglutinin selected from the group consisting of H1, H2, H3, H4, H5, H6, H7, H8, H9, H10 H11, H12, H13, H14, H15, and H16.

In a specific embodiment of the invention, the hemagglutinin is of the H5 sub-type.

The VLPs produced in *Nicotiana tabacum* plants or plant cells displaying hemagglutinin have a favorable size distribution of approximately 50-200 nm. Small particles of 20-200 nm have been found to be associated with draining lymph node dendritic cells and macrophages whereas larger particles typically are only associated with dendritic cells from the injection site (Manolova et al. (2008) Nanoparticles target distinct dendritic cell populations according to their size. Eur. J. Immunol. 38: 1404-1413) making the smaller particles produced in *N. tabacum* more immunogenic.

The VLPs produced In *Nicotiana tabacum* cells also differ in the composition of the lipid bilayer compared to those produced in a *N. benthamiana* cell. The lipid bilayer of virus-like particles produced in *N. tabacum* cells has more fatty acids than sterols.

In particular, the *N. tabacum* produced virus-like particles were shown to contain low concentrations of monounsaturated fatty acids, particularly of C₁₈ monounsaturated fatty acids such as, for example, oleic acid, which is present in the lipid bilayer in a concentration of less than 0.4 µg/mL, less than 0.5 µg/mL, less than 1.0 µg/mL, or less than 1.5 µg/mL when determined according to GC-MS or LC-MS.

The lipid bilayer of the VLPs of any of the preceding paragraphs may have a content of C₂₀ saturated fatty acids, particularly of arachidic acid, greater than 0.5 µg/mL, greater than 0.75 µg/mL, greater than 0.9 µg/mL, or greater than 1.0 µg/mL when determined according to GC-MS or LC-MS.

Further, the *N. tabacum* produced virus-like particles were shown to contain increased concentrations of polyunsaturated fatty acids, particularly of C₁₈ polyunsaturated fatty acids such as, for example, linoleic acid and/or linolenic acid, which is present in the lipid bilayer in a concentration of between 3.0 µg/mL and 5.0 µg/mL when determined according to GC-MS or LC-MS.

Polyunsaturated fatty acids such as linoleic acid are essential for the proper functioning of the immune system and have been implicated in regulating cytokine responses (Fritsche, K (2006) "Fatty acids as modulators of the immune response". Annu. Rev. Nutr. 26: 45-73).

Furthermore, the lipid bilayer of the virus-like particles produced in *N. tabacum* plants or plant cells have an improved cholesterol:sitosterol ratio, which turned out to be >1, when determined by LC-APPCI-MS/MS. Hence, when compared to VLPs produced In *N. benthamiana*, the lipid bilayer of *N. tabacum* VLPs contain approximately 50% more cholesterol, but only half the concentration of sitosterol.

Cholesterol decreases the fluidity and permeability of bilayer membranes leading to increased mechanical stiffness while keeping the membrane fluid (Raffy and Teissie (1999) "Control of lipid membrane stability by cholesterol content" Biophys. J. 76: 2072-2080; Crane and Tamm (2004) "Role of cholesterol in the formation and nature of lipid rafts in planar and spherical model membranes" Biophys. J. 86: 2965-2979). The higher cholesterol content of particles derived from *N*. *tabacum* results in lesser permeability and hence more stable particles with less plant cell proteins contained within the cavity of the particle. Without being bound by this theory, the much higher percentage of cholesterol in particles from N*. tabacum* compared to *N*. *benthamiana* suggests a different organellar origin more resembling mammalian plasma membrane.

The hemagglutinin displayed by the virus-like particles, particularly by particles produced by expressing a recombinant hemagglutinin encoding nucleotide sequence in *N. tabacum* cells, may be S-acylated.

Alternatively, the hemagglutinin displayed by the virus-like particles, particularly by particles produced by expressing a recombinant hemagglutinin encoding nucleotide sequence in *N. tabacum* cells, may be N-glycosylated; or the hemagglutinin displayed by the virus-like particles produced by expressing a recombinant hemagglutinin encoding nucleotide sequence In *N. tabacum* cells may be S-acylated and N-glycosylated.

The hemagglutinin may be an Influenza type A or an Influenza type B hemagglutinin, or an Influenza sub-type A hemagglutinin selected from the group consisting of H1, H2, H3, H4, H5, H6, H7, H8, H9, H10 H11, H12, H13, H14, H15, and H16.

In particular, the hemagglutinin is of the H5 sub-type.

Further disclosed herein is a VLP composition comprising VLPs displaying an influenza hemagglutinin 5 polypeptide (H5), particularly an influenza hemagglutinin 5 polypeptide (H5) as shown in SEQ ID NO: 8, produced in a selected *N. tabacum* variety, breeding line, or cultivar

In the H5 hemagglutinin produced in *N. tabacum* cells only the two cytoplasmic cysteines are post-translationally modified by acylation with palmitic acid. Accordingly, the *N. tabacum* cell produced H5 hemagglutinin as disclosed herein has 2 palmitoyl (palmitate) chains only. This is in contrast to mammalian produced hemagglutinin H5, which has two cytoplasmic and one transmembrane cysteine S-acylated with a mixture of palmitate and stearate.

The herein disclosed tobacco produced H5 hemagglutinin is thus remarkably different from that produced in mammalian systems in that the transmembrane cysteine is not acylated and no stearate (C18) is detected in the acylated protein.

S-acylation greatly increases membrane affinity and is typical for proteins that cycle on and off membranes or in and out of lipid microdomains (see review Hemsley & Grierson (2008) "Multiple roles for protein palmitoylation in plants". Trends In Plant Sci. 13: 295-302). The exclusive presence of palmitate indicates that hemagglutinin in *N. tabacum* partitions into microdomains composed of shorter lipids. In this invention palmitoylation is observed for all *N. tabacum* produced hemagglutinin C-terminal peptides and no peptide is detected having no S-acylation/palmitoylation. S-acylation is catalyzed by Protein S-Acyl transferases. *N. tabacum* has 5 putative S-Acyltransferases whereas *N. benthamiana* only has 2 suggesting that S-acylation is more likely to occur in *N. tabacum* increasing the likelyhood of more stable membrane complexes of hemagglutinin in *N. tabacum* compared to *N. benthamiana* (Hemsley (2009) "Protein S-acylation in plants" (Review). Molecular Membrane Biology 26: 114-125).

The hemagglutinin of influenza virus has N-glycosidic carbohydrate side-chains which vary in number and structure over a wide range among the different hemagglutinin types and sub-types. These oligosaccharides, which are attached to specific sites on the hemagglutinin molecule are capable of interfering with antibody binding, receptor binding, proteolytic activation, and trimer assembly and thus have significant influence on the modulation of the biological properties of the different hemagglutinins.

The hemagglutinin displayed by the virus-like particles produced by expressing a recombinant hemagglutinin encoding nucleotide sequence in *N. tabacum* cells is also N-glycosylated. The glycosidic carbohydrate side-chains of hemagglutinin displayed on *N. tabacum* produced virus-like particles are in general more simple compared to, for example, those on VLPs produced in *N. benthamiana.*

Accordingly, the ratio of simpler N-glycosylated hemagglutinin to fully glycosylated and Lewis-type hemagglutinin is higher for *N. tabacum* produced hemagglutinin compared to hemagglutinin produced in *N. benthamiana.* As a result of the simple N-glycosylation pattern of *N. tabacum* produced hemagglutinin, the *N. tabacum* hemagglutinin exposes a higher amount of protein backbone sequences comprising conserved epitopes.

In summary, the *N. tabacum* produced hemagglutinin is glycosylated at positions Asn 11, Asn 23, Asn 154, Asn 165, Asn 286 and Asn 484, wherein, on average, the major N-glycyan at position Asn 154 has a single GlcNAc, at position Asn 286 two GlcNAcs and at position 484 no GlcNAc at the reducing end.

N-glycosylation at positions Asn 11, Asn 23, Asn 154, Asn 165 and Asn 286 shows a simple structure in *N. tabacum* produced hemagglutinin compared to *N. benthamiana* hemagglutinin, which has some to many Lewis-type N-glycans at positions 11 and 286, respectively..

Especially at positions Asn 154 and 165, hemagglutinin produced in *N. tabacum* displays simpler structures compared to *N. benthamiana-*derived hemagglutinin, and exposes mannose residues. Mannose residues can be found on carbohydrates displayed on the surface of many pathogens including influenza A virus, where they function as ligands for endogenous mannose-binding lectin ('MBL) receptors of the innate immune system inducing pro-inflammatory responses by recruiting and activating macrophages (for review see Marth & Grewal (2008) "Mammalian glycosylation in immunity" Nat. Rev. Immunol. 8: 874-887). Many natural influenza A viruses display high-mannose structures at position Asn 165 (see Ward et al. (1980) "Carbohydrate composition of the oligosaccharide units of the hemagglutinin from the Hong Kong influenza virus A/Memphis/102/72" Biochem. J. 189: 649-652) suggesting that a *N. tabacum*-derived hemagglutinin more resembles the "natural" hemagglutinin configuration of the influenza virus itself making it a better Immunogen for vaccine purposes (compared to *N. benthamiana*)*.*

The VLPs of the preceding paragraphs may be produced in *Nicotiana tabacum* plants or plant cells *in vivo* or *in vitro.*

The VLPs of the preceding paragraphs may be produced in *Nicotiana tabacum* plants or plant cells by incubating said *Nicotiana tabacum* plants or plant cells with *Agrobacterium* cells comprising a binary vector, particularly a minimally-sized binary vector comprising sequence elements, which are essential for maintenance and replication of the plasmid in *Escherichia coli* and *Agrobacterium* cells, and for the transfer of the T-DNA to a tobacco plant cell, and further a T-DNA region, comprising the coding sequence of one or more influenza antigens, particularly an influenza hemagglutinin or an Immunogenic fragment thereof, that is (are) under control of regulatory elements functional in a *Nicotiana tabacum* plant and, optionally, a plant selectable marker gene, wherein the essential sequence elements accounts for at least 60%, 65%, 70%, 75%, 80% of the entire minimally-sized binary vector.

A minimal binary vector may be used comprising, consisting of, or consisting essentially of the following nucleic acid elements:
a) a first nucleic acid element comprising a nucleotide sequence encoding a selectable marker which is functional in *Escherichia coli* and *Agrobacterium* species;
b) a second nucleic acid element comprising a nucleotide sequence of a first origin of replication which is functional in *Escherichia coli*;
c) a third nucleic acid element comprising a nucleotide sequence encoding a replication initiator protein;
d) a fourth nucleic acid element comprising a nucleotide sequence of a second origin of replication, which is different from the first origin of replication and which is functional in *Agrobacterium*; and
e) a fifth nucleic acid element comprising a nucleotide sequence of a T-DNA region comprising a T-DNA right border sequence and a T-DNA left border sequence of a tumour-inducing *Agrobacterium tumefaciens* plasmid or a root-inducing plasmid *of Agrobacterium rhizogenes*;
   wherein the above nucleic acid elements are provided on a circular polynucleotide molecule and are separated by gap nucleotide sequences which have no function in replication, maintenance or nucleic acid transfer, and wherein said gap nucleotide sequences account for less than 20%, 25%, 30%, 35%, 40%, 45%, of the total vector size. Preferably, the gap nucleotide sequences account for less than 20% of the total vector size.

The expressible nucleotide sequence encoding an influenza antigen, particularly an influenza hemagglutinin or an immunogenic fragment thereof, can be cloned in a minimally-sized binary vector comprising sequence elements which are essential for maintenance and replication of the plasmid in *Escherichia coli* and *Agrobacterium* cells, and for the transfer of a T-DNA to a tobacco plant cell, and, optionally, a plant selectable marker gene, wherein the proportion of the essential sequence elements accounts for at least 70% of the nucleotides of the entire minimally-sized binary vector without the expressible nucleotide sequence encoding the influenza antigen.

The vector molecule for use in the composition of the VLPs according to the invention may have a total size of less than 6'000 bp, particularly of less than 5'500 bp, particularly of less than 5'200 bp, particularly of less than 5'100 bp, but especially 5139 bp.

Said minimal binary vector is based on the broad host range plasmid pRK2.

The minimally sized binary vector may have a sequence as shown in SEQ ID NO: 1.

In another aspect, the regulatory sequences operable in plants controlling the expression of the influenza antigen, particularly the influenza hemagglutinin or an immunogenic fragment thereof, comprise a promoter, particularly one of the promoters as disclosed herein below, but particularly a HT-CPMV promoter as such, particularly a HT-CPMV promoter or combined with the minimal 35S CaMV promoter as shown In SEQ ID NO: 2.

Optionally, the regulatory sequences include a 5' non-translated leader sequence, a polyadenylation signal, or one or more enhancers, or a combination of the foregoing. Further contemplated herein are other regulatory sequences as known by those skilled in the art. and as disclosed herein below including a suppressor of gene silencing.

Hence, the binary vector may comprise the expressible nucleotide sequence encoding the influenza antigen, particularly the influenza hemagglutinin or an immunogenic fragment thereof, and further the coding sequence of a suppressor of gene silencing operably associated with regulatory elements that operable in the tobacco plant.

The suppressor of gene silencing may be of viral origin, and particularly a suppressor of gene silencing selected from the group consisting of the p19 protein of cucumber necrotic virus (CNV), the p1 protein of rice yellow mottle virus (RYMV), the p25 protein of potato virus X (PVX), the AC2 protein of African cassava mosaic virus (ACMV), the 2b protein of cucumber mosaic virus (CMV) and the helper-component proteinase (HcPro) of a potyvirus.

In one aspect, the VLPs of the preceding paragraphs are produced in *Nicotiana tabacum* plants comprising the steps of:
i) providing a combination of a selected variety, breeding line, or cultivar of a *Nicotiana tabacum* plant and a selected strain of an *Agrobacterium* species, which variety, breeding line, or cultivar, exhibits less than 10% necrosis, less than 5% necrosis, less than 2% necrosis, less than 1% necrosis, 5 days after leaves of said variety, breeding line, or cultivar have been injected by syringe with the selected *Agrobacterium* strain at a cell density of OD₆₀₀ of 0.32;
ii) infiltrating a whole plant of the selected variety, breeding line, or cultivar of *Nicotiana tabacum* with a suspension of the selected strain of the *Agrobacterium* species comprising a binary vector, particularly a minimally-sized binary vector comprising sequence elements, which are essential for maintenance and replication of the plasmid in *Escherichia coli* and *Agrobacterium* cells, and for the transfer of the T-DNA to a tobacco plant cell, and further a T-DNA region, comprising the coding sequence of one or more influenza antigens, particularly an influenza hemagglutinin or an immunogenic fragment thereof, that is (are) under control of regulatory elements functional in a *Nicotiana tabacum* plant and, optionally, a plant selectable marker gene, wherein the essential sequence elements accounts for at least 60%, 65%, 70%, 75%, 80% of the entire minimally-sized binary vector, at an OD₆₀₀ of between 0.1 and 4.0, said strain comprising an expressible nucleotide sequence encoding the polypeptide under control of regulatory sequences operable in plants;
iii) Incubating the infiltrated plant for a period of between 5 days and 20 days, particularly between 7 days and 15 days, but especially between 8 days and 10 days, under conditions that allow expression of the expressible nucleotide sequence In the infiltrated plant and accumulation of the heterologous polypeptide.

Further contemplated herein is the use of a second suspension of *Agrobacterium* cells comprising a binary vector comprising the coding sequence of the suppressor of gene silencing for infiltrating said selected variety, breeding line, or cultivar of a *Nicotiana tabacum* plant. The second suspension of *Agrobacterium* cells can optionally be of the same strain as the selected *Agrobacterium* strain. The first suspension and second suspension of *Agrobacterium* cells can be infiltrated in any sequence or simultaneously. The first suspension and second suspension of *Agrobacterium* cells can be mixed prior to being used to infiltrate the tobacco plant. Optionally, the first suspension and second suspension of *Agrobacterium* cells are mixed In a defined ratio of the number of cells from each suspension.

The suspension of *Agrobacterium* cells may be used in step (ii) for infiltration of the *Nicotiana tabacum* variety, breeding line, or cultivar has a cell density (OD₆₀₀) in the range of 0.15 to 0.35.

The VLPs of the preceding paragraphs may be produced in a *Nicotiana tabacum* variety, breeding line, or cultivar which is selected from the group consisting of *N. tabacum* accession PM016, PM021, PM92, PM102, PM132, PM204, PM205, PM215, PM216 or PM217 as deposited with NCIMB, Aberdeen, Scotland, or DAC Mata Fina, PO2, BY-64, AS44, RG17, RG8, HB04P, Basma Xanthi BX 2A, Coker 319, Hicks, McNair 944 (MN 944), Burley 21, K149, Yaka JB 125/3, Kasturi Mawar, NC 297, Coker 371 Gold, PO2, Wisliça, Simmaba, Turkish Samsun, AA37-1, B13P, F4 from the cross BU21 x Hoja Parado line 97, Samsun NN, Izmir, Xanthi NN, Karabalgar, Denizli and PO1.

In particular, the VLPs of the preceding paragraphs are produced in a *Nicotiana tabacum* variety, breeding line, or cultivar selected from the group consisting of *Nicotiana tabacum* line PM016, the seeds of which were deposited on 6 January 2011 at NCIMB Ltd, (an International Depositary Authority under the Budapest Treaty, located at Ferguson Building, Craibstone Estate, Bucksbum, Aberdeen, AB21 9YA, United Kingdom) under accession number NCIMB 41798; PM021, the seeds of which were deposited on 6 January 2011 at NCIMB Ltd. under accession number NCIMB 41799; PM092, the seeds of which were deposited on 6 January 2011 at NCIMB Ltd. under accession number NCIMB 41800; PM102, the seeds of which were deposited on 6 January 2011 at NCIMB Ltd. under accession number NCIMB 41801; PM132, the seeds of which were deposited on 6 January 2011 at NCIMB Ltd. under accession number NCIMB 41802; PM204, the seeds of which were deposited on 6 January 2011 at NCIMB Ltd. under accession number NCIMB 41803; PM205, the seeds of which were deposited on 6 January 2011 at NCIMB Ltd. under accession number NCIMB 41804; PM215, the seeds of which were deposited on 6 January 2011 at NCIMB Ltd. under accession number NCIMB 41805; PM216, deposited under accession number NCIMB 41806; and PM217, the seeds of which were deposited on 6 January 2011 at NCIMB Ltd. under accession number NCIMB 41807.

The *Agrobacterium* strain used in the production of the VLPs of the preceding paragraphs may be a strain of *Agrobacterium tumefaciens* selected from the group consisting of AGL1, EHA105, GV2260, GV3101 and Chry5, particularly *Agrobacterium* strain AGL1 or EHA105.

For the production of the VLPs as described herein in a *Nicotiana tabacum* plant, major parts of said plant including plant leaves and/or plant flowers and/or plant stem and/or plant roots, but particularly the entire plant, may be exposed *In situ* to a pressure that is lower than atmospheric pressure or a vacuum.

As an alternative, major parts of the *Nicotiana tabacum* plant including plant leaves and/or plant flowers and/or plant stem and/or plant roots, but particularly the entire plant, may be exposed *in situ* to a pressure that is higher than atmospheric pressure.

After infiltration, the *Nicotiana tabacum* plant may be incubated under daylight conditions for seven to nine hours per day, preferably eight hours per day in order to improve the level of transient expression of the heterologous protein.

In another aspect, the *Nicotiana tabacum* plant may be incubated in an up-right position or, in the alternative, in an inverted position.

In particular, the *Nicotiana tabacum* plant is incubated in an inverted position or under daylight conditions for seven to nine hours per day, or both.

In still another aspect, the *Nicotiana tabacum* plant may (a) prior to infiltration, grown at a density of at least 100 plants per square meter, or (b) after infiltration, at a density of at least 100 plants per square meter, or (c) prior to infiltration, grown at a density of at least 100 plants per square meter, and after infiltration, at a density of at least 100 plants per square meter.

After incubating the plant or plant tissue under suitable conditions that allow the expression construct to express the peptide antigen in a plurality of plant cells, the peptide can self-assembie into a VLP and the VLPs can be detected and quantified in the plant or plant part such as the plant organ or plant tissue or in the cells thereof. After harvesting, VLP isolation may be performed using methods routine in the art. For example, at least a portion of the biomass may be homogenized, and VLPs displaying recombinant peptide antigen extracted and further purified. Extraction may comprise soaking or immersing the homogenate in a suitable solvent. Purification methods include, but are not limited to, immunoaffinity purification and purification procedures based on the specific size of a peptide, protein or protein complex, electrophoretic mobility, biological activity, and/or net charge of the peptide or protein to be isolated, or based on the presence of a tag molecule in the protein. Characterization of the isolated peptide or protein can be conducted by immunoassay or by other methods known In the art. For example, peptides or proteins can be analyzed on SDS-PAGE gels by Western blotting, or by Coomassie blue staining when the peptide or protein is substantially purified.

VLPs produced by the herein disclosed methods may be used as pharmaceuticals, and can be expressed for their utility as nutraceuticals and cosmeceuticals, since these products are used for direct ingestion, injection or application (e.g., topical administration) to humans. The herein disclosed VLPs are also useful in the production of similarly regulated veterinarian products.

The VLPs as disclosed herein may be provided as such or in form of a composition, particularly a pharmaceutical composition. Said compositions may comprise additional medicinal agents, pharmaceutical agents, carriers, buffers, adjuvants, dispersing agents, diluents, and the like depending on the intended use and application.

Administration of the suitable (pharmaceutical) compositions containing the VLPs as disclosed herein, may be effected by different ways known to a person skilled in the art, e.g., by parenteral, subcutaneous, intraperitoneal, topical, transmucosal, transdermal, intrabronchial, intrapulmonary and intranasal, intraventricular, intraarticular, intrathecal, intravaginal, intratracheal, administration and, if desired for local treatment, intralesional administration. Parenteral administrations include intraperitoneal, intramuscular, intradermal, subcutaneous, intravenous or intraarterial administration. The herein disclosed compositions may also be administered directly to the target site, e.g., by biolistic delivery to an external or internal target site, like a specifically effected organ or a tumor.

The herein disclosed VLPs and the pharmaceutical compositions containing said VLPs, may be administered orally, and thus be formulated in a form suitable for oral administration, i.e. as a solid or a liquid preparation. Suitable solid oral formulations include tablets, capsules, pills, granules, pellets and the like. Suitable liquid oral formulations include solutions, suspensions, dispersions, emulsions, oils and the like. If formulated in form of a capsule, the herein disclosed composition comprise, in addition to the active compound and the inert carrier or diluent, a hard gelating capsule.

The herein disclosed VLPs may also, for example, be formulated as suppositories, containing conventional suppository bases for use in human or veterinary medicine or as pessaries, for example, containing conventional pessary bases.

Examples of suitable pharmaceutical carriers, excipients and/or diluents are well known in the art and include, but are not limited to, a gum, a starch (e.g. com starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

Pharmaceutically acceptable carriers for liquid formulations are aqueous or non-aqueous solutions, suspensions, emulsions or oils. Examples of non-aqueous solvents are propylene glycol, polyethylene glycol, and injectable organic esters such as ethyl oleate. Examples of oils are those of animal, vegetable, or synthetic origin, for example, peanut oil, soybean oil, olive oil, sunflower oil, fish-liver oil, another marine oil, or a lipid from milk or eggs.

Aqueous carriers include water, alcoholic/aqueous solutions, emulsions or suspensions, including saline and buffered media such as phosphate buffered saline solutions, water, emulsions, such as oil/water emulsions, various types of wetting agents, sterile solutions etc. Compositions comprising such carriers can be formulated by well known conventional methods. Suitable carriers may comprise any material which, when combined with the biologically active compound of the invention, retains the biological activity.

Preparations for parenteral administration may include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles may include sodium chloride solution, Ringer's dextrose, dextrose and sodium chloride, lactated Ringer's, or fixed oils. Intravenous vehicles may include fluid and nutrient replenishes, electrolyte replenishers (such as those based on Ringer's dextrose), and the like. Preservatives and other additives may also be present including, for example, antimicrobials, anti-oxidants, chelating agents, and inert gases and the like. In addition, the pharmaceutical composition of the present invention might comprise proteinaceous carriers, like, e.g., serum albumin or immunoglobulin, preferably of human origin.

The VLPs as described herein and the pharmaceutical compositions containing said VLPs may be administered topically to body surfaces and thus be formulated in a form suitable for topical administration. Suitable topical formulations include gels, ointments, creams, lotions, drops and the like. For topical administration, the compound of formula (I) is prepared and applied as a solution, suspension, or emulsion in a physiologically acceptable diluent with or without a pharmaceutical carrier.

The pharmaceutical compositions provided herein may also be administered as controlled-release compositions, i.e. compositions In which the active VLP is released over a period of time after administration. Controlled- or sustained-release compositions include formulation in lipophilic depots (e.g. fatty acids, waxes, oils). In another embodiment, the composition is an immediate-release composition, i.e. a composition in which all the VLPs or any additional active ingredient is released immediately after administration.

Further, the VLPs as described herein may or a composition comprising said VLPs may be administered admixed to food, functional food, drinks, medicinal food.

Further examples for suitable formulations are provided in WO 2006/085983.

These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

The VLPs as described herein may be used in human and veterinary medicine for treating humans and animals, including avians, non-human primates, dogs, cats, pigs, goats, sheep, cattle, horses, mice, rats and rabbits.

Suitable dosages of the VLPs as described herein will vary depending upon the condition, age and species of the subject, and can be readily determined by those skilled in the art The total dally dosages of the compound of formula (I) employed in both veterinary and human medicine will suitably be in the range 0,01-2000 mg/kg body-weight, preferably from 0,1-1000 mg/kg body-weight, preferably from 1-100 mg/kg and these may be administered as single or divided doses, and in addition, the upper limit can also be exceeded when this is found to be indicated. Such dosage will be adjusted to the individual requirements in each particular case including the specific compound(s) being administered, the route of administration, the condition being treated, as well as the patient being treated. However, the compounds can also be administered as depot preparations (implants, slow-release formulations, etc.) weekly, monthly or at even longer intervals. In such cases the dosage will be much higher than the dally one and has to be adapted to the administration form, the body weight and the concrete indication. The appropriate dosage can be determined by conducting conventional model tests, preferably animal models. In general, in the case of oral or parenteral administration to adult humans weighing approximately 70 Kg, a daily dosage of about 10 mg to about 10.000 mg, preferably from about 200 mg to about 1.000 mg, should be appropriate, although the upper limit may be exceeded when indicated. The daily dosage can be administered as a single dose or in divided doses, or for parenteral administration; it may be given as continuous infusion.

An effective dose of the VLPs depends at least on the nature of the condition being treated, toxicity, whether the compound(s) is being used prophylactically (lower doses) or against an active infection or condition, the method of delivery, and the pharmaceutical formulation, and will be determined by the clinician using conventional dose escalation studies. It can be expected to be from about 0.05 to about 30 mg/kg body weight per day. For example, for topical delivery the daily candidate dose for an adult human of approximately 70 kg body weight will range from about 1 mg to about 500 mg, generally between about 5 mg and about 40 mg, and may take the form of single or multiple doses or administration sites.

The VLP as the immunogen and the adjuvant can be co-adminlstered concurrently (e.g., within hours of each other) in the same or different composition and, in the latter case, by the same or different route. Alternatively, the adjuvant can be administered prior to or after administration of the immunogen (e.g., about 6, 12, 24, 36, 48, 72, 96 or 120 hours or more before or after administration of the immunogen).

Furthermore, it is envisaged that the herein disclosed pharmaceutical composition might comprise further biologically active agents, depending on the intended use of the pharmaceutical composition. These further biologically active agents may be e.g. antibodies, antibody fragments, hormones, growth factors, enzymes, binding molecules, cytokines, chemokines, nucleic acid molecules and drugs.

### EXAMPLES

The following examples are provided as an illustration and not as a limitation. Unless otherwise indicated, the present invention employs conventional techniques and methods of molecular biology, cell biology, recombinant DNA technology, plant biology, plant breeding and protein production, which are know to those skilled in the pertinent art.

### Example 1: Agroinfiltration of tobacco plants, cultivation of Infiltrated plants and harvesting of plant material

### 1.1 Agroinfiltration

This example describes various methods of infiltrating *Nicotiana tabacum* with *Agrobacterium* cells. Whole plant or plant tissue can be infiltrated with *Agrobacterium* assisted by vacuum, by high pressure or by a syringe without needle. Before infiltration, tobacco plants are grown In the greenhouse in rockwool blocks with 20 hours light period and 4 hours dark period, 26°C/20°C day/night and 70%/50% relative humidity (day/night). Plants are given fertilizer by sub-irrigation.

### 1.1.1 Composition of inoculum

*A.tumefaciens* or *A.rhizogenes* bacteria comprising a binary vector containing a T-DNA with the gene of interest under control of plant regulatory elements is grown up to an OD₆₀₀ >1.6 in YEB-medium comprising 2 g/L Beef extract, 0.4 g/L Yeast extract, 2 g/L Bacto-Peptone, 2 g/L Sucrose, 0.1 g/L MgSO₄ and suitable antibiotics for selection of the respective *Agrobacterium* strain and binary vector, in an Erlenmeyer flask at 28°C and 250 rpm on a rotary shaker. The culture is then diluted 1:100 in fresh LB Broth Miller medium containing 10 mM 2-(N-morpholino) ethanesulfonic acid (MES) and suitable antibiotics and further grown at 28°C and 250 rpm on a rotary shaker up to an OD₆₀₀ >2. Bacteria are collected by centrifugation for 15 minutes at 8'000 g and 4°C. Pelleted bacteria are resuspended in infiltration solution containing 10 mM MgCl₂ and 5 mM MES (referred to herein as infiltration solution) at a final pH of 5.6, and OD₆₀₀ >2. Optionally, bacteria can be further diluted in infiltration solution and acetosyringone can be added to induce virulence. Optionally, a first *Agrobacterium* bacterial suspension prepared as described above, is mixed with a second *Agrobacterium* suspension harbouring a second binary vector with a second expressible gene. A non-limiting example of such a second gene is a coding sequence that encodes a suppressor of gene silencing. Optionally, inoculum can be stored for up to a week at 4-6°C before use.

### 1.1.2 Syringe infiltration

A syringe having the dimensions of a standard 2-ml syringe is filled with the bacterial infiltration solution and, without a needle, pressed against the abaxial side of a leaf. The piston is pushed down to force the entry of the bacterial suspension into the leaf tissue. This is repeated until the majority of the leaf surface is infiltrated. After infiltration, plants are kept in low light for a minimum of 8 hours and during the first day protected from full sunlight. The next day, plants are placed under normal light conditions until harvesting.

### 1.1.3 Vacuum Infiltration

Plants are infiltrated by immersion of the aerial parts in a 10 L beaker filled with a bacterial inoculum and exposing the whole of the infected plant or infected plant parts to greatly reduced atmospheric pressure (generally referred to herein as a vacuum). Vacuum infiltration is performed in a glass bell jar (Schott-Duran Mobilex 300 mm) using a V-710 Büchi pump connected to a V-855 regulator and the pressure is decreased from atmospheric pressure (1 bar) to 50 mbar in 3 to 4 minutes. Once reached, the vacuum in the bell jar is kept for 1 minute followed by a return to atmospheric pressure in approximately 2 seconds. Artificial lighting (80-100 µmol photon/cm²) is kept on during the whole infiltration process to ensure consistent light conditions. Following infiltration, plants are placed along with non-infiltrated control plants in the greenhouse until harvesting. Growth conditions such as fertilization, photoperiod and temperature are the same as used before infiltration. Water and fertilizer are administered to plants using a drip irrigation system.

### 1.2 Incubation of infiltrated tobacco plants in an inverted position.

It was surprisingly found that incubation of infiltrated tobacco plants in an inverted position leads to increased expression of recombinant protein. Infiltrated tobacco plants, especially when incubated at high densities in a greenhouse, tend to fall over because plants and plant stems cannot sustain the weight of the infiltrated leaves. A solution provided is by incubating the infiltrated plants upside down which results in a significant increase in recombinant protein production compared to tobacco plants incubated in normal upright position. Five to six week old plants of *N. tabacum* PM132, the seeds of which were deposited on 6 January 2011 at NCIMB Ltd. under accession number NCIMB 41802, grown in rockwool and all at approximately 28 cm in height, are vacuum-infiltrated as described before with cells of *Agrobacterium* strain Agl1 harboring either the plasmids pC91 that comprises the tGFP expression cassette or pC71 that comprises the H5 expression cassette

Gene construct pC91 is a pBINPLUS (Van Engelen et al. (1995) Transgenic Research 4: 288-290) derivative binary vector comprising the TurboGFP (tGFP) green fluorescent protein gene of Evrogen cloned under the control of the cauliflower mosaic virus 35S promoter and HT-CPMV sequence and the NOS terminator sequence. pC71 is as described before. Tobacco plants are grown and vacuum-infiltrated with cells of *Agrobacterium* strain Agl1 harboring either the plasmids pC91 or pC71, as described above. All tobacco plants are co-infiltrated with pC120 that comprises an expressible HcPro suppressor of gene silencing as described above

All the tobacco plants are also co-infiltrated with pC120 that comprises an expressible HcPro suppressor of gene silencing as described above.

Immediately after infiltration, infiltrated plants are incubated upside-down while illumination is provided from a position above the plants in a greenhouse. At 4 and 6 days after infiltration, 4 plants from each treatment group are harvested and three leaf disks per plant are used to measure tGFP expression. Leaf discs are frozen under liquid nitrogen and ground to a fine powder in a 2 ml eppendorf tube and extraction of tGFP is performed as described above. For the quantification of tGFP expression, 5µL of each of the extracts is diluted to 200µL and fluorescence is measured as described. Three replicates are made.

Plants did not show symptoms of water stress. A few days after hanging the plants upside-down, the stems of plants bend towards the light shone from a position higher above, forming a hook-like structure. tGFP expression and fluorescence is on average two times higher for plants incubated in an inverted position (that is, upside-down) as compared to the normally treated plants that are incubated in an upright position.

### 1.3 High density of growth

This example describes the surprising effect of growing tobacco plants before infiltration at a high density on the expression of tGFP and recombinant H5. The experiment shows that two tobacco varieties PM132 and PM217, seeds of which have been deposited with NCIMB when grown at high density prior to infiltration, remarkably produced approximately 40% more tGFP and 70% more recombinant H5, on a weight-of-infiltrated biomass basis relative to plants grown at lower densities when incubated under normal upright conditions.

### 1.3.1 Plants and infiltrations

To study the effects of growing tobacco at different planting densities, *Nicotiana tabacum* PM132, the seeds of which were deposited on 6 January 2011 at NCIMB Ltd. under accession number NCIMB 41802 and PM217, the seeds of which were deposited on 6 January 2011 at NCIMB Ltd. under accession number NCIMB 41807, plants are grown at two densities: 25 and 100 plants per square meter. At day 46 after sowing, plants are vacuum infiltrated with *A. tumefaciens* strain Agl1 comprising either pC91 containing the tGFP expression cassette or pC71 containing the H5 expression cassette as described before, and each together with *A. tumefaciens* strain Agl1 comprising pC120 containing the HcPro suppressor of gene silencing, as described before. The final OD₆₀₀ used in infiltration is 0.32. For plants grown at both densities, the average plant height, diameter of stomata, chlorophyll content, leaf thickness and water content are measured before infiltration. Plants grown at high densities are larger, have less chlorophyl and thinner leaves, but the diameter of stomata is the same. For PM132, the water content is the same for plants grown at low or high density but for PM217, the water content is much lower when grown at low density. Following infiltration, half of the plants are incubated upright or inverted (upside-down). Measurements are performed on a total of 9 (nine) plants per treatment spread over three replicates of each 3 plants.

Leaves are harvested 5 days post infiltration, an extract is prepared and analysis of tGFP expression are performed, as described above. The extract is diluted 1:1 in GFP buffer and 5µL is mixed with 200µL of GFP buffer for fluorescence quantification. Expression of tGFP in mg/kg fresh weight leaf biomass for both varieties when incubated in normal upside position, is on average 41% higher for plants grown at high density prior to infiltration when compared to plants grown at low density. Similar results are obtained for H5 which showed an average increase of expression when grown at high density between 21-40% when compared to plants grown at low density and incubated in normal upside position post infiltration. To study the effects of inverted incubation on expression of tGFP and H5, half of the infiltrated plants are incubated in an inverted position after infiltration. There is a significant increase of expression of both tGFP and H5 for plants incubated in an inverted position and grown at low density but much less increase of expression for plants grown at high density prior to infiltration. For PM132 plants grown at low density in an inverted position, it results in an increase of 40% of tGFP and 71% of H5. For plants grown at high density prior to infiltration, the increase is only 3% for tGFP and 7% for H5, compared to plants incubated in a normal upright position. Total soluble protein (TSP) is established and the expression of tGFP and H5 is estimated relative to TSP. The expression of tGFP and H5 is always higher for plants grown at high density and incubated in an inverted position. In an upright position, plants grown at low density had an increase of tGFP expression of approximately 10.5%. For plants grown at high density, the increase is approximately 13.6%. For H5, the increase in yield for plants incubated in upright position and grown at low density prior to infiltration is 0.25%, whereas the increase in yield for plants grown at high density is 0.37%. For both tGFP and H5, incubation in an inverted position resulted in an increase of expression (as measured in percentage of TSP). For plants grown at low density, incubation at an inverted position resulted in an increase of biomass by 40-60%. For plants grown at high density, incubation in an inverted position resulted in an increase of 20%. For tGFP the highest protein yield is 16.2% and is obtained when the plants are grown at high density before infiltration and are incubated in an inverted position. This represented an increase of 54% in protein yield in terms of %TSP compared to plants grown at low density and incubated in normal upright position (10.5% of TSP).

### 1.4 Harvesting and material sampling.

Sampling can commence after 16 hours but typically infiltrated leaves or infiltrated areas of a leaf are harvested after 6 days of incubation in the greenhouse. Optionally, infiltrated leaves or infiltrated areas of a leaf, are harvested after 10 days of incubation. Leaf material is placed in a heat-sealable pouch, sealed and placed between layers of dry-ice for at least 10 minutes. After harvesting, all leaf samples are stored at -80°C until further processing. Harvested leaves are homogenized to a fine powder using a coffee-grinder on dry-ice, extracted by (i) two steps of vortexing for 20 seconds each in 3 vol/wt extraction buffer containing 50 mM Tris base, 100 mM NaCl, 1 mM EDTA, 0.2% Triton X-100, final pH 7.5, and (ii) by centrifugation at 20,000 g for 15 minutes. Soluble extracts are kept on ice until analysis.

### Example 2: Binary vectors for transient expression

This example describes binary vectors and the design and development of pC100, which are used in this application. Binary vectors of this application are used for the expression of hemagglutinin in plant cells resulting in the formation of virus-like particles. Non-limiting examples of such an hemagglutinin can be hemagglutinin H5 of influenza strain A/Indonesia/05/2005 (H5N1) or H1 of influenza strain A/California/04/2009 (H1N1).

### 2.1 Construction of T-DNA region and backbone fragment of pC100.

The nucleotide sequence of the multi-copy binary vector pBIN61 (Bendahmane et al., 2000. Plant Journal 21: 73-81) of about 13,500 basepairs in length is analysed for nucleic acids having a function in replication, maintenance, selection of transgenic cells and transfer of T-DNA. A new nucleotide sequence is developed only comprising nucleic acids having a function as described above. The resulting nucleotide sequence is chemically synthesized in two parts. A first fragment containing the T-DNA region bordered by a T-DNA right (RB) and T-DNA left (LB) border sequence, the plant selectable kanamycin resistance (nptII) gene of pBIN61 under control of a nopaline synthase (pNOS) promoter and tNOS terminator and unique Stul, Ascl and EcoRI restriction site is chemically synthesized with flanking Pvull restriction sites and cloned in the Pvull site of the pUC-derived pMK vector (Geneart, Regensburg, Germany) which further contained a ColE1 replication of origin (Col E1 ori) and bacterial kanamycin resistance gene (KmR), resulting in pGA13. A second fragment containing the backbone region with a ColE1 ori and minimal RK2 oriV origin of replication and gene coding for the RK2 derived TrfA replication initiator protein of pBIN61, is chemically synthesized with unique Ascl, Stul and Pvull restriction sites and cloned in the pUC-derived pMA vector (Geneart, Regensburg, Germany) which further contained an ampicillin (ApR) resistance gene, resulting in pGA14.

### 2.2 Design and development of pC100 binary vector.

The minimal plant selectable binary vector pC100 (SEQ ID NO:1) is made by combining two fragments, a first fragment and a second fragment that are *de novo* synthesized. The first fragment contains 1, a kanamycin drug resistance gene functional in *E. coli* and *Agrobacterium* and comprising the neomycin phosphotransferase III gene; 2, a ColE1 origin of replication; 3, a minimal oriV origin of replication (Kowalczyk L. et al., Molecular Microbiology, 2005, 57(5): 1439-1449); 4, the *trf*A1 gene of an IncP plasmid that activates the oriV (Kongsuwan K. et al., J. Bacteriology, 2006, 188(15): 5501-5509), and 5, unique *Asc*l and *Stu*l restriction endonuclease recognition sites at the extreme ends of the fragment for combining said fragment 1 and fragment 2 to generate the minimal binary vector pC100. The second fragment contains the T-DNA region that contained 6, a T-DNA left border sequence of an *Agrobacterium*; 7, a T-DNA right border sequence of an *Agrobacterium*; 8, optionally, a selectable marker gene for selection of a transgenic plant cell and comprising a neomycin phosphotransferase II gene under control of a nopaline synthase promoter and a nopaline synthase terminator sequence of an *Agrobacterium* tumefaciens nopaline plasmid; 9, an unique *Eco*RI restriction endonuclease recognition site for cloning of a foreign gene and located between 7, the T-DNA right border and 8, the selectable marker gene, and 10, unique *Asc*l and *Stu*l restriction endonuclease recognition sites at the extreme ends of the fragment for combining said fragment 2 and fragment 1 to generate the minimal binary vector pC100 (SEQ ID NO:1).

### Example 3: Transient expression of influenza virus-like particle

This example describes the transient expression of hemagglutinin in Nicotiana plant cells resulting in the formation of virus-like particles. Such hemagglutinin may be hemagglutinin H5.

### 3.1 Gene constructs.

The gene coding for the HcPro suppressor of gene silencing of tobacco etch virus (TEV P1-HcPro-P3; SEQ ID NO:4) isolate TEV7DA (GenBank: DQ986288.1) is cloned in the unique EcoRI site of pC100 to generate pC120. The coding sequence for HcPro is under the control of a double cauliflower mosaic virus 35S promoter, the 5' untranslated region of TEV7DA and the nopaline synthase terminator sequence. A sequence coding for segment 4 of hemagglutinin 5 (H5) of influenza H5N1 strain A/Indonesia/05/2005 (Genebank: EF541394) is placed under the control at the 5' end of a minimal cauliflower mosaic virus 35S promoter and 5' UTR of HT-CPMV (SEQ ID NO:2), and at the 3' end of the nopaline synthase terminator and 3' UTR of HT-CPMV (SEQ ID NO:3), and cloned in the unique EcoRI site of pC100 after first deleting the plant selectable kanamycin resistance gene of pC100 to obtain the pPMP1 minimal binary vector as shown in SEQ ID NO: 1.

Optionally, the coding sequence for hemagglutinin is placed under the control of a figwort mosaic virus (FMV) full length transcript (FLt) promoter or FMV-FLt promoter with double enhancer, as disclosed in US Patent 5,994,521 A; a peanut chlorotic streak virus (PCISV) FLt promoter or PCISV-FLt promoter with double enhancer as disclosed in US Patent 5,850,019A; a mirabilis mosaic virus (MMV) FLt promoter or MMV-FLt promoter with double enhancer as disclosed in US Patent 6,420,547B1 (MMV; SEQ ID NO:5); or MMV sub-genomic transcript (Sgt) promoter as disclosed in US Patent 6,930,182B1. Plants are grown as described in Example 1.

### 3.2 Plant material and Agrobacterium inoculum.

*Nicotiana tabacum* Burley 21, PM132, the seeds of which were deposited on 6 January 2011 at NCIMB Ltd. under accession number NCIMB 41802, and PM204, the seeds of which were deposited on 6 January 2011 at NCIMB Ltd. under accession number NCIMB 41803, and *Nicotiana benthaminiana* plants are grown in the greenhouse in rockwool blocks with 20 hours light period, 4 hours night period, under 26°C/20°C day/night temperature and 70%/50% day/night relative humidity. Gene constructs are introduced in *Agrobacterium* tumefaciens Agl1.

Bacteria are grown under conditions reported in Example 1.1 up to a final OD₆₀₀ of 3.5. *Agrobacterium* cultures containing the pC229 gene construct and pC120 suppressor of gene silencing construct are mixed at a 3:1 ratio and diluted to a OD600=0.8 in infiltration solution containing 10 mM MgCl₂ and 5 mM MES, final pH5.6. Alternatively, such cultures can be further diluted for infiltration.

### 3.3 Infiltration of Nicotiana plants and extraction of infiltrated plants.

### 3.3.1 Nicotiana tabacum:

Plants are infiltrated by decreasing air pressure to 900 mbar below atmospheric pressure within 15s, a 60s holding time followed by a return to atmospheric pressure in approximately 2s. Infiltrated *N. benthamiana* plants are incubated in the greenhouse for 5 days before harvesting.

Leaves of infiltrated plants are collected from 10 plants and kept overnight at 4°C in the dark. Leaves are homogenized using a screw press (Green Star Corrupad, GS 1000, Korea Co. or Vincent CP-4) at 15-20 Kg/hr using at least 45-psi pressure on the cone. The green juice extract is collected and sodium metabisulfite is added to the green juice to a final concentration of 10 mM to reduce sample oxidation. The pH of the extract is adjusted to pH5.3 and subsequently incubated at room temperature for 20-30 min without stirring. Celpure P300 (Sigma-Aldrich)(10%) is then added to the extract and mixed for 1 minute. The solution is filtrated through a Whatman filter paper pre-coated with Celpure P300 (10% Celpure P300 slurry in 10 mM sodium metabisulphite).

### 3.3.2 Nicotiana benthaminiana:

Influenza virus-like particles are produced in *N. benthamiana* plants as described in D'Aoust et al. (D'Aoust et al. (2008) Influenza virus-like particles produced by transient expression in Nicotiana benthamiana induce a protective immune response against a lethal viral challenge. Plant Biotechnology Journal 6 (2008) 930-940) using aforementioned gene constructs and cultures, or alternatively by decreasing air pressure to 900 mbar below atmospheric pressure within 15s, a 60s holding time followed by a return to atmospheric pressure in approximately 2s. Infiltrated *N. benthamiana* plants are incubated in the greenhouse for 6 days before harvesting.

Collection of leaves from infiltrated plants and leaf extraction is carried out as described above in Example 3.3.1.

### 3.4 Purification of Virus-Like Particles.

For ultracentrifugation, three sucrose cushions are prepared in ultracentrifuge tubes as follows: 1) 3ml of 80% sucrose; 2) 1.5 ml each of 60 and 45% sucrose; and 3) 1ml each of 60, 45 and 35% sucrose. Clarified and filtered extract samples (up to 13ml) are gently placed on top of the sucrose gradients and subjected to ultracentrifugation in a swinging bucket type rotor (Sorvall Surespin 630; Kendro) at 24,000 rpm for 1 hour at 4°C (135,000 RCFmax). Sucrose concentrated samples are pre-filtered using a 0.45 µm filter and subjected to size exclusion chromatography (SEC) under isocratic conditions on an automated AKTA chromatography system. The running buffer is TBS, pH 7.5 and sample size is 4 ml under a flow rate of 1 ml/min on a HiLoad 16/60 Superdex 200 column (GE Healthcare, 17-1069-01). Fractions containing purified H5 are pooled and concentrated to about 0.3 mg/ml using a 30kDa cut-off Centricon ultrafiltration membrane device (Millipore) and further analysed.

### 3.5 Gel electrophoresis and detection of H5 by Western blotting.

Samples of pooled fractions are directly subjected to SDS-PAGE, Western blotting and Blue Native-PAGE using standard techniques. Alternatively, leaves of the infiltrated plants are first ground to a fine powder using a coffee-grinder on dry-ice and extracted by (i) two steps of vortexing for 20 seconds each in 3 vol/wt extraction buffer containing 50 mM Tris base, 100 mM NaCl, 1 mM EDTA, 0.2% Triton X-100, final pH 7.5, and (ii) by centrifugation at 20,000 *g* for 15 minutes, to determine the presence of H5 in aforementioned infiltrated leaves. SDS-PAGE is on a 4-12% SDS-PAGE gel. As a positive control for H5, commercially available recombinant H5 of Immune Technology Corp. (New York, cat. #IT-003-0052p) is used. After separation, proteins are stained with Imperial M protein stain (Pierce #24615). For Western blotting, the primary antibody is a rabbit anti-HA antibody (H5N1 VN1203/04 #IT 003-005V, Immune Technology Corp., New York). For detection, an HRP-labelled affiniPure goat-anti-rabbit IgG FC-fragment is used (Jackson Laboratories, #111-035-046). Detection is done by chemiluminescence using an Immuno-star HRP Chemiluminescent Kit (BIO-RAD Laboratory, 170-5040). Results are captured using Chimio-Capt 3000 and show the presence of H5 in extracts of plants infiltrated with the pC229 gene construct and fractions after size-exclusion chromatography. The molecular weight is similar to that of commercial recombinant H5. Native-PAGE is performed on 4-16% Bis-Tris pre-cast polyacrylamide gels (Novex, Invitrogen, USA). For loading, samples are treated with digitonin in native polyacrylamide gel electrophoresis (PAGE) sample buffer and incubated for 1 h at 4°C. Subsequently, Native-PAGE G-250 sample additive (Novex, Invitrogen, USA) is added to a final concentration of 0.5% and samples are loaded and run on a 4-16% Bis-Tris PAGE gel. Gels are run at 4°C at 150V constant for the first 60 min. Subsequently, voltage is increased to 250V for another 30 min and gels are stained with Imperial M protein stain. Results of native-PAGE and Western blotting show the successful expression of H5 following transient expression in tobacco and *Nicotiana benthaminiana,* respectively.

### 3.6 Hemagglutination.

Hemagglutinin has the ability to bind to monosaccharide sialic acid which is present on the surface of erythrocytes in red blood cells. Hemagglutination can be used to determine the relative activity of a hemagglutinin protein and is used to determine the biological activity of recombinant H5 present in the virus-like particles in crude extracts of *N. tabacum* transiently expressing H5 as described before. Hemagglutinating activity of the tobacco-produced H5 present in the virus-like particles is measured by incubating 1.5-fold serial dilutions of the plant extract as well as fractions obtained after purification by size-exclusion chromatography, in a 96-well plate with a specific amount of red blood cells. Red blood cells not bound to H5 sink to the bottom of a well and form a precipitate and correctly folded trimeric H5 will bind erythrocytes. Hemagglutinating activity is observed in extracts and the fractions obtained after size exclusion chromatography of extracts of tobacco and *Nicotiana benthaminiana* plants, respectively, infiltrated with the pC229 gene construct.

### Example 4: Purification of Virus-Like Particles by ultracentrifugation over sucrose cushion

This example describes the purification of virus-like particles by sucrose-gradient centrifugation. Such virus-like particles can be obtained by expressing for example influenza hemagglutinin H5 or H1 in plant cells. Such plant cells can be cells of *N*. *tabacum* or *N. benthamiana.*

### 4.1 Isolation of Virus-Like Particles.

Hemagglutinin H5-Virus-Like Particles are produced in *N. benthamiana* or *N. tabacum* according to the present invention. Purification is performed according to the examples and procedures described in the present invention and the quality of the virus-like particles is checked using the panel of analytical tools outlined in Example 6 and 7. Virus-like particles are made in three different *N. tabacum* varieties, *N. tabacum* Burley 21, PM132, the seeds of which were deposited on 6 January 2011 at NCIMB Ltd. under accession number NCIMB 41802, and PM204, the seeds of which were deposited on 6 January 2011 at NCIMB Ltd. under accession number NCIMB 41803, and tested.

### 4.2 Homogenization and extraction.

Leaves of *N. benthamiana* and *N. tabacum* plants expressing hemagglutinin virus-like particles, are harvested six days after agro-infiltration and are kept overnight at 4°C in the dark. Plant biomass, including leaves and stems, is homogenized using a screw press (Green Star Corrupad, GS 1000, Korea Co.) and sodium metabisulphite is added to 10 mM final concentration to avoid sample oxidation.

### 4.3 Clarification and Filtration.

The crude juice obtained from the screw press is quickly adjusted to pH 5.3 using 20% diluted acetic acid. Extract is left at room temperature for 20-30 min without stirring for partial clarification. Filtration is performed through a Whatman filter paper pre-coated with 2 mm high Celpure P300 (10 % Celpure P300 slurry in MBS 10 mM). Vacuum is kept at 508 mbar. Celpure P300 (10%) is added to the extract and mixed for 5 minutes. Finally the extract is filtered gently adding extract-celpure slurry by portions (no more than 2 cm liquid phase over the filtration cake).

### 4.4 Purification by sucrose cushion.

A sucrose cushion of 3 ml is prepared in ultracentrifuge tubes (1.5 ml of 45% and 1.5 ml of 60% sucrose). Clarified and filtered extract samples (up to 13mL) are added on top of the sucrose cushion and subjected to ultracentrifugation into a swinging bucket type rotor (24,000rpm=135,000 RCFmax, 1 hour, 4°C and acceleration and deceleration profiles of 9, Sorvall Surespin 630 rotor, Kendro). The tubes are removed from the rotor and the sucrose is isolated from the remaining extract. Sucrose concentrated samples are pre-filtered using a 0.45µm filter and subjected to gel filtration chromatography under isocratic conditions on an automated AKTA chromatography system (running buffer TBS, pH 7.5, sample size 4 mL, flow rate 1 mL/min, HiLoad 16/60 Superdex 200 column GE Healthcare 17-1069-01) to remove sucrose. Fractions containing the purified H5-VLP are pooled and concentrated to about 0.3 mg/mL using a 30kDa cut-off Centricon ultrafiltration membrane device (Millipore). The purified H5-VLP samples are immediately aliquotted and stored at 4°C until required.

### Example 5: Alternative Virus-Like Particles purification protocol

This example describes an alternative protocol for purifying virus-like particles from extracts of infiltrated plant leaves according to methods described in this invention. This protocol describes the purification by homogenizing leaves in cold homogenization buffer instead of using a screw press.

### 5.1 VLP Purification protocol.

1. Harvest infiltrated leaves at 2 to 10 dpi and homogenize in cold homogenization buffer containing 50 mM Tris pH 8,50 mM NaCl, 0.04% Sodium metabisulfite, 1 mM PMSF in methanol buffered at pH 6 with acetic acid. Optionally a protease inhibitor can be added to the homogenization buffer.
2. Using a mortar and pestle, grind the harvested leaf tissue with a ratio of 1.5 mL homogenization buffer to 1 g of leaf tissue. Glass pipettes are added to the ground mixture to help lyse the plant cells. Squeeze the homogenate through two layers of cheesecloth.
3. Heat lysate @ 42°C for 5 min.
4. Add diatomeous earth to heat treated extract to absorb contamination.
5. Filter the resulting slurry through #1 Whatman filter paper.
6. Spin the homogenate for 10 min at 10,000 x g @ room temp.
7. Filter supernatant through 0.8 µm followed by 0.2 µm Corning Syringe Filters
8. To filtrate, add 60 uL of Fetuin Agarose per 1 mL of supernatant. Fetuin Agarose is first washed 3x in homogenization buffer before adding to the supernatant. Allow mixing for 30 min at room temperature.
9. Wash the column 3x with 10x the column volume using a wash buffer containing 400 mM NaCl, 25 mM Tris, buffered at pH 6.0, to remove unbound proteins and centrifuge at 2000 rpm for 5 minutes. Recover pellet.
10. Elute the proteins from the column with an equal volume of elution buffer containing 1.5 M NaCl, 50 mM MES, buffered at pH 6.0 to which 0.0005% (final concentration) Tween-80 is added. Elute at least 3 times.
11. Concentrate elutant on 10 kDa MWCO centrifuge tubes and store at 4°C on ice, or
12. Take 5.5 ml of 45% sucrose in elution buffer layered over 2.5ml of 60% sucrose in elution buffer. The sample is layered over the 45% sucrose.
   Optionally, steps 11 and 12 can be omitted.
13. Spin for 14 hr at 60,000 x g at 4°C.
14. Puncture the tube and collect the virus band using a syringe and a needle.
15. Store at 4°C in ice or in -80°C until next step.

### Example 6: Two step capture process for cleaning and capturing plant-derived Virus-Like Particles

This example describes a two-step cleaning and capture process for the capture of virus-like particles from plant extracts obtained according to methods described in this application. In a first step green materials are removed from the extract containing the virus-like particles. Such green material can clog and block columns used during chromatography to purify the virus-like particles.

### 6.1 Two step process.

A green plant juice extract is obtained upon homogenizing infiltrated and incubated leaves as described above and by using a screw press. The green plant juice is first treated in a pre-capture step because the green plant juice extract and especially any materials in such extract causing the green colour, favourably stick to most of the ligands used in chromatography and hence cause clogging and blockage of the column used during chromatography purification. The two step capture process for a plant-derived virus-like particle is set up to first clean the extract, the process comprising:
Step 1, binding of impurities and materials determining the green colour of a green plant juice extract in static binding/batch incubation mode using a polyethyleneimine (PEI) ion exchanger attached to a particle with a mean particle size of 150 µm, at pH 7;
Step 2, binding of virus-like particles from the supernatant of Step 1 in expanded bed adsorption mode using a diethylaminoethyl (DEAE) ion exchanger attached to a particle with a mean particle size of 50 µm, at pH 7.

### 6.2 Experimental set-up.

An *N. tabacum* extract of tobacco plants expressing virus-like particles as described and exemplified in this invention is prepared, filtered and diluted 5-fold with deionized water, before use. Final pH is set at pH 7 and total volume for incubation in Step 1 is 150 mL. Conductivity is 5.3 mS/cm. Equilibration of polyethyleneimine (PEI) adsorbent is performed with 5 bed volumes of 0.5 M Tris/HCl buffer pH 7, followed by 10 bed volumes of 10mM Tris/HCl buffer pH 7.0. The ratio of PEI adsorbent to green juice extract in static mode is 1:15. A total of 9.5 mL of PEI adsorbent is used. Incubation is for 30 min at room temperature and 140 mL of the supernatant is collected after incubation, for Step 2.

In Step 2, 120 mL supernatant of Step 1 is adjusted to pH 7.0 and run in expanded bed adsorption mode. Conductivity is 5.4 mS/cm and equilibration of the diethylaminoethyl (DEAE) ion exchanger used in expanded bed mode is with 5 bed volumes of 0.5 M Tris/HCl buffer pH 7, followed by 10 bed volumes of 10 mM Tris/HCl pH 7.0. A total of 12 mL of DEAE adsorbent is used. The expanded bed column is 1 cm in diameter with a column height of 70 cm and bed height is 15 cm. The ratio of DEAE adsorbent to sample is 1:10. The supernatant of Step 1 is loaded onto the column at a flow rate of 3.8 cm/min and after loading the column is washed with washing buffer containing 10 mM Tris/HCl pH 7.0. Bound material is eluted using an elution buffer containing 50 mM Tris/HCl and 1 M NaCl, pH9.0. Run through and wash fractions are collected in 25 mL volumes. The eluate is collected in one fraction and the pH is adjusted during elution to pH 7 by adding 1/10 volume of a 1 M Tris/HCl, pH 6.5 solution to the tube before collecting the eluate.

### 6.3 Detection of hemagglutinin.

The amount of hemagglutinin protein is estimated for the filtered *N. tabacum* green juice extract prior to entering Step 1, the supernatant after incubation in static mode, the flow through, the wash and the eluate after expanded bed mode adsorption and elution, as described before. Total protein content in extracts and fractions is estimated using standard protocols. The amount of hemagglutinin as measured using a hemagglutination assay in filtered green juice extract, starting material for incubation in static mode with a PEI ion exchanger, the supernatant derived upon incubation of extract with the PEI ion exchanger, the starting material for expanded bed adsorption using a DEAE ion exchanger, the run through and eluate upon elution of the expanded bed column, is given in Table 1 together with the total protein content of said fractions and samples. In this experiment most of the materials causing the green colour and clogging of the column are removed in Step 1 using the PEI adsorbent without loss of hemagglutinin protein and the virus-like particles comprising the hemagglutinin protein are captured during expanded bed adsorption mode using a DEAE ion exchanger.

**Table 1. Hemagglutination activity (% of initial extract) and total protein content (mg/mL) for the various extracts and fractions of Step 1 and 2. ND, not determined.**

| Fraction | Step 1 Diluted extract | Step 1 Start material PEI | Step 1 Supernatant after PEI | Step 2 Start material DEAE | Step 2 Run through + wash fractions | Step 2 Eluate from DEAE |
|---|---|---|---|---|---|---|
| Hemagglu tination (in %) | 100 | 100 | 100 | 100 | 0 | 67 |
| Total protein (in mg/mL) | 0.22 | 0.25 | 0.14 | 0.14 | ND | 0.06 |

### Example 7: Large scale capture of Virus-Like Particles using expanded bed chromatography

This example describes large-scale capture of virus-like particles comprising hemagglutinin produced in and extracted from plant extracts and using expanded bed chromatography at an industrial scale.

### 7.1 Extract.

Tobacco plants are infiltrated as described and following infiltration, are incubated in the greenhouse for 6 days before harvesting. Leaves of infiltrated plants are harvested and the biomass is kept overnight at 4°C in the dark and is homogenized using a screw press (Vincent CP-4) at 15-20 Kg/hr using at least 45-psi pressure on the cone. 250 kg crude leaf biomass containing hemagglutinin H5 virus-like particles is taken for further processing. Green plant juice is collected and sodium metabisulfite is added to the green juice to a final concentration of 10 mM. Conductivity is measured and the extract is diluted with water before use to a final conductivity of approximately 10. The extract is kept at room temperature. Total volume after dilution is approximately 1,250 L extract.

### 7.2 Column properties.

Bead composition: epichlorohydrin cross-linked agarose (4% w/v). Core: tungsten carbide (occupying approximately 10-15% of the volume of the bead). Ligand: DEAE ion exchanger. Mean diameter particles: 50 µm. Average density of adsorbent particle: 16 kg/L. Column diameter: 45 cm. Void volume in sedimented status: approximately 40% of packed volume. Total volume: 63 L adsorbent material is added to a 45 cm diameter column. The column is equilibrated with 5 column volumes of 0.5 M Tris-CI buffer, pH 7 followed by 10 column volumes of 10 mM Tris-Cl buffer, pH 7.

### 7.3 Loading.

Loading is performed by pumping upward the 1,250 L extract at a flow rate of 450 cm/h, equivalent to 653 L/hr. The expansion factor is kept at 2.0-2.5 and unbound material is washed out with 10 mM Tris/HCl, pH 7 at the same expansion rate.

### 7.4 Elution.

Influenza virus-iike particles are recovered by elution with 50 mM Tris-Cl and 1M NaCl, pH9 at an expansion rate of 1.2. Eluted fractions are neutralized by adding 1/10 volume of 1M Tris/HCl, pH 6.5 to the container before collecting the elution peak. Fractions containing virus-like particles comprising hemagglutinin as measured according to methods of the present invention, are pooled for further capture and purification.

### 7.5 Performance monitoring.

Chromatography performance is monitored by measurement of UV absorption at 280 nm and 600nm, conductivity and pH. The capture of virus-like particles containing hemagglutinin H5 is quantitatively measured by ELISA and hemagglutination as described in Example 2 or according to D'Aoust et al. (2008, supra). A Bradford assay is used to determine total protein content of fractions. SDS-PAGE is used to establish purity and Western blotting for identification and characterization using hemagglutinin H5-specific antibodies as described in Example 2 and by D'Aoust et al. (2008, supra).

### Example 8: Quantitative microscopic measurements of particle morphology

This example describes methods for determining structural characteristics of the virus-like particles containing hemagglutinin according to methods of the current invention. Length and lateral period of hemagglutinin spikes on virus-like particles is measured by transmission electron microscopy. Virus-like particles size is measured by scanning electron microscopy.

### 8.1 Transmission electron microscopy of leaf samples.

Fresh non-frozen leaves infiltrated and incubated according to the invention, are crushed with 1x phosphate-buffered saline (PBS) buffer (1x1 cm leaf disk in 50 uL of buffer) in an Eppendorf tube. The resulting mixture is centrifuged for 15 min at 10 Kg and the supernatant is placed on an transmission electron microscopy (TEM) grid for 10 min, dried with filter paper and placed on a drop of 3% phosphotungstic acid at pH 6.5 for negative staining. Transmission electron microscopy images are acquired at an accelerating voltage of 80 kV on a JEOL JEM1010 transmission electron microscope and analyzed with ImageJ Processing Toolkit.

### 8.2 Transmission electron microscopy of purified Virus-Like Particles.

One hundred microlitre samples of virus-like particles are placed in an Eppendorf tube. A grid is placed at the bottom of the tube, which is then centrifuged for 25 min at 10K. The grid is removed, dried with filter paper and placed on a drop of 3% phosphotungstic acid at pH 6.5 for negative staining. Microscopic images are acquired at an accelerating voltage of 80 kV on a JEOL JEM1010 transmission electron microscope and analyzed with ImageJ Processing Toolkit.

### 8.3 Scanning Electron Microscopy (SEM).

Scanning electron microscopy is used to image the virus-like particles in suspension since transmission electron microscopy needs a solid support. Scanning electron microscopy allows for the analysis of virus-like particles in their native environment. The following procedures, methods and materials are used: Yellow particles 40-80 nm (Spherotech, Inc Ct Number: CFP-00552-2); Yellow particles 90-300 nm (Spherotech, Inc Ct Number: CFP-0252-2); silica and gold particles (quantomix) Calibration beads of 500nm and 40 nm. QX-102 Capsules (quantomix) are coated with Poly-L-lysine (Sigma Cat. No. P8920) to improve imaging results of particles in suspension. 15 µl of 0.1% Poly-L-Lysine solution is applied to the liquid dish and incubated for one hour at room temperature. The solution is removed and the liquid dish is rinsed twice with distilled water. Finally, the water is removed and the liquid dishes are dried before application of the virus-like particle. A mild centrifugation at 500 g for 5 minutes is used to fix the virus-like paerticles on the Poly-L-Lysine matrix.

### Example 9: Determination of size distribution of Virus-Like Particles by fractionation

This example describes methods to measure size of the virus-like particles obtained according to methods described in this application by fractionation.

### 9.1 AF4-MALLS.

Asymmetric-Flow Field-Flow Fractionation (AF4) combined with Multi Angle Light Scattering (AF4-MALLS) is used to separate the virus-like particles on the basis of their hydrodynamic properties as it does not involve an interaction with a stationary phase. Samples containing virus-like particles are separated by an asymmetrical flow FFF system (Eclipse 3+, Wyatt Technology Europe GmbH) followed by MALLS analysis (DAWN HELEOS II detector, Wyatt Technology Europe GmbH). An Agilent 1100 HPLC system (Agilent Technologies) is used to inject the samples and deliver the mobile phase to the FFF system. The FFF apparatus is assembled with a 350 µm-thick Teflon spacer forming the trapezoidal flow channel. The membrane is made of PES with a 30 kDa molecular weight cutoff. Samples are separated in the normal elution mode where particles elute in the order of increasing size. Samples are first focused at the head of the channel with two opposing lateral flow fields of 0.2 ml/min and a vertical cross-flow field of 0.6 ml/min. Focus time is 2 min. Samples are eluted in 50mM NaNO3, pH 7.5. All samples are eluted with a lateral flow rate of 1.5 ml/min and a cross-flow gradient of 0.6-0 ml/min in 35 min. The injection volume is 50 µl for purified virus-like particles samples. Following FFF separation, virus-like particles are detected with an 18-angle MALS detector. The incident beam wavelength is 680 nm and is generated by 30mW Gallium-arsenide laser. Baseline scattering and peak boundaries are determined using Astra software (Wyatt Technology Europe GmbH). Particle root mean square (rms) radius of gyration (Rg) is calculated by fitting the angular dependence of the light scattering Rayleigh ratios to a second order exponential. The fit is extrapolated to zero angle and the radius is calculated from the slope at zero angle according to the Berry formalism using the Astra software.

### 9.2 Particle sizes.

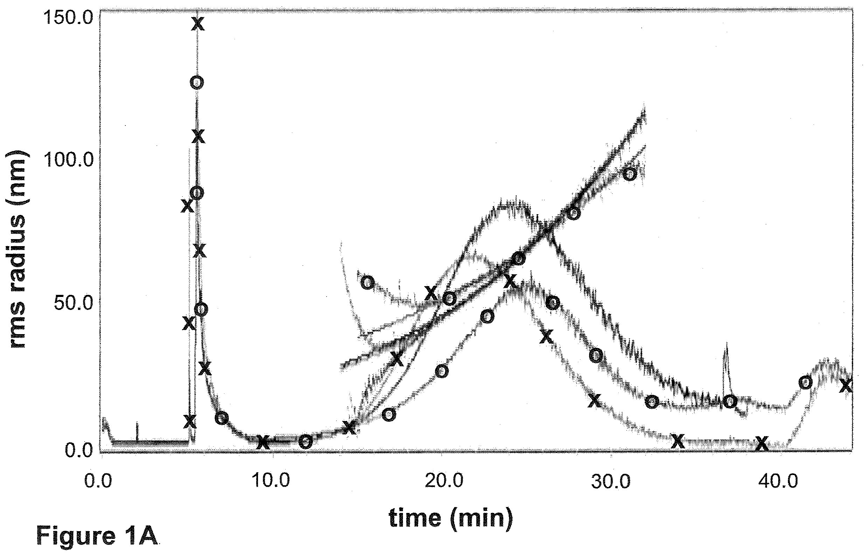

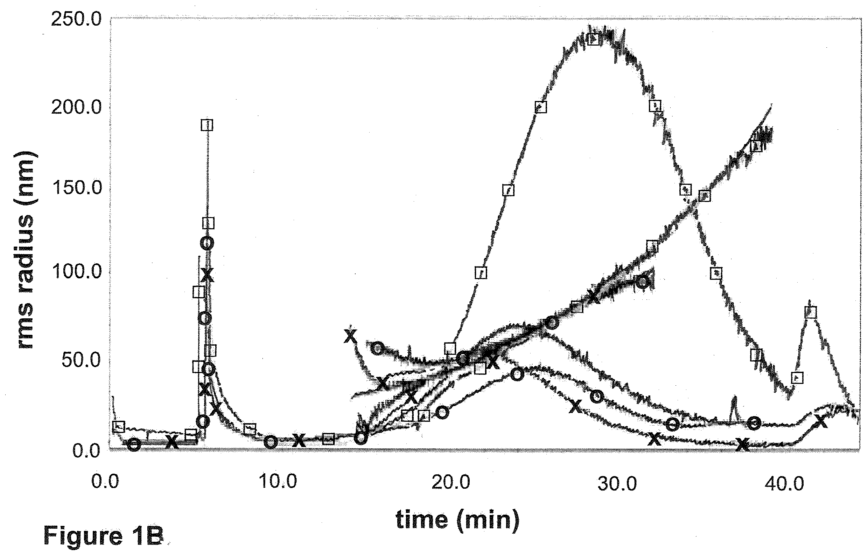

From Figure 1A it can be seen that virus-like particles from *N. tabacum* Burley 21, *N. tabacum* PM132 and PM204, have a size distribution of about 40-220nm in diameter. Differences in size distribution between such virus-like particles is seen with a peak around 45nm for *N. tabacum* PM132, 60 nm for *N. tabacum* PM204 and 50 nm for *N. tabacum* Burley 21 (Figure 1A*). *N*. *tabacum*-derived virus-like particles are significantly smaller compared to those isolated from *N. benthamiana* which have a size distribution of about 100-300nm in diameter (Figure 1B*). The peak top representing the most abundant population here is at 75nm (±0.4%) which is equal to 150 nm particle diameter.
(* All peak values are denoted as radius)

### Example 10: Methods for fatty acid analysis

This example describes methods for the analysis of lipid content and lipid composition of the virus-like particles according to the present invention. Lipids consist of fatty acids. Several classes of lipids exist amongst which the phospholipids represent the main constituent of biological lipid bilayer membranes.

### 10.1 Sample composition and fatty acid analysis by GC-MS.

100 µl of a virus-like particle solution obtained according to the present invention is concentrated in an autosampler vial by removing the solvent to dryness using a gentle nitrogen stream at 65°C. Then, 2 ml hexane and 0.2 ml methanolic potassium hydroxide solution (2M) are added for hydrolysis and esterification. After 10 minutes of ultrasonication, the resulting solution is neutralized with 1M hydrochloric acid. The hexane phase is separated and concentrated to dryness. Resulting fatty acid methyl esters are dissolved in 0.5 ml isooctane and analyzed. An aliquot of 2 µl from the vial is injected pulsed splitless on a capillary column (BPX 90, 30 m x 0.25 mm i.d., 0.25 µm film thickness) in a gas chromatograph (Agilent 6890 Series GC with 5973 mass selective detector, MSD). The injector temperature is 260°C and the oven is programmed to maintain 80°C for 1 min with a subsequent 5°C/min increase to 180°C, and a further 10°C/min increase to 220°C, which is kept for 3 min. Helium is used as carrier gas at a flow rate of 1.2 ml/min. GC-MS screening analyses is predominantly performed using scan mode with a scan range of 50 to 350 amu. In addition, for some analytes and analyses, single ion monitoring (SIM) mode is used. The ion masses used in SIM mode are listed in Table 2.

**Table 2. Ion Mass-to-Charge ratios (m/z)**

| Target analyte | Time period (min) | SIM Ion I (m/z) | SIM Ion II (m/z) | SIM Ion III (m/z) | SIM Ion IV (m/z) |
|---|---|---|---|---|---|
| Palmitic acid methyl ester | 9.0-15.0 | 74 | 87 | 270 | - |
| Oleic acid methyl ester | 16.2-20.0 | 55 | 74 | 87 | 264 |
| Stearic acid methyl ester (deuterated, ISTD) | 20.0-25.0 | 79 | 91 | 150 | - |

Identification of fatty acid methyl esters is first performed by comparison of experimental mass spectra with the NIST mass spectral library. Finally, fatty acids are identified by comparing the retention times and mass spectra with those of reference fatty acid methyl esters compounds.

### Example 11 : Lipid class screening

This example describes methods for lipid class screening by LC-MS/MS. Various phospholipids and phytosterols are targeted by this method.

### 11.1 Sample composition for lipid analysis.

For liquid-liquid-extraction, 375 µL of a 2:1 (v/v) methanol-methylene chloride mixture is added to 100 µL of a virus-like particles solution and the solution is vortexed. Next, 125 µL methylene chloride is added and the solution is vortexed; 125 µL water is added and the solution is vortexed again. Next, the solution is centrifuged at 1000 rpm for 5 min at room temperature to allow separation of the two phases. The upper aqueous phase layer is removed and the lower organic phase layer is transferred to an autosampler vial and 200 µL of a methanolic ammonium acetate solution (1 mM) is added. A blank sample is prepared following the same procedure in parallel using 100 µL of water instead of a virus-like particles solution.

### 11.2 Targeted lipid structures.

Phospholipids targeted by this method are phosphatidylethanolamine, phosphatidylcholine, phosphatidylserine and phosphatidylinositol. Phytosterols targeted are cholesterol, brassicasterol, campesterol, stigmasterol and sitosterol.

### 11.3 Lipid class screening by LC-MS/MS.

For phospholipid analysis, 2 µl is injected from the autosampler vial onto a high performance liquid chromatography (HPLC) column (Phenomenex, Synergi 4µ polar RP, 150 mm x 2.0 mm). Liquid chromatographic separation is performed with a Shimadzu Prominence 20A equipped with 3 pumps, a degasser, a cooled autosampler and a column oven. The flow rate and the gradient program for the LC separation are given in Table 3. The column temperature is 30°C and for the detection of the analytes, a tandem mass spectrometer (Applied Biosystems 4000 Qtrap) is used. Ionization of the analytes is performed using positive as well as negative electrospray ionization (ESI). Analyses are performed in neutral loss or precursor ion scan mode with a scan range from 400 Da to 1200 Da. The instrument parameters for the ion source and the mass analyzer settings for the different compound classes are given in Tables 5 and 6. Data acquisition and data processing are performed with a validated chromatographic data system (Analyst 1.4.1).

**Table 3: Parameter settings for lipid class screening**

| Total time (min) | Flow rate (µL/min) | % of water + 0.1% formic acid solvent | % of methanol + 0.1% formic acid solvent |
|---|---|---|---|
| 0 | 250 | 40 | 60 |
| 1 | 250 | 40 | 60 |
| 18 | 250 | 0 | 100 |
| 23 | 250 | 0 | 100 |
| 24 | 250 | 40 | 60 |
| 26 | 250 | 40 | 60 |

**Table 4. Source parameters for lipid class screening**

| Curtain gas | Ion spray voltage | Temperature | Ion source gas 1 | Ion source gas 2 | Collision gas | Declust ering potential | Entrance potential | Interface heater |
|---|---|---|---|---|---|---|---|---|
| CUR | IS | TEM | GS1 | GS2 | CAD | DP | EP | ihe |
| psi | V | °C | psi | psi | psi | V | V | |
| 10 | 5000 | 200 | 20 | 10 | 10 | 100 | 10 | ON |

**Table 5. Mass analyzer settings for lipid class screening**

| Compound class | Scan type | Loss of/ precursor ion | Polarity |
|---|---|---|---|
| (lyso-)phosphatidylethanolamine | Neutral loss | 141 | Positive |
| phosphatidylserine | Neutral loss | 185 | Positive |
| phosphatidylglycerol | Neutral loss | 172 | Positive |
| phosphatidylserine | Neutral loss | 87 | Positive |
| phosphatidylinositol | Neutral loss | 316 | Negative |
| phosphatidylglycerol | Neutral loss | 228 | Negative |
| Lyso-phosphatidylcholine, sphingomyelin | Precursor ion scan | 184 | Positive |
| phosphatidylinositol | Precursor ion scan | 241 | Negative |
| sphingomyelin | Precursor ion scan | 59 | Positive |

### 11.4 Phytosterol screening.

For phytosterol analysis, 20 µl is injected from the autosampler vial onto a high performance liquid chromatography (HPLC) column (Merck, Chromolith SpeedROD RP-18e, 50 mm x 4.6 mm) coupled to a guard column (Phenomenex C-18, 3 mm x 4 mm, 5 µm). Liquid chromatographic separation is performed with an Agilent 1100 equipped with a binary pump, a degasser, a cooled wellplate autosampler and a column oven. The column oven is set to a temperature of 40°C and the wellplate autosampler to 6°C. The flow rate and the gradient program for the LC separation are given in Table 6. For the detection of sitosterol, stigmasterol, campesterol, bassicasterol and cholesterol, a tandem mass spectrometer (Applied Biosystems 4000 Qtrap) is used. Ionization of analytes is performed using positive atmospheric pressure chemical ionization (APPCI). Analyses are performed in multiple reaction monitoring (MRM) mode. The instrument parameters for the ion source and the mass analyzer settings for the different compounds are given in Tables 8 and 9. Data acquisition and data processing are with a validated chromatographic data system (Analyst 1.4.2). The technique is referred to herein as LC-APPCI-MS/MS.

**Table 6. Gradient program for phytosterof screening**

| Total time (min) | Flow rate (µL/min) | % of methanol + water (75:25, v/v) solvent | % of 2-propanol solvent |
|---|---|---|---|
| 0 | 600 | 100 | 0 |
| 1 | 600 | 100 | 0 |
| 2 | 600 | 0 | 100 |
| 5.5 | 600 | 0 | 100 |
| 5.6 | 1200 | 0 | 100 |
| 7.7 | 1200 | 0 | 100 |
| 7.8 | 1200 | 100 | 0 |
| 10 | 1200 | 100 | 0 |

**Table 7. Source parameters for phytosterol screening**

| Curtain gas | Nebulizer current | Temperature | Ion source gas 1 | Collision gas | Entrance potential | Interface heater |
|---|---|---|---|---|---|---|
| CUR | NC | TEM | GS1 | CAD | EP | ihe |
| psi | mA | °C | psi | psi | V | |
| 10 | 5.0 | 400 | 30 | 4 | 10 | ON |

**Table 8. Mass-dependent settings for phytosterol screening**

| Compound | Mass | | Dwell | Declustering potential | Collision energy | Cell exit potential |
|---|---|---|---|---|---|---|
| | Q1 | Q2 | | DP | CE | CXP |
| | amu | amu | ms | V | eV | V |
| Sitosterol | 397.4 | 257.3 | 100 | 70 | 30 | 10 |
| | 397.4 | 161.4 | 100 | 70 | 30 | 10 |
| | 397.4 | 175.5 | 100 | 70 | 30 | 10 |
| Sitosterol-d6 | 404.4 | 257.3 | 100 | 70 | 30 | 10 |
| Stigmasterol | 395.4 | 297.4 | 100 | 40 | 25 | 10 |
| | 395.4 | 255.3 | 100 | 40 | 25 | 10 |
| | 395.4 | 187.4 | 100 | 40 | 25 | 10 |
| Campestrol | 383.4 | 161.3 | 100 | 40 | 30 | 10 |
| Campestrol-d3 | 386.4 | 161.3 | 100 | 40 | 30 | 10 |
| Brassicasterol | 381.4 | 297.3 | 100 | 40 | 30 | 10 |
| Cholesterol | 369.6 | 147.0 | 100 | 50 | 40 | 10 |
| | 369.6 | 161.1 | 100 | 50 | 40 | 10 |

### Example 12: Fatty acid composition of Virus-Like Particles from N. benthamiana

This example describes the fatty acid composition of three *N*. *benthamiana* samples comprising virus-like particles comprising hemagglutinin according to the present invention.

### 12.1 Results.

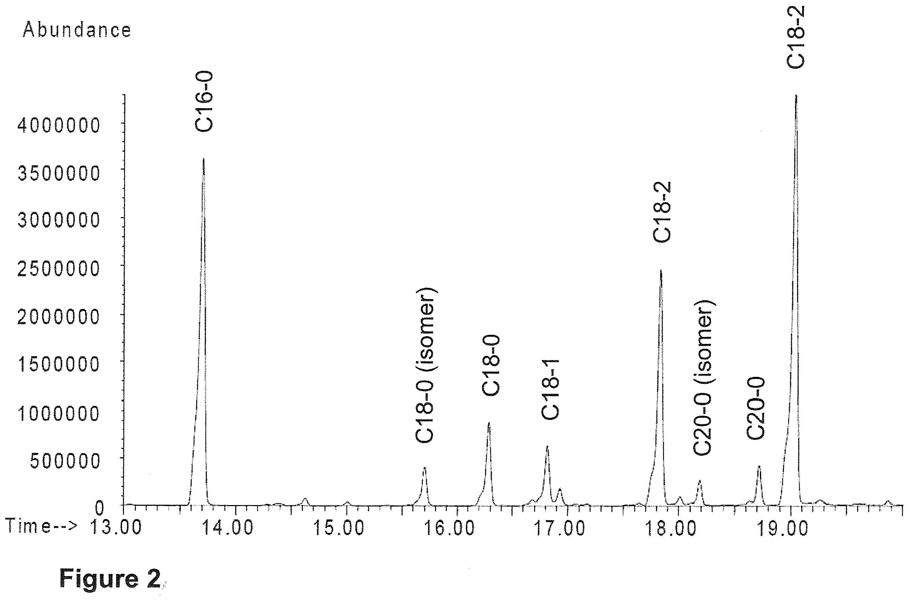

The chromatogram in Figure 2 shows the results of a fatty acid screening of a *N. benthamiana* virus-like particles sample VLP1. Six fatty acids can be seen: palmitic acid (C16-0), stearic acid (C18-0), arachidic acid (C20-0), oleic acid (C18-1), linoleic acid (C18-2) and linolenic acid (C18-3). The two additional fatty acids seen in Figure 2 are probably isomers of stearic acid and arachidic acid because the mass spectra are quite similar to that of stearic acid and arachidic acid. To get quantitative information about the fatty acid concentrations in this virus-like particles sample, a calibration is performed for palmitic acid methyl ester and oleic acid methyl ester. Analytes present in said sample are quantified using these 2 calibrations (Table 9). Palmitic acid and oleic acid are therefore determined quantitatively whereas stearic acid, arachidic acid, linoleic acid and linolenic acid are only determined semi-quantitatively. The fatty acid content of said virus-like particles sample is calculated to be approximately 1.9 pmol/mL of sample solution taken into consideration also the not identified fatty acids.

**Table 9. Fatty acid concentration of N. benthamiana virus-lika particles sample.**

| Analyte | Concentration | | Remarks for calibration |
|---|---|---|---|
| | µg/mL | nmol/mL | |
| Palmitic acid | 38 | 140 | calibrated |
| Stearic acid | 8 | 28 | Palmitic acid calibration used |
| Arachidic acid | 3 | 10 | Palmitic acid calibration used |
| Oleic acid | 7 | 24 | calibrated |
| Linoleic acid | 24 | 82 | Oleic acid calibration used |
| Linolenic acid | 45 | 152 | Oleic acid calibration used |

Fatty acid analysis of two other virus-like particles samples of *N. benthamiana,* VLP2 and VLP3, is done in scan mode, and for palmitic acid and oleic acid also in SIM mode as well. Calibrations are again performed only for palmitic acid and oleic acid and used for quantification of all fatty acids as described above for sample 1. Three technical replicates are performed and the calculated mean values from the 3 technical replicates is given in Table 10.

**Table 10. Fatty acid concentration in N. benthamiana samples VLP2 and 3.**

| Analyte | MS mode | Calibration | µg/mL VLP sample | | | | | | | |
|---|---|---|---|---|---|---|---|---|---|---|
| | | | VLP1 | | | | VLP2 | | | |
| | | | Rep.1 | Rep. 2 | Rep. 3 | Mean | Rep. 1 | Rep. 2 | Rep. 3 | Mean |
| C16-0 | scan | yes | 0.00 | 0.00 | 0.89 | 0,30 | 2.24 | 2.72 | 1.64 | 2.20 |
| C16-0 | SIM | yes | 0.76 | 0.73 | 1.09 | 0.86 | 2.45 | 2.88 | 1.82 | 2.38 |
| C18-0 | scan | no | 0.00 | 0.00 | 0.00 | 0.00 | 0.39 | 0.52 | 0.00 | 0.30 |
| C20-0 | scan | no | 0.00 | 0.00 | 0.00 | 0.00 | 0.00 | 0.00 | 0.00 | 0.00 |
| C18-1 | scan | yes | 0.00 | 0.00 | 0.00 | 0.00 | 0.95 | 0.99 | 0.00 | 0.65 |
| C18-1 | SIM | yes | 0.88 | 0.88 | 1.01 | 0.92 | 1.18 | 1.20 | 1.10 | 1.16 |
| C18-2 | scan | no | 0.28 | 0.00 | 1.70 | 0.66 | 0.89 | 1.40 | 0.55 | 0.95 |
| C18-3 | scan | no | 0.31 | 0.00 | 2.17 | 0.83 | 1.15 | 1.90 | 0.79 | 1.28 |

### Example 13: Phospholipid composition of N. benthamiana Virus-Like Particles

This example describes the phospholipid composition of virus-like particles made in and isolated from *N. benthamiana* according to the present invention.

### 13.1 Lipid profiling and quantification by LC-MS.

A targeted approach by LC-MS allows for the detection and identification of phospholipids by monitoring specific ions related to their characteristic polar head groups. Species eluting at different retention times and with a defined molecular weight can be classified by the presence of a specific head group and further characterized by MS-MS fragmentation allowing for the characterization of the fatty acids linked to it. Using this method the phospholipids phosphatidylethanolamine, phosphatidylcholine, phosphatidylserine and phosphatidylinositol, and sterols brassicasterol, campestrol, stigmasterol and sitosterol are targeted. Sample composition is according to Bligh & Dyer (A rapid method for total lipid extraction and purification. Can J Biochem Physiol 37 (1959) 911-917).

### 13.2 Results.

LC-MS reveals 5 phosphatidylethanolamines (PE's) and 3 phosphatidylcholines (PC's; Table 11). For 3 of the PE's (of MW=739, 741 and 743 respectively) and 1 phosphatidylcholine (of MW=783), 2 possible structures can be proposed from the accurate molecular masses. 2 phosphatidylinositols (PI's) are also identified (Table 11).

**Table 11: Phospholipids represented in virus-like particles derived from N. benthamiana and containing hemagglutinin H5, as identified and characterized by LC-MS**

| Molecular weight | Compound class | R¹ | R² |
|---|---|---|---|
| 755.5615 | phosphatidylcholine | 16-0 | 18-3 |
| 783.5771 | phosphatidylcholine | 18-0 | 18-3 |
| | phosphatidylcholine | 18-1 | 18-2 |
| 757.5615 | phosphatidylcholine | 16-0 | 18-2 |
| 713.4986 | phosphatidylethanolamine | 16-0 | 18-3 |
| 715.5145 | phosphatidylethanolamine | 16-0 | 18-2 |
| 741.5319 | phosphatidylethanolamine | 18-0 | 18-3 |
| | phosphatidylethanolamine | 18-1 | 18-2 |
| 739.5133 | phosphatidylethanolamine | 18-1 | 18-3 |
| | phosphatidylethanolamine | 18-2 | 18-2 |
| 743.5000 | phosphatidylethanolamine | 18-0 | 18-2 |
| | phosphatidylethanolamine | 18-1 | 18-1 |
| 831.5024 | phosphatidylinositol | 16-0 | 18-2 |
| 829.4865 | phosphatidylinositol | 16-0 | 18-3 |

### Example 14: Total lipid composition of Nicotiana-derived Virus-Like Particles

This example describes how the various methodologies described in Examples 15 to 19 and GC-MS measurement of fatty acids after derivatisation of lipids, can be used to determine the total lipid content and composition of the virus-like particles made in *Nicotiana* plant cells according to the present invention. For the quantification of total fatty acids in such virus-like particles, free fatty acids and those constituting phospholipids, sterol lipids and esters are measured.

### 14.1 Approach for profiling lipid content Virus-Like Particles.

Lipids consist of fatty acids and several classes of lipids exist amongst which the phospholipids represent the main constituent of biological lipid bilayer membranes. Phospholipids, sterol lipids and esters and sphingolipids are building blocks of lipid bilayer membranes. Three methods are used to determine the lipid composition of the plant-derived virus-like particles obtained according to methods of the present invention. The first 1) is by GC-MS and is used for the screening and semi-quantification of fatty acids in the virus-like particles. Fatty acids are analyzed as methyl esters. To this end, the hydrolysis of phospholipids and the esterification of their constitutive fatty acids to allow profiling, is optimized. The second methodology is by 2) methods based on LC-MS which is used for the lipid class screening of phospholipids including phosphatidylethanolamines (PE), phosphatidylcholines (PC), phosphatidylserines (PS), phosphatidylinositols (PI) and sphingomyelins (SM). A qualitative and comparative determination of these phospholipids is done. Phytosterols are measured using pure cholesterol, brassicasterol, campesterol, stigmasterol and sitosterol as compounds. Identification of lipids is performed using a high resolution-mass spectrometer for the determination of accurate masses of the parent ions as well as for tandem mass spectrometric fragment ions. Higher order fragmentation experiments are performed with unit resolution to get additional structural information. A more comprehensive analysis of the various lipids of the virus-like particle is done via a non-targeted screening method based on LC-MS. Phospholipids, sphingolipids and sterol lipids are selectively extracted of from the virus-like particles according to Bligh and Dyer (1959, *supra*) to allow the analysis of the resulting mixture and to profile and characterize all the different lipids present within the mixture by LC-MS which allows for lipid class screening. The third method 3) is by analyzing the lipid mixture present in virus-like particles direclty to allow the identification of molecules not detected by method 1 and 2 and to quantify and identify any unknown species.

### 14.2 Derivatisation method.

Take 100µL virus-like particle sample and add 50 µl ISTD (C17-0, 1,2-DiHeptadecanoyl-Glycero-3-Phosphatidylcholine); whirl; add 2 mL Hexane; add 0.4 mL 2M KOH (methanolic); whirl; treat for 10 min by sonication; neutralize with 1M HCl (pH between 3 and 7); remove Hexane; dry under nitrogen gas and redissolve in 0.5 mL Isooctane.

### 14.3 GC-MSD method.

GC column BPX 90, 30 m, 0.25 mm, 0.25 µm. Injection of 3 µl sample at 260 °C, pulsed splittless. Ovenprogram: 1 min, 80°C; 5°C/min increase until 180°C, 0 min; 10°C/min increase until 220°C, 3 min. MSD: SIM. C16-0, m/z: 74 (87, 143, 270); C17-0, m/z: 74 (87, 143, 284); C18-0, m/z: 74 (87, 143, 298); C18-1, m/z: 264 (55, 67, 74); C18-2, m/z: 81 (67, 95, 294); C18-3, m/z: 79 (67, 95, 292); C20-0, m/z: 74 (87, 143, 326). Compounds used for quality control: C16-0: 1,2-DiPalmitoyl-Glycero-3-Phosphatidylethanolamine; C18-0: 1,2-DiStearoyl-Glycero-3-Phosphatidylethanolamine; C18-1: 1,2-DiOleoyl-Glycero-3-Phosphatidylethanolamine; C18-2: 1,2-DiLinoleoyl-Glycero-3-Phosphatidylcholine; C18-3: 1,2-DiLinolenoyl-Glycero-3-Phosphatidylcholine; C20-0: 1,2-DiArachidoyl-Glycero-3-Phosphatidylcholine. C17-0: 1,2-DiHeptadecanoyl-Glycero-3-Phosphatidylcholine (ISTD). Calibration is by methyl ester of fatty acids without sample composition; R^2: 0.997 - 0.999. Quality control compounds are prepared in TRIS buffer.

**Table 12. Concentration and identity of fatty acids (in µg/mL) in various virus-like particles samples. Six fatty acids were identified as methyl esters, namely palmitic acid (C16-0), stearic acid (C18-0), arachidic acid (C20-0), oleic acid (C18-1), linoleic acid (C18-2) and linolenic acid (C18-3). Stearic and arachidic acid were identified as a mixture of two isomers clearly resolved in the optimized chromatographic conditions.**

| Concentration | Concentration in µg/ml | | | | | |
|---|---|---|---|---|---|---|
| | C16-0 | C18-0 | C18-1 | C18-2 | C20-0 | C18-3 |
| *N. tabacum* PM132 sample D | 4.64 | 1.78 | 0.37 | 3.23 | 0.92 | 3.23 |
| *N. tabacum* PM132 sample A | 5.14 | 1.76 | 0.45 | 3.73 | 0.88 | 4.11 |
| *N. tabacum* PM132 sample B | 5.35 | 1.84 | 0.40 | 4.05 | 0.95 | 4.43 |
| *N. tabacum* PM132 sample C | 5.21 | 1.80 | 0.41 | 4.06 | 0.94 | 4.46 |
| *N. tabacum* Burley 21 | 5.73 | 1.86 | 0.41 | 3.99 | 0.97 | 4.32 |
| *N. tabacum* PM204 | 5.37 | 1.91 | 0.43 | 4.26 | 1.01 | 4.58 |
| *N. benthamiana* | 2.73 | 1.14 | 2.07 | 2.12 | 0.38 | 2.09 |

Using the analytical technique, the comparisons show that the lipid bilayer of the VLPs made using *N. tabacum* has a content of oleic acid that is less than 0.5µg/mL, while the content of oleic acid in *N. benthamiana* is about 2. Thus, the amount of oleic acid in N. benthamiana-derived VLPs is at least four to five times that of N. tabacum VLPs.

The comparisons also show that the lipid bilayer of the VLPs made using *N. tabacum* has a content of arachidic acid that is greater than 0.8µg/mL, while the content of arachidic acid in *N. benthamiana* is about 0.4. Thus, the amount of arachidic acid in *N.* tabacum-derived VLPs is at least two times greater than that of *N. benthamiana* VLPs.

### 14.4 Total phytosterols.

Table 13 summarizes the concentrations of phytosterols in virus-like particles obtained from various *N. tabacum* varieties and *N. benthamiana* according to the present invention as measured by LC-APPCI-MS/MS.

**Table 13. Concentrations (ng/mL) and percentage of total (%) of phytosterols in various virus-like particles samples as measured by LC-APPCI-MS/MS**

| Sample | | ng/mL (% of total) | | | | | |
|---|---|---|---|---|---|---|---|
| | | cholesterol | brassicasterol | campesterol | stigmasterol | sitosterol | total |
| *N. tabacum* Burley 21 | A | 2018 (11.45) | 472 (2.79) | 4028 (22.86) | 9200 (52.22) | 1898 (10.77) | 17617 (100) |
| | B | 1988 (11.36) | 450 (2.57) | 4111 (23.5) | 9033 (51.63) | 1914 (10.94) | 17496 (100) |
| *N. tabacum* PM132 | A1 | 1633 (10.37) | 397(2.52) | 3469 (22.02) | 8464 (53.73) | 1790 (11.36) | 15753 (100) |
| | A2 | 1792 (11.58) | 392 (2.53) | 3880 (25.06) | 7806 (50.43) | 1610 (10.40) | 15480 (100) |
| | B1 | 1981 (11.98) | 517(3.13) | 4244 (25.67) | 7969 (48.21) | 1820 (11.01) | 16531 (100) |
| | B2 | 2207 (12.45) | 474 (2.67) | 4323 (24.38) | 8719 (49.18) | 2007 (11.32) | 17730 (100) |
| | C1 | 2019 (11.68) | 456 (2.64) | 4078 (23.59) | 8774 (50.75) | 1960 (11.34) | 17287 (100) |
| | C2 | 2029 (12.66) | 439(2.74) | 3778 (23.58) | 7927 (49.47) | 1851 (11.55) | 16024 (100) |
| *N. tabacum* PM204 | A | 2024 (12.9) | 475 (3.03) | 4025 (25.65) | 7466 (47.57) | 1704 (10.86) | 15694 (100) |
| | B | 1953 (12.20) | 431 (2.69) | 3709 (23.18) | 8270 (51.68) | 1639 (10.24) | 16002 (100) |
| *N. benthamiana* | A | 619 (7.27) | 98 (1.15) | 1922 (22.57) | 4032 (47.36) | 1842 (21.63) | 8514 (100) |

This analysis shows that the ratios of cholesterol and sitersterol are different. In *N.* tabacum-derived VLPs, the ratio of cholesterol and sitersterol are about the same (i.e., about 1.0) while the ratio in *N. benthamiana-dervied* VLPs, the ratio is much lower at about 0.3.

### Total fatty acids of Nicotiana-derived Virus-Like Particles.

Table 14 summarizes the results of phospholipids and phytosterols as measured using various technologies and partly presented in Tables 14 and 15, for the various virus-like particles of the present invention as well as the lipid:protein ratio of said particles. Protein content is measured using standard techniques.

**Table 14. Fatty acids and sterols (µg/mL and % of total) and lipid to protein ratio of various Nicotiana-derived virus-like particles (ND, not determined).**

| | Fatty Acids by GC-MS (µg/mL) (%) | | | | | | Sterols by LC-MS (µg/mL) (%) | | | | | µg/mL (%) | µg/mL | Ratio |
|---|---|---|---|---|---|---|---|---|---|---|---|---|---|---|
| Species | C16-0 | C18-0 | C18-1 | C18-2 | C20-0 | C18-3 | Cholesterol | Brassic a-sterol | Stigmasterol | Campesterol | Sitosterol | Fatty acids + Sterols | Total Protei n | Lipid: Protein |
| *N. tabacum* PM132_B1 | 5.14 (16.2 2) | 1.76 (5.55) | 0.45 (1.42) | 3.73 (11.7 7) | 0.88 (2.78) | 4.11 (12.9 7) | 1.71 (5.4) | 0.39 (1.23) | 8.14 (25.69) | 3.68 (11.61) | 1.70 (5.36) | 31.69 (100) | 80 | 0.40 |
| *N. tabacum* PM132_B2 | 5.35 (15.6 7) | 1.84 (5.39) | 0.4 (1.17) | 4.05 (11.8 6) | 0.95 (2.78) | 4.43 (12.9 8) | 2.09 (6.120 | 0.50 (1.46) | 8.34 (24.43) | 4.28 (12.54) | 1.91 (5.59) | 34.14 (100) | 100 | 0.34 |
| *N. tabacum* PM132_B3 | 5.21 (15.5 3) | 1.8 (5.37) | 0.41 (1.22) | 4.06 (12.1 0) | 0.94 (2.80) | 4.46 (13.3) | 2.02 (6.02) | 0.45 1.34) | 8.35 (24.9) | 3.93 (11.72) | 1.91 (5.69) | 33.54 (100) | 80 | 0.42 |
| *Nicotiana benthamian* a | 2.73 (14.3 4) | 1.14 (5.99) | 2.07 (10.8 7) | 2.12 (11.1 3) | 0.38 (2) | 2.09 (10.9 8) | 0.62 (3.26) | 0.10 (0.53) | 4.03 (21.17) | 1.92 (10.08) | 1.84 (9.66) | 19.04 (100) | 57 | 0.33 |

### Example 15: Methods for protein characterisation

This example describes several methodologies for the structural characterization of the hemagglutinin protein displayed on the virus-like particles of the present invention. The functionality of hemagglutinin as an antigen in vaccine compositions is dependent on several structural features. Most important is the folding and assembly of hemagglutinin H protein into homo-trimers. Another important feature for functionality is the N-glycosylation pattern of the protein including the various glycoforms. Nano-scale liquid chromatography coupled with high resolution-electrospray mass spectrometry or matrixassisted laser desorption ionization-mass spectrometry are used to elucidate the amino acid sequence and N-glycan composition and glycoforms of said hemagglutinin protein.

### 15.1 Sample Composition for H5 Analysis.

For enzymatic digestion in solution, either trichloroacetic acid, a mixture of acetone and 0.1 M methanolic hydrochloric acid (50:50, v/v) or methanolic dichloromethane is added to 50-100 µL of solution containing hemagglutinin H5 obtained or extracted from the virus-like particles of the current invention for protein precipitation. After protein precipitation, the sample is centrifuged for 2 min and the pellet dissolved in 50 µL of 50 mM ammonium bicarbonat buffer. For sample composition without protein precipitation, RapiGest (Waters, UK) is added to 50-100 µL of H5 solution. After addition of RapiGest, the solution is heated to 100°C for 5 min and 50 µL of 50 mM dithiothreitol (DTT) solution in 50 mM ammonium bicarbonat buffer and 25 µL of 100 mM iodoacetamide solution in 50 mM ammonium bicarbonat buffer is added for reduction and alkylation. Alkylation is stopped by adding 5 µL of 50 mM DTT solution in 50 mM ammonium bicarbonat buffer and additional 80 µL of 50 mM ammonium bicarbonat buffer. Subsequently, 2.3 µL of a trypsin solution (20 µg/ml in 50 mM ammonium bicarbonat buffer/acetonitrile 80 : 20) is added and the mixture is incubated at 37°C overnight. Tryptic digestion is stopped by adding 1 µL of formic acid.

### 15.2 Peptide Mass Fingerprinting by LC-ESI-MS.

From the autosampler vial, 5 µL is injected onto a HPLC column (Advanced Material Technology, Halo 018, 100 mm x 2.1 mm, 2.7 µm). Liquid chromatography separation is performed with an Agilent 1100 equipped with a binary pump, degasser, cooled wellplate autosampler and column oven. Column oven is set to a temperature of 60°C. Flow rate and gradient program for the LC separation is given in Table 15. For the detection of analytes, a high resolution-mass spectrometer, a Thermo LTQ-FT Ultra, is used. Ionization of analytes is performed using positive electrospray ionization (ESI). Analyses are performed in scan mode and scan range is from 300 Da to 2000 Da. Instrument parameters for the mass analyzer are given in Table 16.

**Table 15. Gradient program for peptide mass fingerprinting**

| Total time | Flow rate | Water + 0.1 % formic acid solvent | Methanol + 0.1% formic acid solvent |
|---|---|---|---|
| (min) | (µL/min) | (%) | (%) |
| 0 | 200 | 98 | 2 |
| 2 | 200 | 98 | 2 |
| 40 | 200 | 55 | 45 |
| 42 | 200 | 10 | 90 |
| 48 | 200 | 10 | 90 |
| 58 | 200 | 98 | 2 |

**Table 16. Mass analyzer settings**

| Scan | Details | Detection | Fragmentation | Resolution |
|---|---|---|---|---|
| (1) | Scan (300-2000 Da) | FTMS | - | 50000 |
| (2) | MS2 most intense (1) | FTMS | CID | 12500 |
| (3) | MS2 second (1) | FTMS | CID | 12500 |
| (4) | MS2 third (1) | FTMS | CID | 12500 |
| (5) | MS2 most intense (1) | FTMS | ECD | 12500 |
| (6) | MS2 second (1) | FTMS | ECD | 12500 |
| (7) | MS2 third (1) | FTMS | ECD | 12500 |

### 15.3 Peptide Mass Fingerprinting by LC-MALDI-MS by nano-LC.

5 µL sample as prepared above is injected onto a nano-HPLC column (Nanoseparations NS-AC-10 BioSphere 018, 5 µm, 120 A, 360/75 µm, L=10 cm) equipped with a pre-column (Nanoseparations NS-MP-10 BioSphere 018, 5 µm, 120 A, 360/100 pm, L=2 cm). Liquid chromatography separation is performed with a Bruker EASY-nLC II at room temperature. Flow rate and gradient program for the nano-LC separation is given in Table 17. Fractions of 10 s, i.e. 50 nl, are online mixed with a-cyano-4-hydroxycinnamic acid solution and spotted on an anchor chip stainless steel MALDI target (384 MTP, 800 pm anchor) using the Bruker MALDI Spotter Proteineer® fc II.

**Table 17. Gradient program for nano-LC**

| Total time | Flow rate | Water + 0.1% TFA solvent | Acetonitrile + 0.1% TFA solvent |
|---|---|---|---|
| (min) | (nL/min) | (%) | (%) |
| 0 | 300 | 98 | 2 |
| 30 | 300 | 55 | 45 |
| 35 | 300 | 55 | 45 |
| 48 | 300 | 20 | 80 |
| 49.5 | 300 | 10 | 90 |
| 60 | 300 | 10 | 90 |
| 60.9 | 300 | 98 | 2 |
| 61 | 300 | 98 | 2 |

### 15.4 Matrix-Assisted Laser Desorption/Ionization (MALDI) Mass Spectrometry.

For the detection of the analytes, a MALDI mass spectrometer, a Bruker Ultraflextreme ®, is used. Desorption and ionization are performed using a Bruker Smartbeam ® III laser, a modified Nd/YAG laser, with 1 kHz acquisition rate. Analyses are performed in positive mode with a scan range from 700 Da to 4000 Da. The mass analyzer is operated in reflector mode. Spectra are acquired in MS as well as in MS/MS mode. In MS mode, 2500 single spectra from 1 laser shot are added and in MS/MS mode, 2000.

### Example 16: Confirmation of protein sequence integrity.

This example describes experiments to confirm sequence integrity of recombinant hemagglutinin protein as displayed on the surface of the virus-like particles produced according to the present invention. Sequence confirmation is by UPLC-Qtof-MSE and peptide mapping after first reducing and alkylating the protein sample and subsequent endoproteolytic digestion with trypsin and Glu-C.

### 16.1 Sample composition.

Protein samples comprising hemagglutinin H5 of the virus-like particles purified from *N. benthamiana* and *N. tabacum* are reduced in 20mM DTT (30min; 56°C) and ultrafiltered by NanoSep 10K Omega concentrators. For trypsin digestion, after ultrafiltration an aliquot is resolved in 100mM Tris-HCl digestion buffer (pH 8.5) and a further aliquot thereof is treated in 100mM Tris-HCl with 2µg of trypsin (37°C, 18h). For Glu-C digestion, after ultrafiltration, an aliquot is resolved in 50 mM ammoniumhydrogencarbonate buffer (pH 8.0) and a further aliquot is treated in 50mM ammoniumhydrogencarbonate buffer with 2 µg of endoproteinase Glu-C (37°C, 18h).

### 16.2 N. benthamiana hemagglutinin.

Sequence coverage of hemagglutinin derived from virus-like particles isolated from *N. benthamiana* after tryptic digestion is 99.1%. 547 aminoacids are identified out of 552 amino acids in total and no peptides are identified corresponding to the expected signal peptide amino acid sequence. For the tryptic digest, the remaining 0.9% are undetected single amino acid tryptic peptides according to the cleavage specificity of trypsin which cannot be detected by this approach. The primary structure matches exactly the aminoacid sequence of H5 protein from the Indonesia 2005 strain (GenBank: ABP51969.1). Peptide sequencing also indicates efficient processing of the signal peptide consisting of the unmapped sequence MEKIVLLLAIVSLVKS (not shown in the figure). Some product-related impurities like deamidation of asparagine and glutamine residues as well as oxidation of methionine residues were also reported. N-Glycosylation is shown for asparagines at positions 11, 23, 84, 154, 165 and 286.

### 16.3 N.tabacum hemagglutinin.

Sequence coverage of hemagglutinin H5 as obtained from *N. tabacum* Burley 21 is 97.5%, for *N. tabacum* PM132 95.7% and *N. tabacum* PM204, 97.5%. Signal peptides for all three are properly cleaved and both N- and C-terminus are correct and as expected. N-glycosylation is observed for asparagine residues at positions 11, 23, 84, 154, 165, 286.

### Example 17: N-glycosylation pattern of hemagglutinin on Virus-Like Particles

This example describes the N-glycosylation pattern of the hemagglutinin protein comprised within the virus-like particles of the present invention.

### 17.1 Sample composition.

SDS-PAGE gel bands comprising hemagglutinin protein as determined from their respective molecular weight and confirmed by Western analysis, are cut out of the gel and washed in 30% acetonitrile in 25mM NH4HC03, reduced by treatment with 10mM DTT in 25mM NH4HC03 at 56°C for 30 min, and alkylated in 40mM iodacetamide in 25mM 25mM NH4HC03 at room temparature for 20 min. Samples are digested with trypsin overnight and the peptides that are generated are extracted with 50% acetonitrile 5% formic acid in water.

### 17.2 Reversed Phase-Ultra Performance Liquid Chromatography conditions.

Column: UPLC Acquity Column BEH C18; 1.7µm; 1.0x100mm; column oven at 40°C. System A: 0.1% formic acid in water. System B: 0.1% formic acid in acetonitrile. Flow rate: 70µL/min. Injection: 5µL. Gradient: from 0-25 min 2-40% System B; from 25-27 min 40-80% System B; from 27-28 min 80-98% System B.

### 17.3 Data composition and processing.

Retention times of glycopeptides are identified by the extracted ion current chromatogram of the characteristic glycan fragment B-HexNAc (m/z=204.09) that occurs by high energy fragmentation. According to the retention time of the glycan fragments, the scans of the low energy mode acquisition are summed up, centred and deconvoluted by the maximum entropy III algorithm. Resulting masses are matched with glycan databases using GlycoWorkBench 1.1 software (European Carbohydrate Database Project).

### 17.4 N-glycosylation sites of Nicotiana-derived hemagglutinin H5 protein.

Analysis of hemagglutinin H5 isolated and extracted from *N. benthamiana* and *N. tabacum* derived virus-like particles, by UPLC-QTof-MS^{E} using the methodology of ion extraction to identify and characterize N-glycan-containing peptide species, reveals the presence of six N-glycosylation sites (Table 18).

**Table 18. Asparagine (Asn) position on H5 mature protein backbone, glycopeptide sequence and N-glycosylation pattern (deconvoluted patterns in Figure). N is the N-glycosylated asparagine residue.**

| Asn | Glycopeptide sequence | N-glycosylation pattern (Figure) |
|---|---|---|
| 11 | DQICIGYHANNSTEQVDTIMoxEK (SEQ ID NO: 8) | 3 |
| 23 | NVTVTHAQDILEK (SEQ ID NO: 9) | 4 |
| 154 | NSTYPTIK (SEQ ID NO: 10) | 5 |
| 165 | SYNNTNQEDLLVLWGIHHPNDAAEQTR (SEQ ID NO: 11) | 6 |
| 286 | CQTPMGAINSSMPFHNIHPLTIGECPK (SEQ ID NO: 12) | 7 |
| 484 | NGTYNYPQYSEEAR (SEQ ID NO: 13) | 8 |

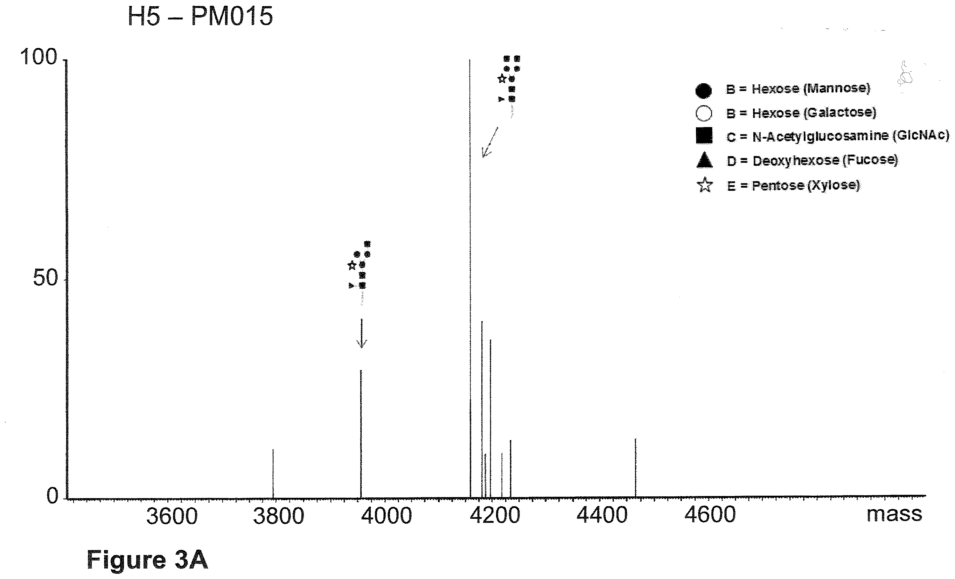

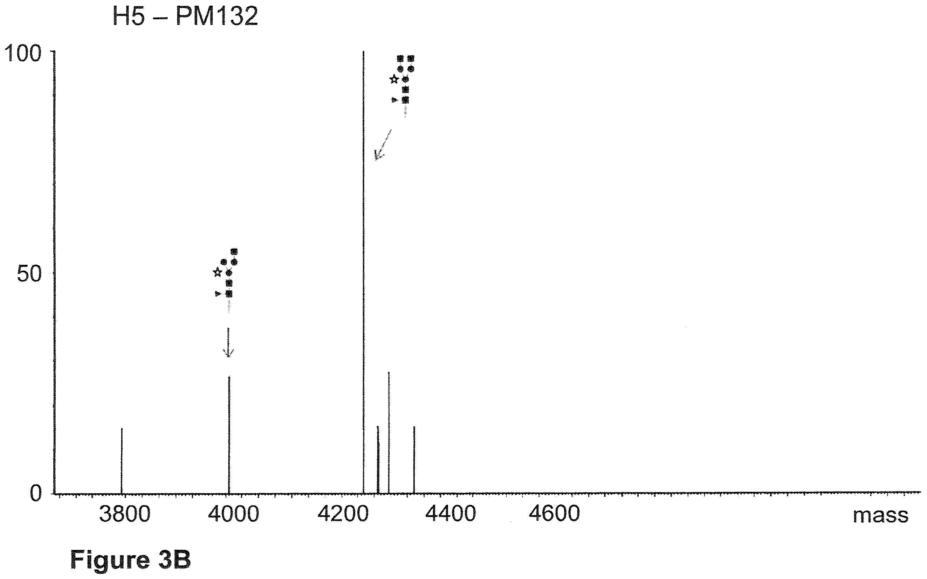

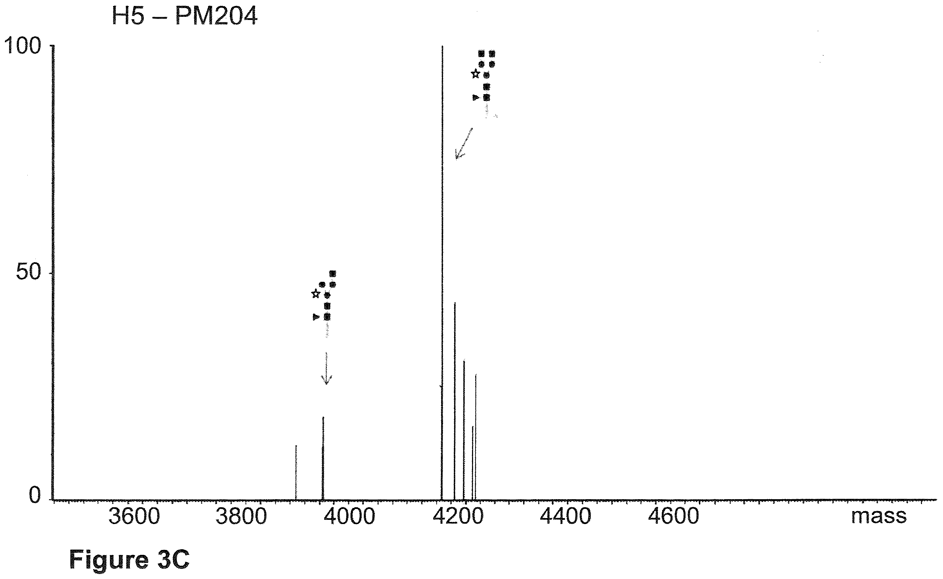

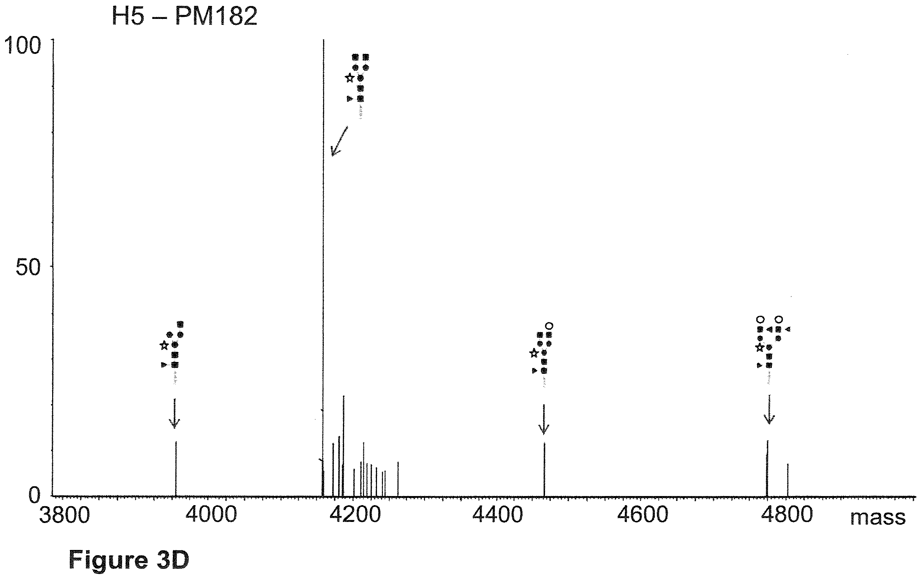

The ion at m/z=204.09 (B-HexNAc) that corresponds to the oxonium ion of N-acetylglucosamine residues which are typical building blocks of N-glycan oligosaccharide structures, is selected as a reference. Deconvoluted N-glycosylation patterns for each of the 6 N-glycosylation sites for *N. tabacum* Burley 21 (H5-PM015), *N. tabacum* PM132 (H5-PM132), *N. tabacum* PM204 (H5-PM204) and N. *benthamiana* (H5-PM182) derived hemagglutinin H5 protein are shown in Figures 3 (Asn-11), 4 (Asn-23), 5 (Asn-154), 6 (Asn-165), 7 (Asn-286) and 8 (Asn-484).

### Example 18: S-Acylation of hemagglutinin

This example describes experiments to assess S-acylation of hemagglutinin displayed on the virus-like particles according to the present invention. Analysis of the C-terminal fragment of hemagglutinin H5 from amino acid 524 to 568 shows the presence of 2 palmitoyl chains.

### 18.1 Chemicals and consumables.

Water is from J.T. Baker (Deventer, The Netherlands), chloroform, TFA and methanol are from Fisher Scientific (Loughborough, UK), Tris Ultra Quality and HCl are from Roth (Karlsruhe, Germany), acetonitrile is from VWR (Darmsta^{dt} Germany), bromelain is from pineapple stem (Sigma-Aldrich, Steinheim, Germany), o-cyano-4-hydroxycinnamic acid (HCCA) is from Bruker Daltonik GmbH (Bremen, Germany), Nanosep Centrifugal Devices are from PALL Life Sciences (Dreieich, Germany).

### 18.2 Sample treatment

Hemagglutinin H5 virus-like particles are produced in *N. tabacum* PM132 according to the invention and isolated by sucrose gradient and treated with 1% CHAPS to destroy the particles. Lipids are removed by size exclusion chromatography in the presence of 1 % CHAPS in phosphate-buffered saline. An amount of 250 µg of sample is dissolved in Tris-HCl buffer, pH 7.4, and digested with approximately 3.8 mg bromelain for 1 h at 37°C. S-acylated peptide fragments are extracted in methanol/chloroform (4:1). The organic phase containing the peptide fragments is spotted together with HCCA on a target plate and the sample is analyzed by MALDI-TOF-MS.

### 18.3 Results.

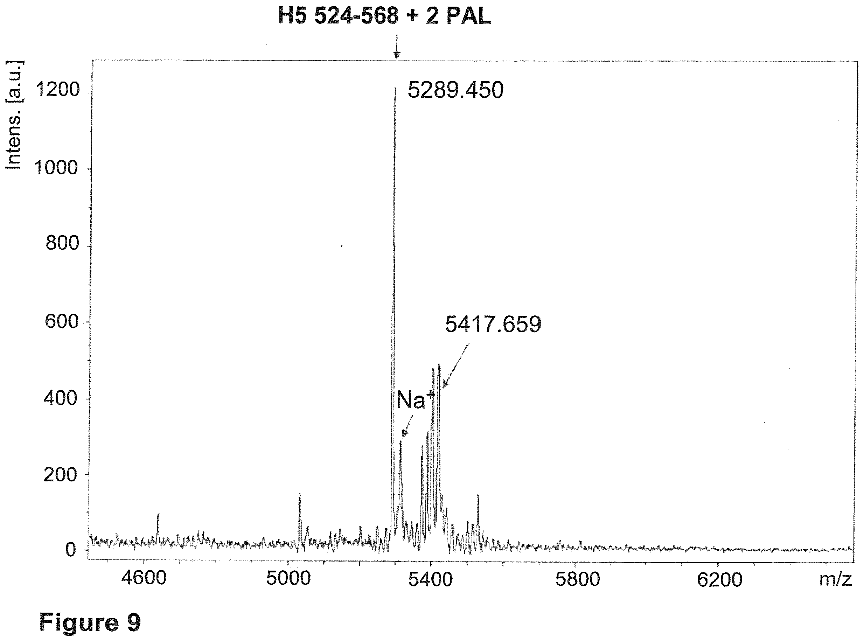

Bromelain digestion generates a C-terminal hemagglutinin H5 fragment comprising amino acids 524 to 568, with 3 potential cysteine acylation sites (see Table 19). The main signal at *m*/*z* 5289.45 is the C-terminal peptide with 2 palmitoyl chains (Figure 9; Table 19). There are no signals corresponding to the C-terminal peptide with no palmitoyl groups (expected mass 4813.791), 1 or 3 palmitoyl or any combination with stearoyl and palmitoyl chains (Figure 9). Further small signals are however detected which suggest alternative cleavage sites of bromelain (Figure 9; Table 19).

**Table 19. Amino acid sequences of C-terminal hemagglutinin H5 peptide fragments, type of acylation and calculated masses. PAL, palmitoyl. Potential cysteine acylation sites are in bold. Alternative splicing by bromelain at lysine (K) is underlined.**

| C-terminal H5 peptide fragment | Fatty acid | Mass (m/z) |
|---|---|---|
| LESIGTYQILSIYSTVASSLALAIMMAGLSLWMCSNGSLQCRICI (SEQ ID NO: 6) | 2 PAL | 5289.450 |
| KLESIGTYQILSIYSTVASSLALAIMMAGLSLWMCSNGSLQCRICI (SEQ ID NO: 7) | 2 PAL | 5417.659 |
| LESIGTYQILSIYSTVASSLALAIMMAGLSLWMCSNGSLQCRICI (SEQ ID NO: 6) | 0 PAL | 4813.791 |

### Example 19: Reducing particle size

This example describes methods to obtain more homogeneous and smaller particles. These methods can be used with the virus-like particles displaying hemagglutinin of the present invention and the various lipid compositions as exemplified in Example 29.

### 19.1 Ultrasound treatment to reduce particle size.

Ultrasound treatment is used to obtain more homogeneous and smaller particles as follows:
1. Add 0.001M fluorescein to a sample of virus-like particles displaying hemagglutinin according to the present invention and/or a mixture thereof with lipids and/or additional compounds as exemplified in Example 29 to visualize the formation of liposomes.
2. Sonicate for 30 min at 42°C using an FS20D sonicator and a setting of 60 sonic bursts per min.
3. Layer sonicated materials over a sucrose gradient comprising 45-60-70-80% sucrose. Volumes of the gradients are chosen depending on sample size.
4. Centrifuge at 65,000 g for 14 hrs @ 4°C.
5. Collect the fluorescent band visualized by UV.
6. Add one volume of dialysis buffer comprising 1.5M NaCl, 50 mM MES, pH 6.0 and 0.0005% Tween-80 to the sample and place the sample in a dialysis tube to remove sucrose by dialyzing against excess dialysis buffer.
7. Check the absorbance at 280 nm.

Optionally, a sample with a concentration of approximately 0.5 mg/mL is analysed by electron microscopy and an Eppendorf Centricon MWCO 10,000 membrane is used to concentrate the sample to approximately half of the collected spin volume.

### 19.2 Rapid extrusion.

Homogeneously sized virus-like particles can also be obtained by sequential extrusion through polycarbonate filters with a pore size ranging from 30 to 200 nm. Filter pore size can be 30, 50, 100 or 200 nm.

### Example 20: Measurement of immune responses and efficacy in mice

This example describes methods for measuring immune responses to influenza hemagglutinin H5 virus-like particles produced according to the present invention, in mice, and protection against a lethal infection with influenza virus upon such immunization.

### 20.1 Measurement of endotoxin and residual DNA.

Endotoxins are determined by the *Limulus* amebocyte lysate test kit (QCL-1000, Lonza, Wakersville, MD) using internal *Escherichia coli* 0111:B4 control. Residual DNA is determined by the PicoGreenH fluorescent dye assay (Invitrogen) and measured by fluorometry using Lambda DNA as standard (Invitrogen).

### 20.2 Immunization and measurements of titres.

Immunization and vaccination studies are performed with 6-8 week-old female BALB/c mice (Charles River Laboratories). A non-limiting example of such an immunization study is an experiment with thirty mice that are randomly divided into six groups of five animals. Optionally, the number of groups and number of animals within a group can be increased or decreased. A non-limiting example of an immunization experiment involves immunizing mice in a two-dose regimen, the second immunization being 3 weeks following the first. Optionally, immunization can be done using a three-dose regimen, the second and third immunization being 3 weeks following the prime immunization and first booster immunization, respectively. Optionally, immunization can be done as a single dose immunization. In a non-limiting example, un-anaesthetized mice are immunized by intramuscular administration in the hind legs with purified virus-like particles comprising hemagglutinin H5 using a dosage of 0.1 pg, 1 µg, 5 µg, 12 µg or 20 µg formulated in Alhydrogel 2% (alum, Accurate Chemical & Scientific Corporation, Westbury, NY, USA) in a 1:1 ratio. Optionally, un-anaesthetized mice are immunized by intranasal, subcutaneous or transdermal administration with purified virus-like particles comprising hemagglutinin H5 using a dosage of 0.1 µg, 1 µg, 5 µg, 12 µg or 20 µg with or without an adjuvant. As a control, one group of mice is immunized with 5 µg of recombinant hemagglutinin H5 formulated in alum and purchased from Immune Technology, and one group of mice is immunized with phosphate-buffered saline formulated in alum. Lateral saphenous vein blood is collected 14 days after priming and second immunization of un-anaesthetized mice. Serum is collected by centrifuging at 8000 g for 10 min and hemagglutinin titres of sera are measured as described (Kendal *et al*., 1982; WHO, 2002). Inactivated A/Indonesia/5/05 virus (Food and Drug Administration, Center for Biologics Evaluation and Research, Rockville, MD, USA) can be used to test mouse serum samples for hemagglutinin activity. Sera are pre-treated with receptor-destroying enzyme II (RDE II of Accurate Chemical and Scientific Corporation) prepared from *Vibrio cholerae.* Hemagglutination assays are performed with 0.5% turkey red blood cells. Hemagglutinin antibody titres can be defined as the reciprocal of the highest dilution causing complete inhibition of hemagglutination.

### 20.3 Lethal challenge in mice.

In a non-limiting example, an experiment is performed with 6-8 week-old female BALB/c mice (Charles River Laboratories) using forty mice randomly divided into five groups of eight mice. Optionally, a different number of groups and mice per group can be used. Mice are immunized with influenza virus-like particles corresponding to a dosage of 0.5 µg, 2.5 µg or 7.5 µg of hemagglutinin and phophate-buffered saline is used for the fourth control group of mice. Antigens can be formulated with alum at a final concentration of 1 % (v/v). Optionally, no adjuvant and no alum is used. In a non-limiting example, mice are immunized intramuscularly on days 0 and 14 and challenged intranasally with one LD₅₀ dose of live influenza virus (A/Vietnam/1194/04; H5N1), 75 days after second immunization. Mice are monitored daily after challenge for behavioural changes, the body weight is measured every 2 days, or daily if animals show signs of disease. Mice having a 25% decrease in body weight are euthanized according to standard procedures and survival is measured over a period of 14 days.

### Example 21: Measurement of efficacy of vaccine in ferrets.

This example describes experiments to test the efficacy of an influenza vaccine comprising virus-like particles displaying hemagglutinin according to the present invention, in ferrets. Ferrets are exquisitely susceptible to infection with human influenza viruses and are widely used for influenza research. Moreover, ferrets develop some of the symptoms of influenza that are seen in humans.

### 21.1 Ferret study.

Male Fitch ferrets are castrated, descented and analysed for being seronegative for representative circulating human influenza A strains. At the time of onset of experiments, ferrets are 6-8 months old and between 0.8 and 1.6 kg. Ferrets are vaccinated twice intramuscularly on days 0 and 21 with a composition comprising influenza virus-like particles according to the present invention and with corresponding dosages of hemagglutinin H5 protein of 0.7 mg, 1.8 mg, 3.7 mg or 11.0 mg, formulated with alum (AlhydrogelH; 1 mg/mL dose). As a control, ferretes are immunized with a solution of phosphate-buffered saline with alum. In a non-limiting example, eight animals per group of the 1.8 mg or 3.7 mg vaccination dose, and of the control group, are challenged intranasally 45 days after the first boost at day 21, with a lethal dose of influenza virus A/Vietnam/1203/04 (H5N1 clade 1 virus). A lethal dose is equivalent to ten times the ferret LD₅₀. Ferrets are monitored for weight loss, temperature changes and loss of activity weekly during the period between vaccination and challenge, and daily during the challenge period. Surviving ferrets are euthanized 14 days post-challenge. Three challenged animals of each group are sacrificed 3 days after post-challenge and lungs and nasal turbinate tissues are collected, weighted, snap frozen in liquid nitrogen for later use to determine virus titres. Homogenized samples are tenfold serially diluted and inoculated into viable 10 to 11 day old embryonated hens eggs and influenza viral titers are measured by the Egg Infectious Dose₅₀ (EID₅₀) assay. Data are expressed as log₁₀(EID₅₀/mL) using the Reed-Muench method (Reed and Muench (1938) A simple method of estimating fifty percent endpoints. The Amer J of Hygeine 27: 493-497). Blood is collected of anaesthetized ferrets via the anterior vena cava before the first and second immunization as well as 14 days after the second immunization. Sera are stored in aliquots at -20°C until use.

### 21.2 Assessment immune responses.

A hemagglutination inhibition assay is as recommended by the World Health Organisation (WHO/CDS/CSR/NCS/2002.5 WHO manual on animal influenza diagnosis and surveillance). Inactivated virus is H5N1 strain A/Indonesia/5/05, A/Vietnam/1203/2004, A/Anhui/01/2005, or A/turkey/Turkey/1/05. Sera are pre-treated with receptor-destroying enzyme II (RDE II of Denka Seiken Co., Tokyo, Japan) overnight at 37°C, phosphate buffered saline is added to obtain a 1:8 and 1:10 dilution. Sera are two-fold serially diluted in V-bottom microtiter plates. Twenty five µL of test virus (2-8 HAU/50 µL) are added to each well and plates are incubated at room temperature for 30 min prior to adding 0.5% horse erythrocytes (Lampire Biologicals, Pipersville, PA). Plates are further incubated at room temperature for 60 to 90 min and hemagglutinin titres are defined as the reciprocal of the highest dilution causing complete inhibition of hemagglutination. Seroconversion is defined as a four-fold increase in hemagglutinin titer from baseline (≤8) to hemagglutinin titers ε32. Seroprotection is defined as the proportion of subjects with hemagglutinin titer ≥40.

### Example 22: Method for determining lipid to protein ratio of Virus-Like Particles

This example describes the sulpho-phospho-vanillin method to determine the total lipid to to protein ratio of a virus-like particle sample.

### 22.1 Sulpho-phospho-vanillin method.

Phosphate buffered saline containing 10mM sodium phosphate, 150mM sodium chloride, pH 7.5, is from Biorad (Biorad 161-0780). 0.6g Vanillin (Sigma 63118) is dissolved in 10mL of absolute ethanol before making up to 100mL with purified water. The resulting solution is then mixed with 400mL of concentrated H2PO4 under constant stirring to generate phospho-vanillin. The phospho-vanilline solution is stored in a dark bottle at room temperature. Oleic acid (Sigma 01008) is dissolved at 10g/L in absolute ethanol and diluted further where appropriate.

### 22.2 Determination of lipid:protein ratio.

Concentrated purified virus-like particles are disrupted by adding 1% (v/v) Triton X-100 and homogenized for 30 seconds at 25°C using a TissueLyzer II (Qiagen) to dissociate the hemagglutinin proteins from the lipids. 0.4 mL of concentrated H2SO4 is added to a test tube containing 0.2mL of the protein-lipid sample to be tested. The tube is heated for 10 minutes in a boiling water bath, subsequently cooled, and a 0.4mL aliquot is placed in a clean dry tube. 1mL of phospho-vanillin reagent is added to a 50µg sample as determined using the Bradford method. The mixture is then incubated in the dark for 45 minutes and the final absorbance value is measured at 525nm wavelength. Sample analyses are always performed in triplicate and the final values are reported as a mean. A standard curve is made with 200, 100, 50, 10 and 0µg of oleic acid and absorbance values are determined using a plate reader. The lipid content is determined directly following the virus-like particles purification.

### 22.3 Lipid:protein ratio of Nicotiana-derived Virus-Like Particles.

The quantity of lipids detected using the sulpho-phospho-vanillin test varied from 130 to 134µg of lipids per 50µg of hemagglutinin protein for three different batches of *N. benthamiana-derived* virus-like particles. The resulting protein to lipid ratio is 0.37 with a standard deviation of 0.05 (Table 20).

**Table 20. Protein to lipid ratio obtained from three different batches of N. benthamiana plant biomass.**

| Batch number | 1 | 2 | 3 |
|---|---|---|---|
| Mean absorbance at 525nm | 0.41 | 0.41 | 0.40 |
| Lipid quantity (µg) | 134 | 132 | 130 |
| Lipid:protein ratio | 0.37 | 0.37 | 0.38 |

As shown in the next table (Table 21), H5-VLP's from batches 1 and 3 showed similar results while H5-VLP's from batch 2 showed very low amounts of both total lipids and proteins though their ratio was still acceptable.

**Table 21. Lipid and protein content of three batches of virus-like particles of N. benthamiana**

| | **Lipid (ug/ml)** | **Protein (ug/ml)** | **Ratio lipid/protein** |
|---|---|---|---|
| VLP 1 | 5 | 24 | 0.2 |
| VLP 2 | 1.3 | 3 | 0.4 |
| VLP 3 | 4.6 | 20 | 0.2 |

### Example 23: Method for altering the lipid composition of Virus-Like Particles

Lipid-based particles such as liposomes, ethosomes, transferosomes, bilosomes and immune stimulating complexes are highly immunogenic and can be used for subcutaneous, intranasal, oral, mucosal or transdermal administration. This example describes methods for changing or altering the lipid composition of virus-like particles. Such methods can be employed to alter the lipid composition of the endomembranes of the virus-like particles of the present invention displaying hemagglutinin. By doing so, physical and/or immunogenic properties of such particles and associated antigen can be changed.

### 23.1 Liposomes.

In a non-limiting example, liposomes may be made comprising phospholipids and cholesterol by lipid film hydration or dehydration-rehydration.

### 23.2 Cationic liposomes.

Cationic liposomes may be made comprising phospholipids and cationic lipids such as for example DOTAP, DOTAP/DOPE, DOTMA, DOSPA, DOSPA/DOPE, DOTAP/cholesterol, DC/cholesterol or dimethyldioctadecylammonium by lipid film hydration or dehydration-rehydration.

### 23.3 ISCOMs.

Immune stimulating complexes may be made comprising *Quillaja* saponins, DC-cholesterol and phospholipids by dialysis, centrifugation, lipid film hydration, ethanol injection or ether injection.

### 23.4 PLUSCOMs.

Cationic cage-like particles can be made by lipid film hydration comprising *Quillaja* saponins, DC-cholesterol and/or phospholipid.

### 23.5 Transferosomes.

Transferosomes can be made by lipid film hydration of phospholipids, sodium-cholate, sodium deoxycholate, sorbitan monolaureate and/or polyoylethylensorbitan monolaureate.

### 23.6 Ethosomes.

Ethosomes can be made by lipid film hydration comprising phospholipids and large quantities of ethanol (20-45%).

### 23.7 Bilosomes.

Bilosomes can be made comprising non-ionic surfactant vesicles and/or bile salts using lipid film hydration.

### 23.8 Lipid implants.

Lipid implants can be made comprising *Quillaja* saponins or imiquimod or α-galactosylceramide, cholesterol or DC-cholesterol or phospholipids using lipid film hydration and freeze-drying and compression.

### Example 24. Polymerizing Virus-Like Particles

This example describes methods to enhance the stability of the virus-like particles of the present invention by polymerizing components in the plane of the virus-like particle lipid bilayer.

### 24.1 Stabilization by polymerization.

Polymerizable functionalities such as but not limited to acrylic, styrenic, acetylene and dienoyl groups, are incorporated in the head, chain or tail region of the lipid of a virus-like particle of the present invention. This way, the virus-like particles of the present invention can be stabilized by either polymerizing the reactive headgroups at the surface of the virus-like particle lipid bilayer, or by polymerizing the reactive groups in phospholipid tails.

### Example 25. Electron-cryo and atomic force microscopy measurements of particle

This example discloses methods to measure the shape, structure and homogeneity of virus-like particles using electron-cryo and atomic force microscopy. High resolution atomic force microscopy allows for the measurement of interactions that hold the virus-like particle together and stability and structural transformations for example due to mechanical stress or pH changes.

### 25.1 Electron cryo-microscopy.

A high resolution electron cryo-microscope (JEM-3200) is used to study the structure of virus-like particles. Virus-like particles are embedded in a thin film of vitreous ice on a specimen grid using a Vitrobot immersion cryo-fixation apparatus. Observation of specimens is by using low dose protocol designed for recording high resolution images of radiation sensitive specimens with minimum electron exposure.

### 25.2 Atomic force microscopy.

For atomic force microscopy, virus-like particles are suspended in a buffer containing 25 mM HEPES and 150 mM NaCl (pH 7.5). As substrate, freshly cleaved highly-oriented pyrolithic graphite (HOPG) is used. Atomic Force Microscope can be AA2000 Atomic Force Microscope or AA5000 Multi-function Scanning Probe Microscope (SPM) Systems apparatus (Angstrom Advanced Inc., Braintree, USA).

### Deposit:

The following seed samples were deposited with NCIMB, Ferguson Building, Craibstone Estate, Bucksburn, Aberdeen AB21 9YA, Scotland, UK on January 6, 2011 under the provisions of the Budapest Treaty in the name of Philip Morris Products S.A:

| PM seed line designation | Deposition date | Accession No |
|---|---|---|
| PM016 | 6 January 2011 | NCIMB 41798 |
| PM021 | 6 January 2011 | NCIMB 41799 |
| PM092 | 6 January 2011 | NCIMB 41800 |
| PM1 02 | 6 January 2011 | NCIMB 41801 |
| PM132 | 6 January 2011 | NCIMB 41802 |
| PM204 | 6 January 2011 | NCIMB 41803 |
| PM205 | 6 January 2011 | NCIMB 41804 |
| PM215 | 6 January 2011 | NCIMB 41805 |
| PM216 | 6 January 2011 | NCIMB 41806 |
| PM217 | 6 January 2011 | NCIMB 41807 |

## Claims

1. A method of producing a VLP displaying one or more influenza hemagglutinin antigens or an immunogenic fragment thereof, wherein said VLPs have an average size distribution of between 40 nm and 250 nm in diameter comprising the steps of:
i. providing a combination of a selected variety, breeding line, or cultivar of a *Nicotiana tabacum* plant and a selected strain of an *Agrobacterium* species, which variety, breeding line, or cultivar, exhibits less than 10% necrosis, less than 5% necrosis, less than 2% necrosis or less than 1% necrosis, 5 days after leaves of said variety, breeding line, or cultivar have been injected by syringe with the selected *Agrobacterium* strain at a cell density of OD₆₀₀ of 0.32;
ii. infiltrating a whole plant of the selected variety, breeding line, or cultivar of *Nicotiana tabacum* with a suspension of the selected strain of the *Agrobacterium* species comprising a binary vector, comprising the coding sequence of one or more influenza antigens that is (are) under control of regulatory elements functional in a *Nicotiana tabacum* plant, at an OD₆₀₀ of between 0.1 and 4.0,
iii. incubating the infiltrated plant for a period of between 5 days and 20 days, under conditions that allow expression of the expressible nucleotide sequence in the infiltrated plant and accumulation of the heterologous polypeptide; and optionally the steps of:
iv. purification/cleaning of the VLPs produced; and
v. capturing of VLPs.

2. The method of claim 1, wherein said binary vector is a minimally-sized binary vector comprising sequence elements, which are essential for maintenance and replication of the plasmid in *Escherichia coli* and *Agrobacterium* cells, and for the transfer of the T-DNA to a tobacco plant cell, and further a T-DNA region and wherein the essential sequence elements accounts for at least 60%, 65%, 70%, 75%, 80% of the entire minimally-sized binary vector.

3. The method of claim 2, wherein the binary vector comprises a plant selectable marker gene.

4. The method of any one of claims 1-2, wherein the infiltrated plant is incubated for a period of between 7 days and 15 days.

5. The method of any one of claims 1-2, wherein the infiltrated plant is incubated for a period of between 8 days and 10 days.

6. The method of any one of claims 1-5, wherein said VLPs have an average size distribution of between 50 nm and 200 nm in diameter.

7. The method of any one of claims 1-6, wherein said VLPs are composed of a lipid bilayer comprising a combination of fatty acids and sterols.

8. The method of any one of claims 1-7, wherein the lipid bilayer of the VLPs has a content of
a. oleic acid of less than 0.5 µg/mL; and/or
b. linoleic acid and/or linolenic acid in a concentration of between 3.0 µg/mL and 5.0 µg/mL; and/or
c. arachidic acid of greater than 0.75 µg/mL.

9. The method of any one of claims 1-8, wherein the VLPs
a. have in the lipid bilayer a cholesterol:sitosterol ratio of >1, when determined by LC-APPCI-MS/MS; and/or
b. exhibit a mass ratio of lipid (fatty acid and sterols) to protein that is between 0.30 and 0.45.

10. The method of any one of claims 1-9, wherein the hemagglutinin is an Influenza type A or an Influenza type B hemagglutinin.

11. The method of claim 10, wherein the Influenza sub-type A hemagglutinin is selected from the group consisting of H1, H2, H3, H4, H5, H6, H7, H8, H9, H10 H11, H12, H13, H14, H15, and H16.

12. The method of claim 11, wherein the Influenza sub-type A hemagglutinin is a hemagglutinin of the H5 sub-type.

13. The method of claim 12, wherein the antigen displayed on the VLPs is an influenza A hemagglutinin of the H5 sub-type as shown in SEQ ID NO: 14.

14. The method of any one of claims 1-13, wherein the hemagglutinin displayed by the virus-like particles is
a. S-acylated;
b. N-glycosylated;
c. S-acylated and N-glycosylated.

15. The method of claim 14, wherein only two cytoplasmic cysteines are post-translationally modified by acylation with palmitic acid.

## Patentansprüche

1. Verfahren zum Herstellen eines VLP, das ein oder mehrere Influenza-Hämagglutinin-Antigene oder ein immunogenes Fragment davon darstellt, wobei die VLPs eine Durchschnittsgrößenverteilung zwischen 40 nm und 250 nm im Durchmesser aufweisen, umfassend die Schritte:
i. Bereitstellen einer Kombination aus einer ausgewählten Varietät, Zuchtlinie oder Kultursorte einer *Nicotiana tabacum* Pflanze und einem ausgewählten Stamm einer *Agrobakterium* Spezies, wobei die Varietät, Zuchtlinie oder Kultursorte 5 Tage, nachdem Blättern der Varietät, Zuchtlinie oder Kultursorte mittels Spritze der ausgewählte *Agrobakterium*-Stamm bei einer Zelldichte von OD₆₀₀ von 0,32 injiziert wurde, weniger als 10 % Nekrose, weniger als 5 % Nekrose, weniger als 2 % Nekrose oder weniger als 1 % Nekrose aufweist;
ii. Infiltrieren einer Gesamtpflanze der ausgewählten Varietät, Zuchtlinie oder Kultursorte von *Nicotiana tabacum* mit einer Suspension des ausgewählten Stamms der *Agrobakterium* Spezies, die einen binären Vektor aufweist, der die Codiersequenz von einem oder mehreren Influenza-Antigenen aufweist, das bzw. die unter der Kontrolle von regulatorischen Elementen sind, die in einer *Nicotiana tabacum* Pflanze funktionell sind, bei einem OD₆₀₀ zwischen 0,1 und 4,0;
iii. Inkubieren der infiltrierten Pflanze für einen Zeitraum zwischen 5 Tagen und 20 Tagen unter Bedingungen, welche die Expression der exprimierbaren Nukleotidsequenz in der infiltrierten Pflanze und die Akkumulation des heterologen Polypeptids ermöglichen; und wahlweise die Schritte:
iv. Reinigen/Abtrennen der produzierten VLPs; und
v. Erfassen der VLPs.

2. Verfahren nach Anspruch 1, wobei der binäre Vektor ein minimaldimensionierter binärer Vektor ist, der Sequenzelemente aufweist, die für die Aufrechterhaltung und Replikation des Plasmids in *Escherichia coli-* und *Agrobakterium*-Zellen und für die Übertragung der T-DNA auf eine Tabakpflanzenzelle wesentlich sind, und weiter eine T-DNA-Region, und wobei die wesentlichen Sequenzelemente mindestens 60 %, 65 %, 70 %, 75 %, 80 % des gesamten minimaldimensionierten binären Vektors ausmachen.

3. Verfahren nach Anspruch 2, wobei der binäre Vektor ein pflanzenselektierbares Markergen aufweist.

4. Verfahren nach einem der Ansprüche 1 bis 2, wobei die infiltrierte Pflanze für einen Zeitraum zwischen 7 Tagen und 15 Tagen inkubiert wird.

5. Verfahren nach einem der Ansprüche 1 bis 2, wobei die infiltrierte Pflanze für einen Zeitraum zwischen 8 Tagen und 10 Tagen inkubiert wird.

6. Verfahren nach einem der Ansprüche 1 bis 5, wobei die VLPs eine Durchschnittsgrößenverteilung zwischen 50 nm und 200 nm im Durchmesser aufweisen.

7. Verfahren nach einem der Ansprüche 1 bis 6, wobei die VLPs aus einer Lipiddoppelschicht bestehen, die eine Kombination aus Fettsäuren und Sterolen aufweist.

8. Verfahren nach einem der Ansprüche 1 bis 7, wobei die Lipiddoppelschicht der VLPs einen Gehalt an
a. Ölsäure von weniger als 0,5 µg/ml; und/oder
b. Linolsäure und/oder Linolensäure in einer Konzentration zwischen 3,0 µg/ml und 5,0 µg/ml; und/oder
c. Arachinsäure von größer als 0,75 µg/ml aufweist.

9. Verfahren nach einem der Ansprüche 1 bis 8, wobei die VLPs
a. in der Lipiddoppelschicht bei Bestimmung durch LC-APPCI-MS/MS ein Cholesterol:Sitosterol-Verhältnis von > 1 aufweisen; und/oder
b. ein Massenverhältnis von Lipid (Fettsäure und Sterole) zu Protein aufweisen, das zwischen 0,30 und 0,45 liegt.

10. Verfahren nach einem der Ansprüche 1 bis 9, wobei das Hämagglutinin ein Influenza Typ A- oder ein Influenza Typ B-Hämagglutinin ist.

11. Verfahren nach Anspruch 10, wobei das Influenza-Subtyp A-Hämagglutinin ausgewählt ist aus der Gruppe bestehend aus H1, H2, H3, H4, H5, H6, H7, H8, H9, H10 H11, H12, H13, H14, H15 und H16.

12. Verfahren nach Anspruch 11, wobei das Influenza-Subtyp A-Hämagglutinin ein Hämagglutinin des Subtyps H5 ist.

13. Verfahren nach Anspruch 12, wobei das auf den VLPs dargestellte Antigen ein Influenza A-Hämagglutinin des H5 Subtyps ist, wie in SEQ ID NO: 14 gezeigt.

14. Verfahren nach einem der Ansprüche 1 bis 13, wobei das durch den virusähnlichen Partikel dargestellte Hämagglutinin
a. S-acyliert ist;
b. N-glykosyliert;
c. S-acyliert und N-glykosyliert ist.

15. Verfahren nach Anspruch 14, wobei nur zwei zytoplasmatische Cysteine durch Acylierung mit Palmitinsäure posttranslational modifiziert sind.

## Revendications

1. Procédé de production d'une particule de type virus présentant un ou plusieurs antigènes de l'hémagglutinine grippale ou un fragment immunogène de ceux-ci, dans lequel lesdites particules de type virus ont une distribution de taille moyenne comprise entre 40 nm et 250 nm de diamètre, comprenant les étapes consistant en :
i. la fourniture d'une combinaison d'une variété, d'une lignée généalogique ou d'un cultivar d'un plant de *Nicotinia tabacum* choisi et d'une souche choisie d'une espèce *d'agrobacterie*, laquelle variété, lignée généalogique ou lequel cultivar présente moins que 10 % de nécrose, moins de 5 % de nécrose, moins de 2 % de nécrose ou moins de 1 % de nécrose, 5 jours après que des feuilles des dits variété, lignée généalogique ou cultivar aient été soumis à une injection par seringue avec la souche *d'agrobactérie* choisie à une densité cellulaire d'OD₆₀₀ de 0,32 ;
ii. l'infiltration d'une plante entière de la variété, lignée de sélection ou cultivar choisi de *Nicotiana tabacum* par une suspension de la souche choisie de l'espèce *d'agrobactérie* comprenant un vecteur binaire, comprenant la séquence codante d'un ou de plusieurs antigènes de la grippe contrôlés par des éléments régulateurs fonctionnels dans une plante de *Nicotiana tabacum,* à une OD₆₀₀ comprise entre 0,1 et 4,0,
iii. l'incubation du plant infiltré pendant une période comprise entre 5 jours et 20 jours dans des conditions qui permettent l'expression de la séquence nucléotidique exprimable dans le plant infiltré et l'accumulation du polypeptide hétérologue ; et, facultativement les étapes consistant en :
iv. la purification / le nettoyage des particules de type virus produits ; et
v. la capture de particules de type virus.

2. Procédé selon la revendication 1 dans lequel ledit vecteur binaire est un vecteur binaire de taille minimale comprenant des éléments de séquence qui sont essentiels pour la maintenance et la réplication du plasmide dans des cellules *d'Escherichia coli* et *d'agrobactérie*, et pour le transfert d'un ADN-T à une cellule de plant de tabac et, en outre, une région d'ADN-T et dans lequel les éléments de séquence essentiels représentent au moins 60 %, 65 %, 70 %, 75 %, 80 % de l'ensemble du vecteur binaire de taille minimale.

3. Procédé selon la revendication 2, dans lequel le vecteur binaire comprend un gène marqueur sélectionnable par une plante.

4. Procédé selon l'une quelconque des revendications 1 et 2, dans lequel la plante infiltrée est incubée pendant une période comprise entre 7 et 15 jours.

5. Procédé selon l'une quelconque des revendications 1 et 2, dans lequel la plante infiltrée est incubée pendant une période comprise entre 8 et 10 jours.

6. Procédé selon l'une quelconque des revendications 1 à 5, dans lequel lesdites particules de type virus ont une distribution de taille moyenne comprise entre 50 nm et 200 nm de diamètre.

7. Procédé selon l'une quelconque des revendications 1 à 6, dans lequel lesdites particules de type virus sont composées d'une bicouche lipidique comprenant une combinaison d'acides gras et de stérols.

8. Procédé selon l'une quelconque des revendications 1 à 7, dans lequel la bicouche lipidique des particules de type virus a une teneur en
a. acide oléique inférieure à 0,5 µg/mL ; et/ou
b. acide linoléique et/ou acide linolénique à une concentration comprise entre 3,0 µg/mL et 5,0 µg/mL ; et/ou
c. acide arachidique supérieur à 0,75 µg/mL.

9. Procédé selon l'une quelconque des revendications 1 à 8, dans lequel les particules de type virus
a. ont dans la bicouche lipidique un rapport cholestérol:sitostérol > 1, lorsque déterminé par LC-APPCI-MS/MS ; et/ou
b. présentent un rapport en masse lipide (acide gras et stérols) à protéine compris entre 0,30 et 0,45.

10. Procédé selon l'une quelconque des revendications 1 à 9, dans lequel l'hémagglutinine est une hémagglutinine de la grippe de type A ou de la grippe de type B.

11. Procédé selon la revendication 10, dans lequel l'hémagglutinine de la grippe de sous-type A est choisie dans le groupe constitué par H1, H2, H3, H4, H5, H6, H7, H8, H9, H10, H11, H12, H13, H14, H15 et H16.

12. Procédé selon la revendication 11, dans lequel l'hémagglutinine de la grippe de sous-type A est une hémagglutinine du sous-type H5.

13. Procédé selon la revendication 12, dans lequel l'antigène affiché sur les particules de type virus est une hémagglutinine de la grippe de type A du sous-type H5, comme indiqué dans SEQ ID NO : 14.

14. Procédé selon l'une quelconque des revendications 1 à 13, dans lequel l'hémagglutinine présentée par les particules de type virus est
a. S-acylée ;
b. N-glycosylée ;
c. S-acylée et N-glycosylée.

15. Procédé selon la revendication 14, dans lequel seules deux cystéines cytoplasmiques sont à modification post-translationnelle par acylation avec de l'acide palmitique.


### SEQUENCE LISTING

<110> Philip Morris Products S.A.
<120> Virus like particles
<130> T1672 PCT BS
<150> EP 11188872.3
   <151> 2011-11-11
<160> 14
<170> BiSSAP 1.0
<210> 1
   <211> 5139
   <212> DNA
   <213> artificial sequences
<220>
   <221> source
   <222> 1..5139
   <223> /mol_type="DNA" /note="vector pPMP1" /organism="artificial sequences"
<400> 1
   ctactagtcc cctagtacat taaaaacgtc cgcaatgtgt tattaagttg tctaagcgtc        60
   aatttgttta caccacaata tatcctgcca ccagccagcc aacagctccc cgaccggcag       120
   ctcggcacaa aatcaccact cgatacaggc agcccatcag gccttgacgg ccttccttca       180
   attcgcccta tagtgagtcg tattacgtcg cgctcactgg ccgtcgtttt acaacgtcgt       240
   gactgggaaa accctggcgt tacccaactt aatcgccttg cagcacatcc ccctttcgcc       300
   agctggcgta atagcgaaga ggcccgcacc gaaacgccct tcccaacagt tgcgcagcct       360
   gaatggcgaa tgggagcgcc ctgtagcggc cactcaaccc tatctcggtc tattcttttg       420
   atttataagg gattttgccg atttcggcct attggttaaa aaatgagctg atttaacaaa       480
   aatttaacgc gaattttaac aaaatattaa cgcttacaat ttaggtggca cttttcgggg       540
   aaatgtgcgc ggaaccccta tttgtttatt tttctaaata cattcaaata tgtatccgct       600
   catgagacaa taaccctgat aaatgcttca ataatattga aaaaggaaga gtatgattga       660
   acaggatggc ctgcatgcgg gtagcccggc agcgtgggtg gaacgtctgt ttggctatga       720
   ttgggcgcag cagaccattg gctgctctga tgcggcggtg tttcgtctga gcgcgcaggg       780
   tcgtccggtg ctgtttgtga aaaccgatct gagcggtgcg ctgaacgagc tgcaggatga       840
   agcggcgcgt ctgagctggc tggccaccac cggtgttccg tgtgcggcgg tgctggatgt       900
   ggtgaccgaa gcgggccgtg attggctgct gctgggcgaa gtgccgggtc aggatctgct       960
   gtctagccat ctggcgccgg cagaaaaagt gagcattatg gcggatgcca tgcgtcgtct      1020
   gcataccctg gacccggcga cctgtccgtt tgatcatcag gcgaaacatc gtattgaacg      1080
   tgcgcgtacc cgtatggaag cgggcctggt ggatcaggat gatctggatg aagaacatca 1140
   gggcctggca ccggcagagc tgtttgcgcg tctgaaagcg agcatgccgg atggcgaaga 1200
   tctggtggtg acccatggtg atgcgtgcct gccgaacatt atggtggaaa atggccgttt 1260
   tagcggcttt attgattgcg gccgtctggg cgtggcggat cgttatcagg atattgcgct 1320
   ggccacccgt gatattgcgg aagaactggg cggcgaatgg gcggatcgtt ttctggtgct 1380
   gtatggcatt gcggcaccgg atagccagcg tattgcgttt tatcgtctgc tggatgaatt 1440
   tttctaataa ctgtcagacc aagtttactc atatatactt tagattgatt taaaacttca 1500
   tttttaattt aaaaggatct aggtgaagat cctttttgat aatctcatga ccaaaatccc 1560
   ttaacgtgag ttttcgttcc actgagcgtc agaccccgta gaaaagatca aaggatcttc 1620
   ttgagatcct ttttttctgc gcgtaatctg ctgcttgcaa acaaaaaaac caccgctacc 1680
   agcggtggtt tgtttgccgg atcaagagct accaactctt tttccgaagg taactggctt 1740
   cagcagagcg cagataccaa atactgttct tctagtgtag ccgtagttag gccaccactt 1800
   caagaactct gtagcaccgc ctacatacct cgctctgcta atcctgttac cagtggctgc 1860
   tgccagtggc gataagtcgt gtcttaccgg gttggactca agacgatagt taccggataa 1920
   ggcgcagcgg tcgggctgaa cggggggttc gtgcacacag cccagcttgg agcgaacgac 1980
   ctacaccgaa ctgagatacc tacagcgtga gctatgagaa agcgccacgc ttcccgaagg 2040
   gagaaaggcg gacaggtatc cggtaagcgg cagggtcgga acaggagagc gcacgaggga 2100
   gcttccaggg ggaaacgcct ggtatcttta tagtcctgtc gggtttcgcc acctctgact 2160
   tgagcgtcga tttttgtgat gctcgtcagg ggggcggagc ctatggaaaa acgccagcaa 2220
   cgcggccttt ttacggttcc tggccttttg ctggcctttt gctcattagg caccccaggc 2280
   tttacccgaa cgaccgagcg cagcgagtca gtgagcgagg aagcggagag cgcccaatac 2340
   gcaaggaaac agctatgacc atgttaatgc agctggcacg acaggtttcc cgactggaaa 2400
   gcgggcagtg aaaggaaggc ccatgaggcc agttaattaa cgatcgagta ctaaatgcca 2460
   gtaaagcgct ggctgctgaa cccccagccg gaactgaccc cacaaggccc tagcgtttgc 2520
   aatgcaccag gtcatcattg acccaggcgt gttccaccag gccgctgcct cgcaactctt 2580
   cgcaggcttc gccgacctgc tcgcgccact tcttcacgcg ggtggaatcc gatccgcaca 2640
   tgaggcggaa ggtttccagc ttgagcgggt acggctcccg gtgcgagctg aaatagtcga 2700
   acatccgtcg ggccgtcggc gacagcttgc ggtacttctc ccatatgaat ttcgtgtagt 2760
   ggtcgccagc aaacagcacg acgatttcct cgtcgatcag gacctggcaa cgggacgttt 2820
   tcttgccacg gtccaggacg cggaagcggt gcagcagcga caccgattcc aggtgcccaa 2880
   cgcggtcgga cgtgaagccc atcgccgtcg cctgtaggcg cgacaggcat tcctcggcct 2940
   tcgtgtaata ccggccattg atcgaccagc ccaggtcctg gcaaagctcg tagaacgtga 3000
   aggtgatcgg ctcgccgata ggggtgcgct tcgcgtactc caacacctgc tgccacacca 3060
   gttcgtcatc gtcggcccgc agctcgacgc cggtgtaggt gatcttcacg tccttgttga 3120
   cgtggaaaat gaccttgttt tgcagcgcct cgcgcgggat tttcttgttg cgcgtggtga 3180
   acagggcaga gcgggccgtg tcgtttggca tcgctcgcat cgtgtccggc cacggcgcaa 3240
   tatcgaacaa ggaaagctgc atttccttga tctgctgctt cgtgtgtttc agcaacgcgg 3300
   cctgcttggc ctcgctgacc tgttttgcca ggtcctcgcc ggcggttttt cgcttcttgg 3360
   tcgtcatagt tcctcgcgtg tcgatggtca tcgacttcgc caaacctgcc gcctcctgtt 3420
   cgagacgacg cgaacgctcc acggcggccg atggcgcggg cagggcaggg ggagccagtt 3480
   gcacgctgtc gcgctcgatc ttggccgtag cttgctggac catcgagccg acggactgga 3540
   aggtttcgcg gggcgcacgc atgacggtgc ggcttgcgat ggtttcggca tcctcggcgg 3600
   aaaaccccgc gtcgatcagt tcttgcctgt atgccttccg gtcaaacgtc cgattcattc 3660
   accctccttg cgggattgcc ccgactcacg ccggggcaat gtgcccttat tcctgatttg 3720
   acccgcctgg tgccttggtg tccagataat ccaccttatc ggcaatgaag tcggtcccgt 3780
   agaccgtctg gccgtccttc tcgtacttgg tattccgaat cttgccctgc acgaatacca 3840
   gcgacccctt gcccaaatac ttgccgtggg cctcggcctg agagccaaaa cacttgatgc 3900
   ggaagaagtc ggtgcgctcc tgcttgtcgc cggcatcgtt gcgccacata tcgattatga 3960
   tagaatttac aagctataag gttattgtcc tgggtttcaa gcattagtcc atgcaagttt 4020
   ttatgctttg cccattctat agatatattg ataagcgcgc tgcctatgcc ttgccccctg 4080
   aaatccttac atacggcgat atcttctata taaaagatat attatcttat cagtattgtc 4140
   aatatattca aggcaatctg cctcctcatc ctcttcatcc tcttcgtctt ggtagctttt 4200
   taaatatggc gcttcataga gtaattctgt aaaggtccaa ttctcgtttt catacctcgg 4260
   tataatctta cctatcacct caaatggttc gctgggttta tcgcccggga gggttcgaga 4320
   agggggggca ccccccttcg gcgtgcgcgg tcacgcgcac agggcgcagc cctggttaaa 4380
   aacaaggttt ataaatattg gtttaaaagc aggttaaaag acaggttagc ggtggccgaa 4440
   aaacgggcgg aaacccttgc aaatgctgga ttttctgcct gtggacagcc cctcaaatgt 4500
   caataggtgc gcccctcatc tgtcagcact ctgcccctca agtgtcaagg atcgcgcccc 4560
   tcatctgtca gtagtcgcgc ccctcaagtg tcaataccgc agggcactta tccccaggct 4620
   tgtccacatc atctgtggga aactcgcgta aaatcaggcg ttttcgccga tttgcgaggc 4680
   tggccagctc cacgtcgccg gccgaaatcg agcctgcccc tcatctgtca acgccgcgcc 4740
   gggtgagtcg gcccctcaag tgtcaacgtc cgcccctcat ctgtcagtga gggccaagtt 4800
   ttccgcgagg tatccacaac gccggcggcc gcggtgtctc gcacacggct tcgacggcgt 4860
   ttctggcgcg tttgcagggc catagacggc cgccagccca gcggcgaggg caaccagccc 4920
   ggtgagcgtc gcaaaggcgc tcggtcttgg cgcgccaacc ctgtggttgg catgcacata 4980
   caaatggacg aacggataaa ccttttcacg cccttttaaa tatccgatta ttctaataaa 5040
   cgctcttttc tcttaggttt acccgccaat atatcctgtc aaacactgat agtttaaact 5100
   gaaggcggga aacgacaatc tgcctgcagg aattgaatt 5139
<210> 2
   <211> 517
   <212> DNA
   <213> artificial sequences
<220>
   <221> source
   <222> 1..517
   <223> /mol_type="DNA" /note="5' UTR HT-CPMV" /organism="artificial sequences"
<400> 2
   tattaaaatc ttaataggtt ttgataaaag cgaacgtggg gaaacccgaa ccaaaccttc 60
   ttctaaactc tctctcatct ctcttaaagc aaacttctct cttgtctttc ttgcgtgagc 120
   gatcttcaac gttgtcagat cgtgcttcgg caccagtaca acgttttctt tcactgaagc 180
   gaaatcaaag atctctttgt ggacacgtag tgcggcgcca ttaaataacg tgtacttgtc 240
   ctattcttgt cggtgtggtc ttgggaaaag aaagcttgct ggaggctgct gttcagcccc 300
   atacattact tgttacgatt ctgctgactt tcggcgggtg caatatctct acttctgctt 360
   gacgaggtat tgttgcctgt acttctttct tcttcttctt gctgattggt tctataagaa 420
   atctagtatt ttctttgaaa cagagttttc ccgtggtttt cgaacttgga gaaagattgt 480
   taagcttctg tatattctgc ccaaatttgt cgggccc 517
<210> 3
   <211> 188
   <212> DNA
   <213> artificial sequences
<220>
   <221> source
   <222> 1..188
   <223> /mol_type="DNA" /note="3' UTR HT-CPMV" /organism="artificial sequences"
<400> 3 agaccggg 188
<210> 4
   <211> 2781
   <212> DNA
   <213> artificial sequences
<220>
   <221> source
   <222> 1..2781
   <223> /mol_type="DNA" /note="P1-HcPro-P3" /organism="artificial sequences"
<400> 4
   atggcactca tctttggcac agtcaacgct aacatcctga aggaagtgtt cggtggagct      60
   cgtatggctt gcgttaccag cgcacatatg gctggagcga atggaagcat tttgaagaag     120
   gcagaagaga cctctcgtgc aatcatgcac aaaccagtga tcttcggaga agactacatt     180
   accgaggcag acttgcctta cacaccactc catttagagg tcgatgctga aatggagcgg     240
   atgtattatc ttggtcgtcg cgcgctcacc catggcaaga gacgcaaagt ttctgtgaat     300
   aacaagagga acaggagaag gaaagtggcc aaaacgtacg tggggcgtga ttccattgtt     360
   gagaagattg tagtgcccca caccgagaga aaggttgata ccacagcagc agtggaagac     420
   atttgcaatg aagctaccac tcaacttgtg cataatagta tgccaaagcg taagaagcag     480
   aaaaacttct tgcccgccac ttcactaagt aacgtgtatg cccaaacttg gagcatagtg     540
   cgcaaacgcc atatgcaggt ggagatcatt agcaagaaga gcgtccgagc gagggtcaag     600
   agatttgagg gctcggtgca attgttcgca agtgtgcgtc acatgtatgg cgagaggaaa     660
   agggtggact tacgtattga caactggcag caagagacac ttctagacct tgctaaaaga     720
   tttaagaatg agagagtgga tcaatcgaag ctcacttttg gttcaagtgg cctagttttg     780
   aggcaaggct cgtacggacc tgcgcattgg tatcgacatg gtatgttcat tgtacgcggt     840
   cggtcggatg ggatgttggt ggatgctcgt gcgaaggtaa cgttcgctgt ttgtcactca     900
   atgacacatt atagcgacca tcaccatcac catcacgcgt ccgacaaatc aatctctgag     960
   gcattcttca taccatactc taagaaattc ttggagttga gaccagatgg aatctcccat    1020
   gagtgtacaa gaggagtatc agttgagcgg tgcggtgagg tggctgcaat cctgacacaa    1080
   gcactttcac cgtgtggtaa gatcacatgc aaacgttgca tggttgaaac acctgacatt    1140
   gttgagggtg agtcgggagg aagtgtcacc aaccaaggta agctcctagc aatgctgaaa    1200
   gaacagtatc cagatttccc aatggccgag aaactactca caaggttttt gcaacagaaa    1260
   tcactagtaa atacaaattt gacagcctgc gtgagcgtca aacaactcat tggtgaccgc    1320
   aaacaagctc cattcacaca cgtactggct gtcagcgaaa ttctgtttaa aggcaataaa    1380
   ctaacagggg ccgatctcga agaggcaagc acacatatgc ttgaaatagc aaggttcttg    1440
   aacaatcgca ctgaaaatat gcgcattggc caccttggtt ctttcagaaa taaaatctca 1500
   tcgaaggccc atgtgaataa cgcactcatg tgtgataatc aacttgatca gaatgggaat 1560
   tttatttggg gactaagggg tgcacacgca aagaggtttc ttaaaggatt tttcactgag 1620
   attgacccaa atgaaggata cgataagtat gttatcagga aacatatcag gggtagcaga 1680
   aagctagcaa ttggcaattt gataatgtca actgacttcc agacgctcag gcaacaaatt 1740
   caaggcgaaa ctattgagcg taaagaaatt gggaatcact gcatttcaat gcggaatggt 1800
   aattacgtgt acccatgttg ttgtgttact cttgaagatg gtaaggctca atattcggat 1860
   ctaaagcatc caacgaagag acatctggtc attggcaact ctggcgattc aaagtaccta 1920
   gaccttccag ttctcaatga agagaaaatg tatatagcta atgaaggtta ttgctacatg 1980
   aacattttct ttgctctact agtgaatgtc aaggaagagg atgcaaagga cttcaccaag 2040
   tttataaggg acacaattgt tccaaagctt ggagcgtggc caacaatgca agatgttgca 2100
   actgcatgct acttactttc cattctttac ccagatgtcc tgagtgctga attacccaga 2160
   attttggttg atcatgacaa caaaacaatg catgttttgg attcgtatgg gtctagaacg 2220
   acaggatacc acatgttgaa aatgaacaca acatcccagc taattgaatt cgttcattca 2280
   ggtttggaat ccgaaatgaa aacttacaat gttggaggga tgaaccgaga tatggtcaca 2340
   caaggtgcaa ttgagatgtt gatcaagtcc atatacaaac cacatctcat gaagcagtta 2400
   cttgaggagg agccatacat aattgtcctg gcaatagtct ccccttcaat tttaattgcc 2460
   atgtacaact ctggaacttt tgagcaggcg ttacaaatgt ggttgccaaa tacaatgagg 2520
   ttagctaacc tcgctgccat cttgtcagcc ttggcgcaaa agttaacttt ggcagacttg 2580
   ttcgtccagc agcgtaattt gattaatgag tatgcgcagg taattttgga caatctgatt 2640
   gacggtgtca gggttaacca ttcgctatcc ctagcaatgg aaattgttac tattaagctg 2700
   gccacccaag agatggacat ggcgttgagg gaaggtggct atgctgtgac ctctgcagat 2760
   cgttcaaaca tttggcaata a 2781
<210> 5
   <211> 721
   <212> DNA
   <213> Mirabilis mosaic virus
<220>
   <221> source
   <222> 1..721
   <223> /mol_type="DNA" /organism="Mirabilis mosaic virus"
<400> 5
   tggagattca gaaaaatctc catcaacaaa taatccaagt aaggattaat ggattgatca 60
   acatccttac cgctatgggt aagattgatg aaaagtcaaa aacaaaaatc aattatgcac 120
   accagcatgt gttgatcacc agctattgtg ggacaccaat ttcgtccaca gacatcaaca 180
   tcttatcgtc ctttgaagat aagataataa tgttgaagat aagagtggga gccaccacta 240
   aaacattgct ttgtcaaaag ctaaaaaaga tgatgcccga cagccacttg tgtgaagcat 300
   gtgaagccgg tccctccact aagaaaatta gtgaagcatc ttccagtggt ccctccactc 360
   acagctcaat cagtgagcaa caggacgaag gaaatgacgt aagccatgac gtctaatccc 420
   acaagaattt ccttatataa ggaacacaaa tcagaaggaa gagatcaatc gaaatcaaaa 480
   tcggaatcga aatcaaaatc ggaatcgaaa tctctcatct ctctctacct tctctctaaa 540
   aaacacttag atgtgtgagt aatcacccac ttggggttgt aatatgtagt agtaaataag 600
   ggaaccttag ggtataccat tgttgtaata ttattttcag tatcaataaa ataatctttc 660
   agtttatctt atattcattt gtgtgacacc gtattcccat aaaaccgatc ctaatctctc 720
   c 721
<210> 6
   <211> 45
   <212> PRT
   <213> artificial sequences
<220>
   <221> SOURCE
   <222> 1..45
   <223> /mol_type="protein" /note="C-terminal hemagglutinin H5 peptide fragment" /organism="artificial sequences"
<400> 6
   Leu Glu Ser Ile Gly Thr Tyr Gln Ile Leu Ser Ile Tyr Ser Thr Val
   1               5                   10                  15
   Ala Ser Ser Leu Ala Leu Ala Ile Met Met Ala Gly Leu Ser Leu Trp
               20                  25                  30
   Met Cys Ser Asn Gly Ser Leu Gln Cys Arg Ile Cys Ile
           35                  40                  45
<210> 7
   <211> 46
   <212> PRT
   <213> artificial sequences
<220>
   <221> SOURCE
   <222> 1..46
   <223> /mol_type="protein" /note="C-terminal hemagglutinin H5 peptide fragment" /organism="artificial sequences"
<400> 7
   Lys Leu Glu Ser Ile Gly Thr Tyr Gln Ile Leu Ser Ile Tyr Ser Thr
   1               5                   10                  15
   Val Ala Ser Ser Leu Ala Leu Ala Ile Met Met Ala Gly Leu Ser Leu
               20                  25                  30
   Trp Met Cys Ser Asn Gly Ser Leu Gln Cys Arg Ile Cys Ile
           35                  40                  45
<210> 8
   <211> 23
   <212> PRT
   <213> artificial sequences
<220>
   <221> SOURCE
   <222> 1..23
   <223> /mol_type="protein" /note="Peptide fragment obtained from H5 mature protein backbone" /organism="artificial sequences"
<220>
   <221> VARIANT
   <222> 20
   <223> /replace="Xaa = Mox"
<400> 8
   Asp Gln Ile Cys Ile Gly Tyr His Ala Asn Asn Ser Thr Glu Gln Val
   1               5                   10                  15
   Asp Thr Ile Xaa Met Glu Lys
               20
<210> 9
   <211> 13
   <212> PRT
   <213> artificial sequences
<220>
   <221> SOURCE
   <222> 1..13
   <223> /mol_type="protein" /note="Peptide fragment obtained from H5 mature protein backbone " /organism="artificial sequences"
<400> 9
   Asn Val Thr Val Thr His Ala Gln Asp Ile Leu Glu Lys
   1               5                   10
<210> 10
   <211> 8
   <212> PRT
   <213> artificial sequences
<220>
   <221> SOURCE
   <222> 1..8
   <223> /mol_type="protein" /note="Peptide fragment obtained from H5 mature protein backbone" /organism="artificial sequences"
<400> 10
   Asn Ser Thr Tyr Pro Thr Ile Lys
   1               5
<210> 11
   <211> 27
   <212> PRT
   <213> artificial sequences
<220>
   <221> SOURCE
   <222> 1..27
   <223> /mol_type="protein" /note="Peptide fragment obtained from H5 mature protein backbone" /organism="artificial sequences"
<400> 11
   Ser Tyr Asn Asn Thr Asn Gln Glu Asp Leu Leu Val Leu Trp Gly Ile
   1               5                   10                  15
   His His Pro Asn Asp Ala Ala Glu Gln Thr Arg
               20                  25
<210> 12
   <211> 27
   <212> PRT
   <213> artificial sequences
<220>
   <221> SOURCE
   <222> 1..27
   <223> /mol_type="protein" /note="Peptide fragment obtained from H5 mature protein backbone" /organism="artificial sequences"
<400> 12
   Cys Gln Thr Pro Met Gly Ala Ile Asn Ser Ser Met Pro Phe His Asn
   1               5                   10                  15
   Ile His Pro Leu Thr Ile Gly Glu Cys Pro Lys
               20                  25
<210> 13
   <211> 14
   <212> PRT
   <213> artificial sequences
<220>
   <221> SOURCE
   <222> 1..14
   <223> /mol_type="protein" /note="Peptide fragment obtained from H5 mature protein backbone " /organism="artificial sequences"
<400> 13
   Asn Gly Thr Tyr Asn Tyr Pro Gln Tyr Ser Glu Glu Ala Arg
   1               5                   10
<210> 14
   <211> 1707
   <212> DNA
   <213> artificial sequences
<220>
   <221> source
   <222> 1..1707
   <223> /mol_type="DNA" /note="Mature Optimized HEI (H5)" /organism="artificial sequences"
<400> 14
   atggagaaaa tagtgcttct tcttgcaata gtcagtcttg ttaaaagtga tcagatttgc      60
   attggttacc atgcaaacaa ttcaacagag caggttgaca caatcatgga aaagaacgtt     120
   actgttacac atgcccaaga catactggaa aagacacaca acgggaagct ctgcgatcta     180
   gatggagtga agcctctaat tttaagagat tgtagtgtag ctggatggct cctcgggaac     240
   ccaatgtgtg acgaattcat caatgtaccg gaatggtctt acatagtgga gaaggccaat     300
   ccaaccaatg acctctgtta cccagggagt ttcaacgact atgaagaact gaaacaccta     360
   ttgagcagaa taaaccattt tgagaaaatt caaatcatcc ccaaaagttc ttggtccgat     420
   catgaagcct catcaggagt tagctcagca tgtccatacc tgggaagtcc ctcctttttt     480
   agaaatgtgg tatggcttat caaaaagaac agtacatacc caacaataaa gaaaagctac     540
   aataatacca accaagagga tcttttggta ctgtggggaa ttcaccatcc taatgatgcg     600
   gcagagcaga caaggctata tcaaaaccca accacctata tttccattgg gacatcaaca     660
   ctaaaccaga gattggtacc aaaaatagct actagatcca aagtaaacgg gcaaagtgga     720
   aggatggagt tcttctggac aattttaaaa cctaatgatg caatcaactt cgagagtaat     780
   ggaaatttca ttgctccaga atatgcatac aaaattgtca agaaagggga ctcagcaatt     840
   atgaaaagtg aattggaata tggtaactgc aacaccaagt gtcaaactcc aatgggggcg     900
   ataaactcta gtatgccatt ccacaacata caccctctca ccatcgggga atgccccaaa     960
   tatgtgaaat caaacagatt agtccttgca acagggctca gaaatagccc tcaaagagag    1020
   agcagaagaa aaaagagagg actatttgga gctatagcag gttttataga gggaggatgg    1080
   cagggaatgg tagatggttg gtatgggtac caccatagca atgagcaggg gagtgggtac    1140
   gctgcagaca aagaatccac tcaaaaggca atagatggag tcaccaataa ggtcaactca    1200
   atcattgaca aaatgaacac tcagtttgag gccgttggaa gggaatttaa taacttagaa    1260
   aggagaatag agaatttaaa caagaagatg gaagacgggt ttctagatgt ctggacttat    1320
   aatgccgaac ttctggttct catggaaaat gagagaactc tagactttca tgactcaaat    1380
   gttaagaacc tctacgacaa ggtccgacta cagcttaggg ataatgcaaa ggagctgggt    1440
   aacggttgtt tcgagttcta tcacaaatgt gataatgaat gtatggaaag tataagaaac    1500
   ggaacgtaca actatccgca gtattcagaa gaagcaagat taaaaagaga ggaaataagt    1560
   ggggtaaaat tggaatcaat aggaacttac caaatactgt caatttattc aacagtggcg    1620
   agttccctag cactggcaat catgatggct ggtctatctt tatggatgtg ctccaatgga    1680
   tcgttacaat gcagaatttg catttaa                                         1707